(19) Europäisches Patentamt
European Patent Office
Office européen des brevets

(11) Veröffentlichungsnummer: **0 023 707**
**B1**

# (12) EUROPÄISCHE PATENTSCHRIFT

(45) Veröffentlichungstag der Patentschrift: **30.04.86**

(51) Int. Cl.⁴: **C 07 D 487/04,** A 61 K 31/55 // C07D231/38, C07D231/16, C07D231/14, C07C59/185, (C07D487/04, 243:00, 231:00)

(21) Anmeldenummer: **80104544.4**

(22) Anmeldetag: **01.08.80**

## (54) Substituierte Tetraazatricyclen, Verfahren zu ihrer Herstellung, ihre Verwendung und sie enthaltende Arzneimittel.

**Verbunden mit 82105942.5 (europäische Anmeldenummer) durch Entscheidung vom 07.10.83.**

(30) Priorität: **03.08.79 CH 7128/79**
**10.08.79 CH 7333/79**
**23.04.80 CH 3149/80**

(43) Veröffentlichungstag der Anmeldung: **11.02.81 Patentblatt 81/6**

(45) Bekanntmachung des Hinweises auf die Patenterteilung: **30.04.86 Patentblatt 86/18**

(84) Benannte Vertragsstaaten: **AT BE CH DE FR GB IT LI LU NL SE**

(56) Entgegenhaltungen: **DE - A - 2 707 270**

(73) Patentinhaber: **Byk Gulden Lomberg Chemische Fabrik GmbH, Byk-Gulden-Strasse 2, D-7750 Konstanz (DE)**

(72) Erfinder: **Rainer, Georg, Dr., Josef-Anton-Feuchtmayer-Strasse 7, D-7750 Konstanz (DE)**
Erfinder: **Riedel, Richard, Dr., Brüelstrasse 22, D-7750 Konstanz (DE)**
Erfinder: **Klemm, Kurt, Dr., Im Weinberg 2, D-7753 Allensbach (DE)**
Erfinder: **Eltze, Manfrid, Dr., Schützenstrasse 20, D-7750 Konstanz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 023 707 B1

LIBER, STOCKHOLM 1986

**Beschreibung**

Die Erfindung betrifft Tetraazatricyclen, Verfahren zu ihrer Herstellung, ihre Verwendung und sie enthaltende Arzneimittel.

Die erfindungsgemäßen Verbindungen werden in der pharmazeutischen Industrie als Zwischenprodukte und zur Herstellung von Medikamenten verwendet.

In der deutschen Auslegeschrift DE-AS 17 95 183 werden Pyridobenzidiazepine mit ulkushemmender, sekretionshemmender, antitussiver und teilweise antiemetischer Wirkung beansprucht. Dagegen sind aus der deutschen Offenlegungsschrift DE-OS 16 45 956 Pyrazolobenzoxazepine und Pyrazolobenzothiazepine bekannt, denen eine entzündungshemmende und antipyretische Wirkung zugesprochen wird. Pyrazolobenzoxazepine und Pyrazolobenzothiazepine bzw. Pyrazolobenzodiazepine werden in der De-OS 27 07 269 bzw. in der De-Os 27 07 270 beschrieben, wonach sie insbesondere eine urikosurische und urikostatische Wirksamheit, daneben aber auch analgetische, antiphlogistische, antidepressive, antiarrhytmische und diuretische Wirkungen aufweisen. Es wurden nun Pyrazolobenzodiazepinone konzipiert, die überraschende pharmakologische Wirkungen aufweisen.

Gegenstand der Erfindung sind substituierte Pyrazolobenzodiazepinone der allgemeinen Formel I

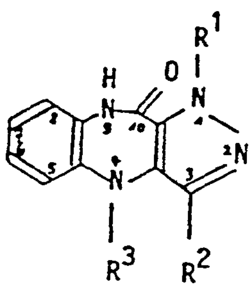

(I),

Verbindungen der Formel I, worin
$R^1$ einen Alkylrest mit 1 bis 4 Kohlenstoffatomen,
$R^2$ ein Wasserstoffatom oder einen Alkylrest mit 1 bis 4 Kohlenstoffatomen,
$R^3$ die Gruppe -CO-A-$R^4$,
$R^4$ die Gruppe -N($R^5$)$R^6$ und
$R^5$ einen Alkylrest mit 1 bis 4 Kohlenstoffatomen oder einen Alkyl- oder 2-Methylallylrest bedeuten,
$R^6$ eine der Bedeutungen von $R^5$ hat oder die Gruppe $-(CH_2)_m-N(R^7)R^8$ darstellt oder
$R^5$ und $R^6$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl- oder Ethylgruppe oder durch eine Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1 H-1,4-diazepin-1-yl-gruppe bedeuten und
$R^7$ eine Alkylgruppe mit 1 bis 4 Kohlenstoffatomen,
$R^8$ eine Alkylgruppe mit 1 bis 4 Kohlenstoffatomen,
A eine geradkettige oder verzweigte Alkylengruppe mit 1 bis 5 Kohlenstoffatomen und
m 2 oder 3 bedeuten,
und ihre Säureadditionssalze sind pharmakologisch wirksame Verbindungen.

Verbindungen der Formel I, worin
$R^1$ einen Alkylrest mit 1 bis 4 Kohlenstoffatomen,
$R^2$ ein Wasserstoffatom oder einen Alkylrest mit 1 bis 4 Kohlenstoffatomen,
$R^3$ ein Wasserstoffatom oder die Gruppe -CO-A-$R^4$,
$R^4$ ein Halogenatom und
A eine geradkettige oder verzweigte Alkylengruppe mit 1 bis 5 Kohlenstoffatomen bedeuten,
sowie ihre Säureadditionssalze sind Zwischenprodukte zur Herstellung der pharmakologisch wirksamen Verbindungen.

Alkylreste mit 1 bis 4 Kohlenstoffatomen sind der Methyl-, Ethyl-, Propyl-, Isopropyl-, n-Butyl-, Isobutyl-, sek.-Butyl-, tert.-Butylrest. Von den Alkylresten sind bei $R^1$, $R^2$, $R^5$, $R^6$, $R^7$ und $R^8$ der Methyl- und Ethylrest bevorzugt. Besonders bevorzugt als Alkylrest $R^1$ und $R^2$ ist der Methylrest.

Halogenatome (Hal) sind das lod-, das Brom- und insbesondere das Chloratom.

Alkylengruppen mit 1 bis 5 Kohlenstoffatomen sind die Trimethylen-, Tetramethylen-, Pentamethylen-, Propylen-, Ethylmethylengruppe, bevorzugt die Ethylengruppe, insbesondere die Methylengruppe.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze werden durch dem Fachmann bekannte Verfahren in pharmakologisch verträgliche Salze übergeführt. Als solche seien beispielsweise genannt wasserlösliche oder wasserunlösliche Säureadditionssalze, wie das Hydrobromid, Hydroiodid, Nitrat, Acetat, Benzoat, Hibenzat [2-(4-Hydroxybenzoyl)-benzoat], Fendizoat (2-[(2'-Hydroxy-4-biphenylyl)-carbonyl]-benzoat), Propionat, Butyrat, Sulfosalicylat, Laurat, Oxalat, Amsonat (4,4'-Diaminostilben-2,2'-disulfonat), Embonat

[4,4'-Methylen-bis-(3-hydroxy-2-naphthoat)], Metembonat [4,4'-Methylen-bis-(3-methoxy-2-naphthoat)], Stearat, 2-Hydroxy-3-naphthoat, 3-Hydroxy-2-naphthoat, insbesondere das Hydrochlorid, Phosphat, Sulfat, Citrat, Gluconat, Maleat, Malat, Fumarat, Succinat, Tartrat, Tosilat (p-Toluolsulfonat), Mesilat (Methansulfonat), Amidosulfonat.

Eine Ausgestaltung der Erfindung sind Zwischenprodukte der allgemeinen Formel I*

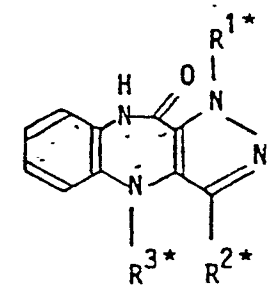

(I*),

worin
$R^{1*}$ einen Methyl- oder Ethylrest,
$R^{2*}$ ein Wasserstoffatom, einen Methyl- oder Ethylrest,
$R^{3*}$ ein Wasserstoffatom oder die Gruppe -CO-A*-$R^{4*}$
$R^{4*}$ ein Chloratom und
A* eine geradkettige oder verzweigte Alkylengruppe mit 1 oder 2 Kohlenstoffatomen bedeuten,
und ihre Säureadditionssalze.

Bevorzugte Vertreter der Ausgestaltung I* sind solche, in denen A* eine Methylengruppe bedeutet.

Besonders bevorzugte Vertreter der Ausgestaltung I* sind solche, in denen $R^{1*}$ einen Methyl- oder Ethylrest und $R^{2*}$ ein Wasserstoffatom, einen Methyl- oder Ethylrest, $R^{3*}$ ein Wasserstoffatom oder die Gruppe .CO-$CH_2$-Cl bedeuten, sowie ihre Säureadditionssalze.

Eine weitere Ausgestaltung der Erfindung sind pharmakologisch wirksame substituierte Pyrazolobenzodiazepinone der allgemeinen Formel I**

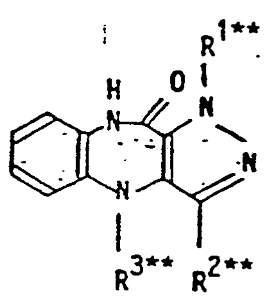

(I**),

worin
$R^{1**}$ einen Methyl- oder Ethylrest,
$R^{2**}$ ein Wasserstoffatom, einen Methyl- oder Ethylrest,
$R^{3**}$ die Gruppe -CO-A**-$R^{4**}$,
$R^{4**}$ die Gruppe -N($R^{5**}$)$R^{6**}$ und
$R^{5**}$ einen Alkylrest mit 1 bis 4 Kohlenstoffatomen oder einen Allyl- oder 2-Methylallylrest bedeuten,
$R^{6**}$ die Bedeutung von $R^{5**}$ hat oder die Gruppe -$(CH_2)_{m**}$-N($R^{7**}$)$R^{8**}$
darstellt oder
$R^{5**}$ und $R^{6**}$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexa-hydro-1H-1,4-diazepin-1-yl-gruppe bedeuten und
$R^{7**}$ eine Methyl- oder Ethylgruppe,
$R^{8**}$ eine Methyl- oder Ethylgruppe,
m** 2 oder 3,
A** eine geradkettige oder verzweigte Alkylengruppe mit 1 oder 2 Kohlenstoffatomen bedeuten,
und ihre Säureadditionssalze.

Eine Gruppe von Vertretern der Ausgestaltung I** sind solche, in denen $R^{1**}$ einen Methyl- oder Ethylrest, $R^{2**}$ ein Wasserstoffatom, einen Methyl- oder Ethylrest, $R^{5**}$ einen Methyl- oder Ethylrest bedeuten, $R^{6**}$ die Bedeutung von $R^{5**}$ hat oder die Gruppe $(CH_2)_{m**}$-N($R^{7**}$)$R^{8**}$ darstellt oder $R^{5**}$ und $R^{6**}$ gemeinsam unter Einschluß des Stickstoffatoms einen Pyrrolidino-, Piperidino- oder Hexahydroazepin-1-ylrest, $R^{7**}$ und $R^{8**}$ einen Methyl- oder Ethylrest, m** 2 und A** eine Methylengruppe bedeuten, sowie ihre pharmakologisch verträglichen Säureadditionssalze.

Eine andere Gruppe von Vertretern der Ausgestaltung I** sind solche, in denen $R^{1**}$ einen Methyl- oder Ethylrest, $R^{2**}$ ein Wasserstoffatom, einen Methyl- oder Ethylrest, $R^{5**}$ und $R^{6**}$ gemeinsam unter Einschluß des Stickstoffatoms eine in 4-Stellung durch eine Methyl-, Ethyl- oder Benzylgruppe substituierte Piperazin-1-yl-gruppe, eine 2,4-Dimethyl-piperazin-1-yl-gruppe oder eine in 4-Stellung durch eine Methyl- oder Ethylgruppe substituierte Hexahydro-1H-1,4-diazepin-1-yl-gruppe und A** eine Methylengruppe bedeuten, sowie ihre pharmakologisch verträglichen Säureadditionssalze.

Bevorzugte Vertreter der Ausgestaltung I** sind solche, in denen $R^{1**}$ einen Methyl- oder Ethylrest, $R^{2**}$ ein Wasserstoffatom, einen Methyl- oder Ethylrest, $R^{5**}$ und $R^{6**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze.

Besonders bevorzugt ist der Vertreter der Ausgestaltung I**, in dem $R^{1**}$ und $R^{2**}$ einen Methylrest, $R^{5**}$ und $R^{6**}$ unter Einschluß des Stickstoffatoms eine in 4-Postition durch eine Methylgruppe substituierte Piperazin-1-yl-gruppe und A** eine Methylengruppe bedeuten, sowie seine pharmakologisch verträglicken Säureadditionssalze.

Als Vertreter der erfindungsgemäßen Verbindungen seien beispielsweise genannt
4-[2-(Di-n-propylamino)-propionyl]-1,3-dimethyl-1,4,9,10-tetrahydropyrazolo[4,3-b][1,5]benzodiazepin-10-on,
4-[4-(Di-n-butylamino)-butyryl]-1,3-dimethyl-1,4,9,10-tetrahydropyrazolo-[4,3-b][1,5]benzodiazepin-10-on,
4-[2-(Diethylamino)-propionyl]-1,3-dimethyl-1,4,9,10-tetrahydropyrazolo[4,3-b][1,5]benzodiazepin-10-on,
4-[5-(Diisopropylamino)-valeryl]-1,3-dimethyl-1,4,9,10-tetrahydropyrazolo[4,3-b][1,5]benzodiazepin-10-on,
4-[Diisobutylaminoacetyl]-1,3-diethyl-1,4,9,10-tetrahydropyrazolo[4,3-b[1,5]benzodiazepin-10-on,
4-[N-n-Butyl-tert.-butylaminoacetyl]-1-ethyl-1,4,9,10-tetrahydropyrazolo[4,3-b][1,5]benzodiazepin-10-on,
4-[4-(Diallylamino)-butyryl]-1,3-dimethyl-1,4,9,10-tetrahydropyrazolo[4,3-b][1,5]benzodiazepin-10-on,
4-[Di-sek.-butylaminoacetyl]-1-ethyl-3-methyl-1,4,9,10-tetrahydropyrazolo[4,3-b][1,5]benzodiazepin-10-on,
4-[2-(N-Ethyl-n-butylamino)-propionyl]-1,3-dimethyl-1,4,9,10-tetrahydropyrazolo[4,3-b][1,5]benzodiazepin-10-on,
1,3-Dimethyl-4-[5-(N-methyl-n-butylamino)-valeryl]-1,4,9,10-tetrahydropyrazolo[4,3-b][1,5]benzodiazepin-10-on,
1-Methyl-4-[N-methyl-sek.-butylaminoacetyl]-1,4,9,10-tetrahydropyrazolo[4,3-b][1,5]benzodiazepin-10-on,
1,3-Dimethyl-4-[5-(N-methyl-tert.-butylamono)-valeryl]-1,4,9,10-tetrahydropyrazolo[4,3-b][1,5]benzodiazepin-10-on,
1,3-Dimethyl-4-[2-piperidinopropionyl]-1,4,9,10-tetrahydropyrazolo[4,3-b][1,5]benzodiazepin-10-on,
4-[4-(Hexahydroazepin-1-yl)-butyryl]-1,3-dimethyl-1,4,9,10-tetrahydropyrazolo[4,3-b][1,5]benzodiazepin-10-on,
4-[3-(Di-n-butylamino)-propionyl]-1,3-dimethyl-1,4,9,10-tetrahydropyrazolo[4,3-b][1,5]benzodiazepin-10-on,
4-[3-(Diallylamino)-propionyl]-1,3-dimethyl-1,4,9,10-tetrahydropyrazolo[4,3-b][1,5]benzodiazepin-10-on,
4-[3-(Di-sek.-butylamino)-propionyl-1,3-dimethyl-1,4,9,10-tetrahydropyrazolo[4,3-b][1,5]benzodiazepin-10-on,
4-[3-(N-n-Butyl-tert.-butylamino)-propionyl]-1,3-dimethyl-1,4,9,10-tetrahydropyrazolo[4,3-b][1,5]benzodiazepin-10-o-n,
4-[3-(N-Ethyl-n-butylamino)-propionyl]-1,3-dimethyl-1,4,9,10-tetrahydropyrazolo[4,3-b][1,5]benzodiazepin-10-on,
1,3-Dimethyl-4-[3-(N-methyl-sek.-butylamino)-propionyl]-1,4,9,10-tetrahydropyrazolo[4,3-b][1,5]benzodiazepin-10-on,
1,3-Dimethyl-4-[3-piperidinopropionyl]-1,4,9,10-tetrahydropyrazolo[4,3-b][1,5]benzodiazepin-10-on,
1-Methyl-4-[(4-methylpiperazin-1-yl)-acetyl]-3-propyl-1,4,9,10-tetrahydropyrazolo[4,3-b][1,5]benzodiazepin-10-on,
3-Butyl-1-methyl-4-[(4-methylpiperazin-1-yl)acetyl]-1,4,9,10-tetrahydropyrazolo[4,3-b][1,5]benzodiazepin-10-on,
4-[3-(Hexahydroazepin-1-yl)propionyl]-1,3-dimethyl-1,4,9,10-tetrahydropyrazolo[4,3-b][1,5]benzodiazepin-10-on,
4-[Bromacetyl]-1,3-dimethyl-1,4,9,10-tetrahydropyrazolo[4,3-b][1,5]benzodiazepin-10-on,
4-[Iodacetyl]-1,3-dimethyl-1,4,9,10-tetrahydropyrazolo[4,3-b][1,5]benzodiazepin-10-on,
4-[3-Brompropionyl]-1,3-dimethyl-1,4,9,10-tetrahydropyrazolo[4,3-b][1,5]benzodiazepin-10-on,
4-[3-Iodpropionyl]-1,3-dimethyl-1,4,9,10-tetrahydropyrazolo[4,3-b][1,5]benzodiazepin-10-on,
4-[Chloracetyl]-1,3-dimethyl-1,4,9,10-tetrahydropyrazolo[4,3-b][1,5]benzodiazepin-10-on,
bevorzugt
1,3-Dimethyl-4-[(4-methylpiperazin-1-yl)-acetyl]-1,4,9,10-tetrahydropyrazolo[4,3-n][1,5]benzodiazepin-10-on,
1,3-Dimethyl-1,4,9,10-tetrahydropyrazolo[4,3-b][1,5]benzodiazepin-10-on
und
4-Chloracetyl-1,3-dimethyl-1,4,9,10-tetrahydropyrazolo[4,3-b][1,5]benzodiazepin-10-on.

Die substituierten Pyrazolobenzodiazepinone der allgemeinen Formel I und ihre Säureadditionssalze bzw. die Ausgestaltungen I* und I** besitzen wertvolle Eigenschaften, die sie gewerblich verwertbar machen. Die substituierten Pyrazolobenzodiazepinone der allgemeinen Formel I, in der $R^3$ die Gruppe $-CO-A-N(R^5)R^6$ bedeutet und A, $R^5$ und $R^6$ die oben angegebenen Bedeutungen haben, bzw. die der Ausgestaltung I** sind durch eine ausgezeichnete Magen- und Darmschutzwirkung bei Warmblütlern gekennzeichnet, sie hemmen z.B. die Bildung von Magengeschwüren. Ferner weisen sie, bedingt durch eine geringe Toxizität und das Fehlen wesentlicher Nebenwirkungen eine günstige therapeutische Breite auf. Im übrigen haben die Verbindungen nur eine geringe anticholinerge Wirkung. Die substituierten Pyrazolobenzodiazepinone der allgemeinen Formel I, in der $R^3$ ein Wasserstoffatom oder die Gruppe -CO-A-Hal bedeutet und A und Hal die oben angegebenen Bedeutungen haben, bzw. die der Ausgestaltung I* sind wertvolle Zwischenprodukte bei der Herstellung der pharmakologisch wirksamen und therapeutisch einsetzbaren erfindungsgemäßen Verbindungen.

Die ausgezeichnete Wirksamkeit der pharmakologisch wirksamen substituierten Pyrazolobenzodiazepinone und ihrer pharmakologisch, d.h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden. Beispielsweise werden akuter und

chronischer Ulcus ventriculi und Ulcus duodeni, Gastritis oder hyperacider Reizmagen bei Mensch oder Tier behandelt.

Die erfindungsgemäßen pharmakologisch wirksamen Verbindungen werden daher zur Behandlung von Säugetieren, die an einer der oben genannten Krankheiten erkrankt sind, eingesetzt, wobei man dem erkrankten Säugetier eine therapeutisch wirksame und pharmakologisch verträgliche Menge einer oder mehrerer pharmakologisch wirksamer Verbindungen der allgemeinen Formeln I, I**, deren bevorzugter Vertreter und/oder deren Salze verabreicht. Gegenstand der Erfindung sind somit auch die erfindungsgemaßen pharmakologisch wirksamen Verbindungen zur Verwendung bei der Bekämpfung der oben angegebenen Krankheiten. Ebenso umfaßt die Erfindung die Verwendung erfindungsgemßer pharmakologisch wirksamer Verbindungen bei der Herstellung von Arzneimitteln, die zur Bekämpfung der angeführten Krankheiten eingesetzt werden.

Ein weiterer Gegenstand der Erfindung sind Arzneimittel, die ein oder mehrere pharmakologisch wirksame Pyrazolobenzodiazepinone der allgemeinen Formel I und/oder ihre pharmakologisch verträglichen Säureadditionssalze enthalten.

Ausgestaltungen der Arzneimittel sind solche, die Pyrazolobenzodiazepinone der Formel I** oder ihre bevorzugten Vertreter und/oder ihre pharmakologisch verträglichen Säureadditionssalze enthalten.

Die Arzneimittel werden nach an sich bekannten Verfahren hergestellt. Als Arzneimittel werden die erfindungsgemäßen Verbindungen entweder als solche oder vorzugsweise in Kombination mit geeigneten pharmazeutische Trägerstoffen eingesetzt. Enthalten die pharmazeutischen Zubereitungen neben den erfindungsgemäßen Verbindungen pharmazeutische Trägerstoffe, beträgt der Wirkstoffgehalt dieser Mischungen 0,5 bis 95, vorzugsweise 15 bis 75, Gewichtsprozent der Gesamtmischung. Die Arzneimittel werden beispielsweise für die orale, rektale oder parenterale (z.B. intravenöse) Gabe in geeigneten Dosen formuliert. Die Tagesdosis in der Humanmedizin liegt im allgemeinen bei oraler Gabe zwischen 0,01 bis 5 mg/kg, vorzugsweise 0,05 und 2,5, insbesondere 0,1 bis 1,5 mg/kg Körpergewicht. Eine Einzelgabe enthält den oder die Wirkstoffe in einer Menge von 0,01 bis 2,5, vorzugsweise 0,01 bis 1,5, insbesondere 0,05 bis 0,5 mg/kg Körpergewicht.

Die pharmazeutischen Zubereitungen bestehen bevorzugt aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigenz, als Farbstoff oder als Konservierungsmittel dienen.

Sollen die erfindungsgemäßen substituierten Pyrazolobenzodiapinone und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Sekretionshemmer, wie $H_2$-Blocker, z.B. Cimetidin; Magen- und Darmtherapeutika, z.B. Metoclopramid, Bromoprid, Tiaprid; Tranquilizer, wie Benzodiazepine, beispielsweise Diazepam; Spasmolytika, z.B. Bietamiverin, Camylofin; Anticholinergica, z.B. Oxyphencyclimin, Phencarbamid; Glucocorticoide, wie Prednisolon, Fluocortolon, Betamethason; nichtsteroidale Antiphlogistika, wie Arylessigsäuren und -propionsäuren, Heteroarylessigsäuren und -propionsäuren, Benzothiazincarboxamiddioxide, Pyrazolidindione, Chinazolinone, z.B. Ibuprofen, Naproxen, Diclofenac, Fenbufen, Indometacin, Lonazolac, Sudoxicam, Prioxicam, Phenylbutazon, Bumadizon-Calcium, Proquazon; Lokalanaesthetika, beispielsweise Tetracain, Procain; gegebenenfalls auch Fermente, Vitamine, Aminosäuren etc. enthalten.

Ein weiterer Gegenstand der Erfindung ist ein Verfahren zur Herstellung der substituierten Pyrazolobenzodiazepinone der allgemeinen Formel I, sowie ihrer Säureadditionssalze.

Das Verfahren ist dadurch gekennzeichnet, daß man Anilinopyrazolcarbonsäurederivate der allgemeinen Formel II

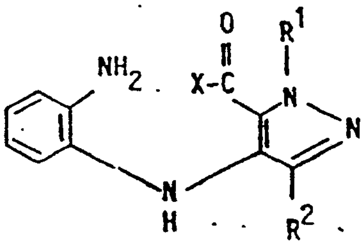 (II),

worin
X eine Fluchtgruppe bedeutet,
unter Abspaltung einer Verbindung HX cyclisiert und gegebenenfalls anschließend in dem erhaltenen Reaktionsprodukt der Formel I, worin $R^3$ ein Wasserstoffatom bedeutet, das Wasserstoffatom durch eine -CO-A-$R^4$-Gruppe substituiert und/oder erhaltene Basen in die Säureadditionssalze oder erhaltene Säureadditionssalze in die freie Base oder pharmakologisch verträgliche Särueadditionssalze überführt.

Vorzugsweise stellt X eine -OH-Gruppe, eine -O-$R^9$-Gruppe oder eine -N($R^{10}$)$R^{11}$-Gruppe dar, worin

$R^9$ eine Alkylgruppe, eine Cycloalkylgruppe, eine gegebenenfalls substituierte Aralkylgruppe oder einen gegebenenfalls substituierte Arylgruppe und
$R^{10}$ ein Wasserstoffatom, eine Alkylgruppe, eine Cycloalkylgruppe, eine gegebenenfalls substituierte Aralkylgruppe oder eine gegebenenfalls substituierte Arylgruppe bedeuten,
$R^{11}$ eine der Bedeutungen von $R^{10}$ hat oder
$R^{10}$ und $R^{11}$ gemeinsam unter Einschluß des Stickstoffatoms, an das sie gebunden sind, einen gegebenenfalls substituierten nichtaromatischen heterocyclischen Rest bedeuten.

Als Alkylreste $R^9$, $R^{10}$ und $R^{11}$ kommen geradkettige oder verzweigte Alkylgruppen mit 1 bis 11, vorzugsweise 1 bis 5, Kohlenstoffatomen in Betracht.

Als Cycloalkylgruppen kommen solche mit 3 bis 9, vorzugsweise 5 bis 8, Kohlenstoffatomen in Betracht.

Als Aralkylgruppen kommen solche mit Arylgruppen, die bis zu 12 Kohlenstoffatome enthalten, und Alkylgruppen, die 1 bis 4 Kohlenstoffatome enthalten, in Frage, von denen die mit 6 Kohlenstoffatomen im Arylrest und 1 bis 4 Kohlenstoffatomen im Alkylrest, vor allem mit 1 Kohlenstoffatom im Alkylrest, bevorzugt sind. Beispielsweise seien genannt die Benzyl-, Phenethyl- und Phenylpropylgruppe, von denen die Benzylgruppe bevorzugt ist. Die Aralkylgruppen sind gegebenenfalls auch substituiert, von denen die im Arylrest monosubstituierten bevorzugt sind, unter anderem durch Halogenatome, wie Fluor-, Chlor- oder Bromatome, Alkyl- und/oder Alkoxygruppen mit 1 bis 4 Kohlenstoffatomen. Beispielsweise gennat seien die 4-Chlorbenzyl-, die 3-Chlorbenzyl-, die 4-Brombenzyl-, die 2-Fluorbenzyl-, die 4-Fluorbenzyl-, die 4-Methylbenzyl-, die 4-Methoxybenzylgruppe.

Als Arylreste, die gegebenenfalls substituiert sein können, kommen solche mit 6 bis 10 Kohlenstoffatomen in Frage, beispielsweise der Phenyl- oder Naphthylrest, insbesondere der Phenylrest. Die Arylreste können ferner in beliebiger Stellung, beispielsweise mit 2, vorzugsweise mit 1 Substituenten, substituiert sein, wobei die energetisch begünstigten Stellen bevorzugt sind. Als Substituenten seien u.a. genannt Halogenatome, beispielsweise Fluor und Brom, vorzugsweise Chlor, Alkyl- oder Alkoxygruppen mit jeweils 1 bis 4 Kohlenstoffatomen. Bespiele für substituierte Arylgruppen sind die 2-Chlorphenyl-, 3-Chlorphenyl- 4-Chlorphenyl-, 3-Bromphenyl-, 4-Bromphenyl-, 4-Fluorphenyl-, 3-Methylphenyl-, 4-Methylphenyl-, 3,4-Dichlorphenyl-, 3-Chlor-4-methylphenyl-, 4-Methoxyphenyl-, 4-Ethoxyphenyl-, 3,4-Dimethoxyphenyl-, Cumenyl-, und 4-Chlor-1-naphthylgruppe, von denen die durch Halogenatome substituierten Phenylgruppen bevorzugt sind.

Aryl- und Aralkylgruppen können auch Heteroatome im Ring, beispielsweise Stickstoff-, Sauerstoff- ode Schwefelatome tragen. Beispielsweise seien genannt der 3-Pyridyl-, der 2-(-1-Phenyl-4-pyrazolyl)-ethyl- und der 2-Methyl-5-benzothiazolylrest.

Als nichtaromatische heterocyclische Reste kommen solche in Betracht, die sich von gesättigten und ungesättigten Heterocyclen ableiten. Beispielsweise seien genannt die Morpholinogruppe; die Thiomorpholinogruppe; die Pyrrolidinogruppe; die Piperidinogruppe; die Piperazinogruppe; die Hexahydroazepin-1-yl-gruppe; die Hexahydro-1H-1,4-diazepin-1-yl-gruppe; eine 4-Alkyl- oder 4-Aralkylpiperazin-1-yl-gruppe; wie die 4-Methyl- oder 4-Ethyl- oder 4-Benzylpiperazin-1-yl-gruppe; eine Dialkylpiperazin-1-yl-gruppe, wie die 2,4-Dimethylpiperazin-1-yl-gruppe; eine 4-Alkyl- oder 4-Aralkyl-hexahydro-1H-1,4-diazepin-1-yl-gruppe; wie die 4-Methyl- oder 4-Ethyl- oder 4-Benzylhexahydro-1H-1,4-diazepin-1-yl-gruppe; die 1,2,3,6-Tetrahydropyrid-1-yl-gruppe.

Die Cyclisierung der Verbindungen der Formel II zu den erfindungsgemäßen Pyrazolobenzodiazepinonen der Formel I, in denen $R^3$ ein Wasserstoffatom bedeutet, wird nach an sich bekannten Verfahren durchgeführt. So erfolgt die Cyclisierung der Carbonsäuren II (X = -OH) beispielsweise durch Erwärmen, vorzugsweise in Gegenwart eines Protonen abgebenden Mittels, auf Temperaturen zwischen 90°C und 220°C, vorzugsweise 120°C bis 160°C, ohne oder vorzugsweise in Gegenwart eines inerten Lösungsmittels. Als Protonen abgebende Mittel kommen anorganischen Säuren und organische Säuren, beispielsweise Halogenwasserstoffsäuren, wie Chlorwasserstoffsäure, Phosphorsäure, p-Toluolsulfonsäure oder Essigsäure in Frage. Als inerte Lösungsmittel kommen wässerige oder nicht wässerige Medien, wie Toluol, Xylol, Chlorbenzol, o-Dichlorbenzol, Trichlorbenzol oder Diphenylether in Betracht. Die Abspaltung von Wasser in nicht-wässerigem Reaktionsmedium kann gegebenenfalls mit Hilfe eines Wasserabscheiders oder durch Zusatz eines Trockemittels, z.B. eines Molekularsiebs, erleichtert werden.

Die Cyclisierung kann auch mit Hilfe eines chemischen Kondensationsmittels vorgenommen werden, durch welches intermediär reaktive Säurederivate als Zwischenstufen, wie beispielsweise Säurehalogenide, gemischte Säureanhydride, O-Acylharnstoffe oder aktivierte Ester, gebildet werden. Als chemische Kondensationsmittel seien beispielsweise genannt Dicyclohexylcarbodiimid, Chlorameisensäureethylester, Chlorameisensäureisopropylester, Chloracetonitril, Phosphoroxidtrichlorid. Diese Cyclisierungsreaktion wird in einem inerten Lösungsmittel, beispielsweise Tetrahydrofuran, Dioxan, Dimethylformamid, N-Methylpyrrolidon bei Temperaturen von −10°C bis 120°C, vorzugsweise 0°C bis 60°C, gegebenenfalls in Gegenwart einer Hilfsbase, z.B. von wasserfreiem Alkalimetallcarbonat, Triethylamin oder Pyridin, durchgeführt.

Im Falle de Carbonsäureester II (X = -0-$R^9$) oder der Carboxamide II [X = -N($R^{10}$)$R^{11}$] wird die Cyclisierung bei Temperaturen zwischen 0°C und 200°C, vorzugsweise 20 und 160°C, ohne oder in Gegenwart eines inerten Lösungsmittels, gegebenenfalls in Gegenwart eines basischen oder vorzugsweise sauren Katalysators durchgeführt. Die Reaktionszeiten betragen 15 Minuten bis 4 Stunden. Als Lösungsmittel kommen beispielsweise Alkohole, wie Ethanol oder Glykol; Ether, wie Dioxan ode Diphenylether; aromatische Kohlenwasserstoffe, wie Toluol, Xylol oder o-Dichlorbenzol; oder Dimethylsulfoxid in Frage. Als Katalysatoren seien genannt basische Katalysatoren, wie Alkalimetallalkonolate, z.B. Natriumethylat oder Kalium-tert.-butanolat, oder vorzugsweise

Natriumhydrid; oder saure Katalysatoren, wie organische oder anorganische Säuren, z.B. Essigsäure, Chloressigsäure, p-Toluolsulfonsäure, o-Chlorbenzoesäure, p-Tolylsäure, Nikotinsäure, Trifluoressigsäure, Fumarsäure, Chlorwasserstoffsäure, Phosphorsäure ode Kaliumhydrogensulfat, vorzugsweise Benzoesäure, wobei bis zu 2–3 Mol saurer Katalysator je Mol Ausgangsverbindung eingestzt werden. Wird der Aminoester der allgemeinen Formel II ($X = -O-R^9$) durch Reduktion einer Nitroverbindung der allgemeinen Formel VI hergestellt, wird bei geeigneten Reaktionsbedingungen direkt der Tricyclus der allgemeinen Formel I ($R^3 = -H$) gebildet. Bevorzugt wird die Cyclisierung mit den Aminoverbindungen II [$X = -N(R^{10})R^{11}$] durchgeführt.

Die sich gegebenenfalls anschließende Substitution erfolgt nach an sich bekannten Methoden.

Zur Herstellung der Pyrazolbenzodiazepinone der allgemeinen Formel I, in der $R^3$ eine Gruppe CO-A-Hal bedeutet, werden das erhaltene Reaktionsprodukt der Formel I, worin $R^1$ und $R^2$ die oben angegebenen Bedeutungen haben und $R^3$ ein Wasserstoffatom bedeutet, oder seine Säureadditionssaltze mit Verbindungen der allgemeinen Formeln III Hal-A-CO-Hal' (III) oder IV $[Hal-A-CO]_2O$ (IV), worin Hal' eine der Bedeutungen vol Hal hat und A und Hal die oben angegebenen Bedeutungen haben, umgesetzt. Diese Umsetzung wird ohne oder vorzugsweise in einem inerten Lösungsmittel bei Raumtemperatur oder erhöhter Temperatur, maximal bei der Siedetemperatur des Lösungsmittels, gegebenenfalls in Gegenwart einer Hilfsbase und/oder eines Acylierungskatalysators, vorgenommen. Die Säurehalogenide III sind gegenüber den Säureanhydriden IV bevorzugt. Als Säurehalogenid III ist Chloracetylchlorid, als Säureanhydrid IV ist Chloressigsäureanhydrid bevorzugt. Als Lösungsmittel seien beispielsweise genannt aromatische Kohlenwasserstoffe, wie Toluol, Xylol oder Chlorbenzol; offenkettige oder cyclische Ether, wie Diisopropylether oder Dioxan; chlorierte Kohlenwasserstoffe, wie Dichlorethan, andere Lösungsmittel, wie Pyridin, Acetonitril oder Dimethylformamid.

Als Hilfsbasen seien z.B. tertiäre organische Basen, wie Triethylamin und Ethyldiisopropylamin, oder Pyridin; oder anorgansiche Basen wie wasserfreie Alkalimetall- oder Erdalkalimetallcarbonate oder -hydrogencarbonate oder Erdalkalimetalloxide, genannt. Als Acylierungskatalysatoren kommen beispielsweise in Frage Imidazol, Pyridin oder 4-Dimethylaminopyridin.

Das Verfahren zur Herstellung der Zwischenprodukte der allgemeinen Formel I ist also dadurch gekennzeichnet, daß man ein Anilinopyrazolderivat der allgemeinen Formel II cyclisiert und gegebenenfalls anschließend durch Verbindungen der allgemeinen Formeln III oder IV substituiert und/oder die erhaltene Base oder deren Säureadditionssalze ineinander überführt. Bei der Herstellung der Zwischenprodukte der allgemeinen Formel I* werden entsprechende Ausgangsstoffe eingesetzt.

Zur Herstellung der Pyrazolobenzodiazepinone der allgemeinen Formel I, in der $R^1$ und $R^2$ die oben angegebene Bedeutung haben und $R^3$ eine Gruppe $-CO-A-N(R^5)R^6$ bedeutet, wird das erhaltene Reaktionsprodukt der Formel I, worin $R^3$ eine Gruppe -CO-A-Hal bedeutet, mit sekundären Aminen der allgemeinen Formel V $HN(R^5)R^6$ (V) umgesetzt, wobei $R^5$, $R^6$, A und Hal die obige Bedeutung haben.

Die Umsetzung erfolgt in einem inerten Lösungsmittel bei Temperaturen zwischen 0° und Siedetemperatur des Lösungsmittels entweder mit wenigstens 2 Molen sekundärem Amin V oder mit 1 bis 2 Molen sekundärem Amin V und einer Hilfsbase. Als Lösungsmittel kommen beispielsweise in Frage chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone, wie Aceton; Acetonitril oder Dimethylformamid. Als Hilfsbase seien beispielsweise genannt tertiäre organische Basen, wie Triethylamin, N-Methylpiperidin, Diethylanilin oder Pyridin, oder anorganische Basen, wie Alkalimetall- oder Erdalkalimetallcarbonate oder -hydrogencarbonate, Erdalkalimetalhydroxide oder -oxide. Gegebenenfalls kann die Reaktion durch Zusatz von Alkalimetalliodiden beschleunigt werden. Die Reaktionszeiten betragen je nach Menge und Art des eingesetzten Amins V zwischen 15 Minuten und 80 Stunden. Bei Umsetzung von Ausgansverbindungen, in denen A eine Alkylengruppe mit 2 bis 5 Kohlenstoffatomen darstellt, kann die Reaktion auch unter Abspaltung von H-Hal verlaufen; die intermediär gebildete, ggf. isolierbare Alkenylverbindung reagiert mit dem sekundären Amin V zum gleichen Endprodukt.

Das Verfahren zur Herstellung der pharmakologisch wirksamen Pyrazolobenzodiazepinone der allgemeinen Formel I ist also dadurch gekennzeichnet, daß man Verbindungen der Formel I, worin $R^3$ eine Gruppe -CO-A-Hal bedeutet, mit Verbindungen der allgemeinen Formel V umsetzt und gegebenenfalls anschließend die erhaltene Base in ein pharmakologisch verträgliches Säureadditionssalz oder ein erhaltenes Säureadditionssalz in die freie Base oder ein pharmakologisch verträgliches Säureadditionssalz überführt.

Säureadditionssalze erhält man durch Auflösen der erhaltenen freien Base in einem geeigneten Lösungsmittel, z.B. Wasser, Aceton, einem Alkanol, wie Ethanol oder Isopropanol, einem offenkettigen oder cyclischen Ether, wie Diethylether oder Tetrahydrofuran, das die gewünschte Säure enthält oder dem die gewünschte Säure anschließend zugegeben wird. Die Salze werden durch Filtrieren, Ausfällen mit einem Nichtlösungsmittel für das Säureadditionssalz oder durch Verdampfen des Lösungsmittels gewonnen. Salze können auch durch Überführung in die Base und weitere Umsetzung mit einer anderen Säure in andere Salze, z.B. pharmakologisch verträgliche Säureadditionssalze, übergeführt werden.

Erhaltene Salze können z.B. durch Alkalisieren mit väßrigem Natrium- oder Kaliumhydroxid in die freie Base umgewandelt werden, die dann durch geeignete Maßnahmen, z.B. Lösungsmittelextraktion mit einem nicht mit Wasser mischbaren Lösungsmittel, wie Chloroform, Diethylether, Toluol, gewonnen wird.

Die Herstellung der Ausgangsverbindungen der allgemeinen Formel II erfolgt durch Reduktion von Nitroverbindungen der allgemeinen Formel VI

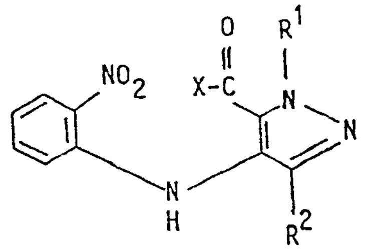 (VI),

worin $R^1$, $R^2$ und X die oben angeführte Bedeutung haben.

Die Reduktion der Nitroverbindungen erfolgt mittels üblicher Methoden, beispielsweise mit Natriumdithionit, Hydrazinhydrat und Raney-Nickel oder mit Wasserstoff in Gegenwart von Katalysatoren, wie Raney-Nickel oder Palladium auf Kohle, drucklos oder bei erhöhtem Druck, bei Raumtemperatur oder erhöhter Temperatur, in den üblichen Lösungsmitteln, wie Wasser, Alkoholen, Ethern oder Eisessig, gegebenenfalls in Gegenwart von Mineralsäuren, wie Chlorwasserstoffsäure oder Perchlorsäure.

Die Herstellung der Anilinopyrazolcarbonsäuren der allgemeinen Formel II (X = -OH) erfolgt alternativ aus den entsprecheneden Säurederivaten der allgemeinen Formel II [X = $-O-R^9$ oder $-N(R^{10})R^{11}$] durch Hydrolyse im sauren oder alkalischen Milieu nach an sich bekannten Methoden.

Die Herstellung von Ausgangverbindungen der allgemeinen Formel VI (X = -OH) wird durch Hydrolyse von Estern oder Amiden der allgemeinen Formel VI [X = $-O-R^9$ bzw. $-N(R^{10})R^{11}$] in saurem oder alkalischem Milieu unter üblichen Bedingungen vorgenommen. Vice versa erhält man die Ester oder Amide der allgemeinen Formel VI [X = $-O-R^9$ bzw. $-N(R^{10})R^{11}$] aus den Säuren der allgemeinen Formel VI (X = -OH) nach üblichen Methoden. z.B. durch Überführung der Säure in ein Säurehalogenid und dessen Umsetzung mit $R^9$-OH, z.B. einem entsprechenden Alkohol oder Phenol bzw. dem entsprechenden Alkoholat oder Phenolat, oder einem Amin $HN(R^{10})R^{11}$.

Die Ester der allgemeinen Formel VI, insbesondere die Alkylester, bzw. die Amide der allgemeinen Formel VI [X = $-O-R^9$ bzw. $-N(R^{10})R^{11}$] werden durch Umsetzung von Aminopyrazolcarbonsäureestern der allgemeinen Formel VII bzw. Aminopyrazolcarbonsäureamiden der allgemeinen Formel VII [X = $-O-R^9$ bzw.$-N(R^{10})R^{11}$] mit o-Halogennitrobenzolen, z.B. mit o-Bromnitrobenzol under Zusatz von Kupferkatalysatoren oder mit o-Chlornitrobenzol, vorzugsweise o-Fluornitrobenzol oder einem Gemisch aus o-Chlornitrobenzol und Fluornitrobenzol, wie se bei der Umsetzung von o-Chlornitrobenzol mit Kaliumfluorid entsteht, unter Zusatz eines Deprotonierungsmittels, z.B. Natriumhydrid, Kaliumcarbonat oder eines tertiären Amins, und gegebenenfalls eines Kronenethers, erhalten.

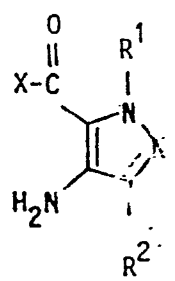 (VII)

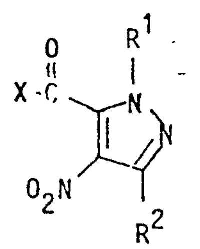 (VIII)

Die Verbindungen der allgemeinen Formel VII werden durch Reduktion von Verbindungen der allgemeinen Formel VIII nach üblichen Methoden dargestellt. Verbindungen der allgemeinen Formel VIII werden aus den entsprechenden nach literaturbekannten oder analogen Methoden hergestellten nichtnitrierten Pyrazolcarbonsäuren durch Nitrierung und gegebenenfalls deren Umwandlung in Ester oder Amide hergestellt. Unsubstituierte Carboxamide der allgemeinen Formel VIII werden durch Hydrolyse entsprechender Nitrile erhalten, die nach literaturbekannten Verfahren aus entsprechenden 5-Halogen-pyrazolen und Cyaniden hergestellt werden. Herstellungsverfahren für Ausgangsprodukte sind z.B. beschrieben in: H.A.De Wald et al., J. Med. Chem. 16, 1346(1973; US-PS 3,657,271; UP-PS 3,939,161; C. Musante, Gazz. chim.ital. **75**, 121-136(1945); C.A. Rojahn, Ber.Dt.Chem.Ges. **59**, 607-611 (1926); L.B. Townsend et al., J. Rog. Chem. **39**, 2023-2027 (1974); DE-OS 22 50 316; UP-PS 3,553,209.

Zur Herstellung der Verbindungen I* bzw. I** werden entsprechende Ausgangsverbindungen II*, II**, III*, III**, IV*, IV**, V** eingesetzt.

Die folgenden Beispiele dienen zur näheren Erläuterung der Erfindung. "F." bedeutet "Schmelzpunkt"; "Kp." bedeutet "Siedepunkt"; "Zers." steht für "under Zersetzung".

**Beispiele**

**Beispiel 1**

3,5 g 4-Chloracetyl-1,3-dimethyl-1,4,9,10-tetrahydropyrazolo[4,3-b][1,5]bezodiazepin-10-on, 8,1 g N-Methylpiperazin und 50 ml Toluol werden 2 Stunden bei 80°C gerührt. Man versetzt mit 60 ml verdünnter Natriumhydrogencarbonatlösung, trennt die Schichten, schüttelt die wässerige Phase noch mehrmals mit Toluol aus und engt sie im Vakuum zur Trockne ein. Den Rückstand rührt man mit 100 ml Isopropanol, filtriert und engt das Filtrat im Vakuum ein. Den Rückstand (4,0 g) reinigt man durch Rühren mit Diethylether und Umkristallisieren aus Toluol und erhält 2,2 g 1,3-Dimethyl-4-[(4-methylpiperazin-1-yl)acetyl]-1,4,9,10-tetrahydropyrazolo[4,3-b][1,5]benzodiazepin-10-on, F. 186–188°C.

**Beispiel 2**

Man rührt 15,0 g 4-Chloracetyl-1,3-dimethyl-1,4,9,10-tetrahydropyrazolo[4,3-b][1,5]benzodiazepin-10-on, 21 g N-Methylpiperazin und 70 ml Dioxan 1 Stunde bei 80°C, wobei das Dünnschichtchromatogramm bereits nach 20 Minuten vollständige Umsetzung anzeigt, und engt die Lösung im Vakuum zur Trockne ein. Man versetzt den Rückstand mit 150 ml Isopropanol und 40 ml Wasser, tropft 25 ml konzentrierte Salzsäure zu, kühlt im Eisbad und erhält 1,3-Dimethyl-4-[(4-methylpiperazin-1-yl)acetyl]-1,4,9,10-tetrahydropyrazolo[4,3-b][1,5]benzodiazepin-10-on-dihydrochlorid im Gemisch mit N-Methylpiperazinhydrochlorid. Man löst die Hydrochloride in Wasser und Chloroform, stellt mit 2n Natronlauge auf pH 8,2, schüttelt die wässerige Phase erschöpfend mit Chloroform aus, trocknet die organische Lösung und engt sie im Vakuum zur Trockne ein. Man erhält 17,6 g 1,3-Dimethyl-4-[(4-methylpiperazin-1-yl)acetyl]-1,4,9,10-tetrahydropyrazolo[4,3-b][1,5]benzodiazepin-10-on, F. 186–188°C (aus Toluol). Dihydrochlorid F. 222–224°C (Zers.), Hemifumarat F. 157–258°C (Zers.); Succinat F. 172–174°C.

Analog erhält man
1,3-Dimethyl-4-(morpholinoacetyl)-1,4,9,10-tetrahydropyrazolo[4,3-b][1,5]benzodiazepin-10-on (F. 201–203°C),
4-[(4-Benzylpiperazin-1-yl)acetyl]-1,3-dimethyl-1,4,9,10-tetrahydropyrazolo[4,3-b][1,5]benzodiazepin-10-on (F. 215–218°C),
4-[(4-Ethylpiperazin-1-yl)acetyl]-1,3-dimethyl-1,4,9,10-tetrahydropyrazolo[4,3-b][1,5]benzodiazepin-10-on,
4-[(2,4-Dimethyl-piperazin-1-yl)acetyl]-1,3-dimethyl-1,4,9,10-tetrahydropyrazolo[4,3-b][1,5]benzodiazepin-10-on,
4- ]N-(2-Dimethylaminoethyl)-N-methylamino]acetyl -1,3-dimethyl-1,4,9,10-tetrahydropyrazolo[4,3-b][1,5]benzodiazepin-10-on (F. 84–86°C),
4- [N-(2-Diethylaminoethyl)-N-ethylamino]acetyl -1,3-dimethyl-1,4,9,10-tetrahydropyrazolo[4,3-b][1,5]benzodiazepin-10-on (F. 139,5–141°C),
4- [N-(2-Dimethylaminoethyl)-N-ethylamino]acetyl -1,3-dimethyl-1,4,9,10-tetrahydropyrazolo[4,3-b][1,5]benzodiazepin-10-on (F. 93–95,5°C),
4-[(Hexahydro-4-methyl-1H-1,4-diazepin-1-yl)acetyl]-1,3-dimethyl-1,4,9,10-tetrahydropyrazolo[4,3-b][1,5]benzodiazepin-10-on
durch Umsetzung von 4-Chloracetyl-1,3-dimethyl-1,4,9,10-tetrahydropyrazolo[4,3-b][1,5]benzodiazepin-10-on mit Morpholin, N-Benzylpiperazin, N-Ethylpiperazin, 1,3-Dimethylpiperazin, N,N,N'-Trimethyl-ethylendiamin, N,N,N'-triethylethylendiamin, N'-Ethyl-N,N-dimethylethylendiamin bzw. Hexahydro-1-methyl-1H-1,4-diazepin.

**Beispiel 3**

Man erhitzt 2,0 g 4-Chloacetyl-1,3-dimethyl-1,4,9,10-tetrahydropyrazolo[4,3-b][1,5]benzodiazepin-10-on, 0,55 g Pyrrolidin, 0,85 g gemahlenes Natriumcarbonat und 15 ml absolutes Ethanol 2 Stunden zum Sieden, filtriert die heiße Lösung und engt im Vakuum ein. Man löst in Dichlormethan, wäscht die organische Lösung bei pH 7 mit Wasser, engt sie ein und erhält 2,05 g 1,3-Dimethyl-4-(pyrrolidinoacetyl)-1,4,9,10-tetrahydropyrazolo[4,3-b][1,5]benzodiazepin-10-on, F. 114–116°C (aus Toluol).

**Beispiel 4**

Man rührt 2 g 4-Chloracetyl-1,3-dimethyl-1,4,9,10-tetrahydropyrazolo[4,3-b][1,5]benzodiazepin-10-on, 3,7 g Piperidin und 15 ml Dioxan 1 Stunde bei 80°C, engt im Vakuum ein und kristallisiert den Rückstand aus Isopropanol/Wasser und Toluol/Petrolether um. Man erhält 2,0 g 1,3-Dimethyl-4-(piperidinoacetyl)-1,4,9,10-tetrahydropyrazolo[4,3-b][1,5]benzodiazepin-10-on, F. 156–158°C.

Analog erhält man
1,3-Dimethyl-4-(pyrrolidinoacetyl)-1,4,9,10-tetrahydropyrazolo[4,3-b][1,5]benzodiazepin-10-on (F. 114–116°C),
4-(Diethylaminoacetyl)-1,3-dimethyl-1,4,9,10-tetrahydropyrazolo[4,3-b][1,5BSbenzodiazepin-10-on (F. 142,5–144°C),
4-(Di-n-propylaminoacetyl)-1,3-dimethyl-1,4,9,10-tetrahydropyrazolo[4,3-b][1,5]benzodiazepin-10-on,
4-(Di-n-butylaminoacetyl)-1,3-dimethyl-1,4,9,10-tetrahydropyrazolo[4,3-b][1,5]benzodiazepin-10-on,
4-(N-Ethyl-n-butylaminoacetyl)-1,3-dimethyl-1,4,9,10-tetrahydropyrazolo[4,3-b][1,5]benzodiazepin-10-on,
4-(Diallylaminoacetyl)-1,3-dimethyl-1,4,9,10-tetrahydropyrazolo[4,3-b][1,5]benzodiazepin-10-on,
4-[(Hexahydroazepin-1-yl)acetyl]-1,3-dimethyl-1,4,9,10-tetrahydropyrazolo[4,3-b][1,5]benzodiazepin-10-on,
1,3-Dimethyl-4-[(N-methyl-n-butylamino)acetyl]-1,4,9,10-tetrahydropyrazolo[ 4,3-b][1,5]benzodiazepin-10-on
durch analoge Umsetzung von 4-Chloracetyl-1,3-dimethyl]-1,4,9,10-tetrahydropyrazolo[4,3-b][1,5] zodiazepin-10-on

mit Pyrrolidin, Diethylamin, Di-n-propylamin, Di-n-butylamin, N-Ethyl-n-butylamin, Diallylamin, Hexahydroazepin bzw. N-Methyl-n-butylamin.

**Beispiel 5**

Man rührt 2,0 g 4-Chloracetyl-1,3-dimethyl-1,4,9,10-tetrahydropyrazolo[4,3-b][1,5]benzodiazepin-10-on, 5,6 ml 40 %ige wässerige Dimethylaminlösung und 10 ml Dichlormethan 2 Stunden bei 35°C, versetzt mit 0,35 g Natriumcarbonat und engt im Vakuum zur Trockne ein. Man versetzt mit wenig Wasser, schüttelt die Lösung wiederholt mit Chloroform aus, trocknet die organische Lösung mit Natriumsulfat und engt sie zur Trockne ein. Man erhält 1,9 g 4-(Dimethylaminoacetyl)-1,3-dimethyl-1,4,9,10-tetrahydropyrazolo[4,3-b][1,5]benzodiazepin-10-on, F. 178–179°C (aus Toluol).

**Beispiel 6**

Man rührt 2,0 g 4-Chloracetyl-1,3-dimethyl-1,4,9,10-tetrahydropyrazolo[4,3-b][1,5]benzodiazepin-10-on, 9 ml Diisopropylamin, 100 mg Kaliumiodid und 15 ml Dioxan 70 Stunden unter Rückfluß und engt im Vakuum die Lösung ein. Man versetzt mit Wasser und verdünnter Salzsäure bis pH 4, schüttelt die Lösung mit Ethylmethylketon aus, klärt mit Aktivkohle und fällt aus der wässerigen Lösung unter Kühlen mit Eis mit verdünnter Natronlauge bei pH 9,5 farblose Kristalle aus, die aus Petrolether (Kp. 50–70°C)/Essigsäureethylester (1:1) umkristallisiert werden. Man erhält 1,8 g 4-(Diisopropylaminoacetyl)-1,3-dimethyl-1,4,9,10-tetrahydropyrazolo[4,3-b][1,5]benzodiazepin-10-on, F. 154–156°C.

Analog erhält man
4-(N-n-Butyl-tert.-butylaminoacetyl)-1,3-dimethyl-1,4,9,10-tetrahydropyrazolo[4,3-b][1,5]benzodiazepin-10-on,
4-(Diisobutylaminoacetyl)-1,3-dimethyl-1,4,9,10-tetrahydropyrazolo[4,3-b][1,5]benzodiazepin-10-on,
4-(Di-sek.-butylaminoacetyl)-1,3-dimethyl-1,4,9,10-tetrahydropyrazolo[4,3-b][1,5]benzodiazepin-10-on,
1,3-Dimethyl-4-(N-methyl-tert.-butylaminoacetyl)-1,4,9,10-tetrahydropyrazolo[4,3-b][1,5]benzodiazepin-10-on,
1,3-Dimethyl-4-(N-methyl-sek.-butylaminoacetyl)-1,4,9,10-tetrahydropyrazolo[4,3-b][1,5]benzodiazepin-10-on durch Umsetzung von 4-Chloracetyl-1,3-dimethyl-1,4,9,10-tetrahydropyrazolo[4,3-b][1,5]benzodiazepin-10-on mit N-n-Butyl-tert.-butylamin, Diisobutylamin, Di-sek.-butylamin, N-Methyl-tert.-butylamin bzw. N-Methyl-sek.-butylamin.

**Beispiel 7**

3,5 g 4-Chloracetyl-1-ethyl-3-methyl-1,4,9,10-tetrahydropyrazolo[4,3-b][1,5]benzodiazepin-10-on, 4,4 g N-Methylpiperazin und 20 ml Dioxan werden entsprechend Beispiel 2 umgesetzt. Man erhält 3,0 g 1-Ethyl-3-methyl-4-[(4-methylpiperazin-1-yl)acetyl]-1,4,9,10-tetrahydropyrazolo[4,3-b][1,5]benzodiazepin-10-on, F. 145–146°C.

Analog erhält man durch Umsetzung von
4-Chloracetyl-1-ethyl-3-methyl-1,4,9,10-tetrahydropyrazolo[4,3-b][1,5]benzodiazepin-10-on mit 1-Ethylpiperazin bzw. 1,3-Dimethylpiperazin statt mit N-Methylpiperazin
1-Ethyl-4-[(4-ethylpiperazin-1-yl)acetyl]-3-methyl-1,4,9,10-tetrahydropyrazolo[4,3-b][1,5]benzodiazepin-10-on bzw.
4-[(2,4-Dimethyl-piperazin-1-yl)acetyl]-1-ethyl-3-methyl-1,4,9,10-tetrahydropyrazolo[4,3-b][1,5]benzodiazepin-10-on.

**Beispiel 8**

Analog Beispiel 2 erhält man
4-[(4-Ethylpiperazin-1-yl)acetyl]-1-isopropyl-3-methyl-1,4,9,10-tetrahydropyrazolo[4,3-b][1,5]benzodiazepin-10-on,
3-Ethyl-4-[(4-ethylpiperazin-1-yl)acetyl]-1-methyl-1,4,9,10-tetrahydropyrazolo[4,3-b][1,5]benzodiazepin-10-on,
4-[(4-Ethylpiperazin-1-yl)acetyl]3-isopropyl-1-methyl-1,4,9,10-tetrahydropyrazolo[4,3-b[1,5]benzodiazepin-10-on,
4-[(4-Ethylpiperazin-1-yl)acetyl]-1-methyl-1,4,9,10-tetrahydropyrazolo[4,3-b][1,5]benzodiazepin-10-on,
1-Ethyl-4-[(4-ethylpiperazin-1-yl)acetyl]-1,4,9,10-tetrahydropyrazolo[4,3-b][1,5]benzodiazepin-10-on,
4-[(4-Ethylpiperazin-1-yl)acetyl]-1-isopropyl-1,4,9,10-tetrahydropyrazolo[4,3-b][1,5]benzodiazepin-10-on,
4-[3-(4-Ethylpiperazin-1-yl)propionyl]-1,3-dimethyl-1,4,9,10-tetrahydropyrazolo[4,3-b][1,5]benzodiazepin-10-on,
4-[2-(4-Ethylpiperazin-1-yl)propionyl]-1,3-dimethyl-1,4,9,10-tetrahydropyrazolo[ 4,3-b][1,5]benzodiazepin-10-on
4-[(2,4-Dimetyl-piperazin-1-yl)acetyl]-1-isopropyl-3-methyl-1,4,9,10-tetrahydropyrazolo[4,3-b][1,5]benzodiazepin-10--on,
4-[(2,4-Dimethyl-piperazin-1-yl)acetyl]-3-ethyl-1-methyl-1,4,9,10-tetrahydropyrazolo[4,3-b][1,5]benzodiazepin-10-on,
4-[(2,4-Dimethyl-piperazin-1-yl)acetyl]-3-isopropyl-1-methyl-1,4,9,10-tetrahydropyrazolo[4,3-b][1,5]benzodiazepin-10--on,
4-[(2,4-Dimethyl-piperazin-1-yl)acetyl]-1-methyl-1,4,9,10-tetrahydropyrazolo[4,3-b][1,5]benzodiazepin-10-on,
4-[(2,4-Dimethyl-piperazin-1-yl)acetyl]-1-ethyl-1,4,9,10-tetrahydropyrazolo[4,3-b][1,5]benzodiazepin-10-on,
4-[(2,4-Dimethyl-piperazin-1-yl)acetyl]3-isopropyl-1,4,9,10-tetrahydropyrazolo[4,3-b][1,5]benzodiazepin-10-on,
4-[3-(2,4-Dimethyl-piperazin-1-yl)propionyl]-1,3-dimethyl-1,4,9,10-tetrahydropyrazolo[4,3-b][1,5]benzodiazepin-10-on,
4-[2-(2,4-Dimetyl-piperazin-1-yl)propionyl]-1,3-dimethyl-1,4,9,10-tetrahydropyrazolo[4,3-b][1,5]benzodiazepin-10-on durch Umsetzung von
4-Chloracetyl-1-isopropyl-3-methyl-1,4,9,10-tetrahydropyrazolo[4,3-b][1,5]benzodiazepin-10-on,
4-Chloracetyl-3-ethyl-1-methyl-1,4,9,10-tetrahydropyrazolo[4,3-b][1,5]benzodiazepin-10-on,

4-Chloracetyl-3-isopropyl-1-methyl-1,4,9,10-tetrahydropyrazolo[4,3-b][1,5]benzodiazepin-10-on
4-Chloracetyl-1-methyl-1,4,9,10-tetrahydropyrazolo[4,3-b][1,5]benzodiazepin-10-on
4-Chloracetyl-1-ethyl-1,4,9,10-tetrahydropyrazolo[4,3-b][1,5]benzodiazepin-10-on
4-Chloracetyl-1-isopropyl-1,4,9,10-tetrahydropyrazolo[4,3-b][1,5]benzodiazepin-10-on
4-(3-Chlorpropionyl)-1,3-dimethyl-1,4,9,10-tetrahydropyrazolo[4,3-b][1,5]benzodiazepin-10-on bzw.
4-(2-Chlorpropionyl)-1,3-dimethyl-1,4,9,10-tetrahydropyrazolo[4,3-b][1,5]benzodiazepin-10-on
mit 1-Ethylpiperazin bzw. 1,3-Dimethylpiperazin.

**Beispiel 9**

Analog Beispiel 2 erhält man
1-Isopropyl-3-methyl-4-[(4-methyl-piperazin-1-yl)acetyl]-1,4,9,10-tetrahydropyrazolo[4,3-b][1,5]benzodiazepin-10-on,
3-Isopropyl-1-methyl-4-[(4-methyl-piperazin-1-yl)acetyl]-1,4,9,10-tetrahydropyrazolo[4,3-b][1,5]benzodiazepin-10-on,
3-Ethyl-1-methyl-4-[(4-methyl-piperazin-1-yl)acetyl]-1,4,9,10-tetrahydropyrazolo[4,3-b][1,5]benzodiazepin-10-on F. 214–216°C),
1-Methyl-4-[(4-methyl-piperazin-1-yl)acetyl]-1,4,9,10-tetrahydropyrazolo[4,3-b][1,5]benzodiazepin-10-on (F. 198,5–200,5°C),
1-Ethyl-4-[(4-methyl-piperazin-1-yl)acetyl]-1,4,9,10-tetrahydropyrazolo[4,3-b][1,5]benzodiazepin-10-on,
1-Isopropyl-4-[(4-methyl-piperazin-1-yl)acetyl]-1,4,9,10-tetrahydropyrazolo[4,3-b][1,5]benzodiazepin-10-on,
1,3-Dimethyl-4-[3-(4-methyl-piperazin-1-yl)propionyl]-1,4,9,10-tetrahydropyrazolo[4,3-b][1,5]benzodiazepin-10-on (F. 105–110°C),
3-Ethyl-1-methyl-4-[3-(4-methyl-piperazin-1-yl)propionyl]-1,4,9,10-tetrahydropyrazolo[4,3-b][1,5]benzodiazepin-10-on,
3-Isopropyl-1-methyl-4-[3-(4-methyl-piperazin-1-yl)propionyl]-1,4,9,10-tetrahydropyrazolo[4,3-b][1,5]benzodiazepin-10-on,
1-Methyl-4-[3-(4-methyl-piperazin-1-yl)propionyl]-1,4,9,10-tetrahydropyrazolo[4,3-b][1,5]benzodiazepin-10-on,
1-Ethyl-4-[3-(4-methyl-piperazin-1-yl)propionyl]-1,4,9,10-tetrahydropyrazolo[4,3-b][1,5]benzodiazepin-10-on,
1-Isopropyl-4-[3-(4-methyl-piperazin-1-yl)propionyl]-1,4,9,10-tetrahydropyrazolo[4,3-b][1,5]benzodiazepin-10-on,
1,3-Dimethyl-4[2-(4-methyl-piperazin-1-yl)propionyl]-1,4,9,10-tetrahydropyrazolo[4,3-b][1,5]benzodiazepin-10-on,
3-Ethyl-1-metyl-4-[2-(4-methyl-piperazin-1-yl)propionyl]-1,4,9,10-tetrahydropyrazolo[4,3-b][1,5]benzodiazepin-10-on,
3-Isopropyl-1-methyl-4-[2-(4-methyl-piperazin-1-yl)propionyl]-1,4,9,10-tetrahydropyrazolo[4,3-b][1,5]benzodiazepin-10-on,
1-Methyl-4-[2-(4-methyl-piperazin-1-yl)propionyl]-1,4,9,10-tetrahydropyrazolo[4,3-b][1,5]benzodiazepin-10-on,
1-Ethyl-4-[2-(4-methyl-piperazin-1-yl)propionyl]-1,4,9,10-tetrahydropyrazolo[4,3-b][1,5]benzodiazepin-10-on,
1-Isopropyl-4-[2-(4-methyl-piperazin-1-yl)propionyl]-1,4,9,10-tetrahydropyrazolo[4,3-b][1,5]benzodiazepin-10-on,
1,3-Dimethyl-4-[4-(4-methyl-piperazin-1-yl)butyryl]-1,4,9,10-tetrahydropyrazolo[4,3-b][1,5]benzodiazepin-10-on,
1-Methyl-4-[4-(4-methyl-piperazin-1-yl)butyryl]-1,4,9,10-tetrahydropyrazolo[4,3-b][1,5]benzodiazepin-10-on,
1-Ethyl-4-[4-(4-methyl-piperazin-1-yl)butyryl]-1,4,9,10-tetrahydropyrazolo[4,3-b][1,5]benzodiazepin-10-on,
1,3-Dimethyl-4-[5-(4-methyl-piperazin-1-yl)valeryl]-1,4,9,10-tetrahydropyrazolo[4,3-b][1,5]benzodiazepin-10-on,
1-Methyl-4-[5-(4-methyl-piperazin-1-yl)valeryl]-1,4,9,10-tetrahydropyrazolo[4,3-b][1,5]benzodiazepin-10-on,
1-Ethyl-4-[5-(4-methyl-piperazin-1-yl)valeryl]-1,4,9,10-tetrahydropyrazolo[4,3-b][1,5]benzodiazepin-10-on,
durch Umsetzung von
4-Chloracetyl-1-isopropyl-3-methyl-1,4,9,10-tetrahydropyrazolo[4,3-b][1,5]benzodiazepin-10-on,
4-Chloracetyl-3-isopropyl-1-methyl-1,4,9,10-tetrahydropyrazolo[ 4,3-b][1,5]benzodiazepin-10-on,
4-Chloracetyl-3-ethyl-1-methyl-1,4,9,10-tetrahydropyrazolo[4,3-b][1,5]benzodiazepin-10-on,
4-Chloracetyl-1-methyl-1,4,9,10-tetrahydropyrazolo[4,3-b][1,5]benzodiazepin-10-on,
4-Chloracetyl-1-ethyl-1,4,9,10-tetrahydropyrazolo[4,3-b][1,5]benzodiazepin-10-on,
4-Chloracetyl-1-isopropyl-1,4,9,10-tetrahydropyrazolo[4,3-b][1,5]benzodiazepin-10-on,
4-(3-Chlorpropionyl)-1,3-dimethyl-1,4,9,10-tetrahydropyrazolo[4,3-b][1,5]benzodiazepin-10-on,
4-(3-Chlorpropionyl)-3-ethyl-1-methyl-1,4,9,10-tetrahydropyrazolo[4,3-b][1,5]benzodiazepin-10-on,
4-(3-Chlorpropionyl)-3-isopropyl-1-methyl-1,4,9,10-tetrahydropyrazolo[4,3-b][1,5]benzodiazepin-10-on,
4-(3-Chlorpropionyl)-1-methyl-1,4,9,10-tetrahydropyrazolo[4,3-b][1,5]benzodiazepin-10-on,
4-(3-Chlorpropionyl)-1-ethyl-1,4,9,10-tetrahydropyrazolo[4,3-b][1,5]benzodiazepin-10-on,
4-(3-Chlorpropionyl)-1-isopropyl-1,4,9,10-tetrahydropyrazolo[4,3-b][1,5]benzodiazepin-10-on,
4-(2-Chlorpropionyl)-1,3-dimethyl-1,4,9,10-tetrahydropyrazolo[ 4,3-b][1,5]benzodiazepin-10-on,
4-(2-Chlorpropionyl)-3-ethyl-1-methyl-1,4,9,10-tetrahydropyrazolo[4,3-b][1,5]benzodiazepin-10-on,
4-(2-Chlorpropionyl)-3-isopropyl-1-methyl-1,4,9,10-tetrahydropyrazolo[4,3-b][1,5]benzodiazepin-10-on,
4-(2-Chlorpropionyl)-1-methyl-1,4,9,10-tetrahydropyrazolo[4,3-b][1,5]benzodiazepin-10-on,
4-(2-Chlorpropionyl)-1-ethyl-1,4,9,10-tetrahydropyrazolo[4,3-b][1,5]benzodiazepin-10-on,
4-(2-Chlorpropionyl)-1-isopropyl-1,4,9,10-tetrahydropyrazolo[4,3-b][1,5]benzodiazepin-10-on,
4-(4-Chlorbutyryl)-1,3-dimethyl-1,4,9,10-tetrahydropyrazolo[4,3-b][1,5]benzodiazepin-10-on,
4-(4-Chlorbutyryl)-1-methyl-1,4,9,10-tetrahydropyrazolo[4,3-b][1,5]benzodiazepin-10-on,
4-(4-Chlorbutyryl)-1-ethyl-1,4,9,10-tetrahydropyrazolo[4,3-b][1,5]ben iazepin-10-on,
4-(5-Chlorvaleryl)-1,3-dimethyl-1,4,9,10-tetrahydropyrazolo[4,3-b][1,5]benzodiazepin-10-on,
4-(5-Chlorvaleryl)-1-methyl-1,4,9,10-tetrahydropyrazolo[4,3-b][1,5]benzodiazepin-10-on bzw.
4-(5-Chlorvaleryl)-1-ethyl-1,4,9,10-tetrahydropyrazolo[ 4,3-b][1,5]benzodiazepin-10-on

mit N-Methylpiperazin.

**Beispiel 10**

Analog Beispiel 5 erhält man
4-(Dimethylaminoacetyl)-1-ethyl-3-methyl-1,4,9,10-tetrahydropyrazolo[4,3-b][1,5]benzodiazepin-10-on,
4-(Dimethylaminoacetyl)-1-isopropyl-3-methyl-1,4,9,10-tetrahydropyrazolo[4,3-b][1,5]benzodiazepin-10-on,
4-(Dimethylaminoacetyl)-3-ethyl-1-methyl-1,4,9,10-tetrahydropyrazolo[4,3-b][1,5]benzodiazepin-10-on,
4-(Dimethylaminoacetyl)-3-isopropyl-1-methyl-1,4,9,10-tetrahydropyrazolo[4,3-b][1,5]benzodiazepin-10-on,
4-(Dimethylaminoacetyl)-1-methyl-1,4,9,10-tetrahydropyrazolo[4,3-b][1,5]benzodiazepin-10-on,
4-(Dimethylaminoacetyl)-1-ethyl-1,4,9,10-tetrahydropyrazolo[4,3-b][1,5]benzodiazepin-10-on,
4-(Dimethylaminoacetyl)-1-isopropyl-1,4,9,10-tetrahydropyrazolo[4,3-b][1,5]benzodiazepin-10-on,
4-[3-(Dimethylamino)-propionyl]-1,3-dimethyl-1,4,9,10-tetrahydropyrazolo[ 4,3-b][1,5]benzodiazepin-10-on,
4-[2-(Dimethylamino)-propionyl)-1,3-dimethyl-1,4,9,10-tetrahydropyrazolo[4,3-b][1,5]benzodiazepin-10-on,
4-[4-(Dimethylamino)-butyryl]-1,3-dimethyl-1,4,9,10-tetrahydropyrazolo[4,3-b][1,5]benzodiazepin-10-on,
4-[5-(Dimethylamino)valeryl]-1,3-dimethyl-1,4,9,10-tetrahydropyrazolo[4,3-b][1,5]benzodiazepin-10-on,
durch Umsetzung von
4-Chloracetyl-1-ethyl-3-methyl-1,4,9,10-tetrahydropyrazolo[4,3-b][1,5]benzodiazepin-10-on,
4-Chloracetyl-1-isopropyl-3-methyl-1,4,9,10-tetrahydropyrazolo[4,3-b][1,5]benzodiazepin-10-on,
4-Chloracetyl-3-ethyl-1-methyl-1,4,9,10-tetrahydropyrazolo[4,3-b][1,5]benzodiazepin-10-on,
4-Chloracetyl-3-isopropyl-1-methyl-1,4,9,10-tetrahydropyrazolo[4,3-b][1,5]benzodiazepin-10-on,
4-Chloracetyl-1-methyl-1,4,9,10-tetrahydropyrazolo[4,3-b][1,5]benzodiazepin-10-on,
4-Chloracetyl-1-ethyl-1,4,9,10-tetrahydropyrazolo[4,3-b][1,5]benzodiazepin-10-on,
4-Chloracetyl-1-isopropyl-1,4,9,10-tetrahydropyrazolo[ 4,3-b][1,5]benzodiazepin-10-on,
4-(3-Chlorpropionyl)-1,3-dimethyl-1,4,9,10-tetrahydropyrazolo[4,3-b][1,5]benzodiazepin-10-on,
4-(2-Chlorpropionyl)-1,3-dimethyl-1,4,9,10-tetrahydropyrazolo[4,3-b][1,5]benzodiazepin-10-on,
4-(4-Chlorbutyryl)-1,3-dimethyl-1,4,9,10-tetrahydropyrazolo[4,3-b][1,5]benzodiazepin-10-on bzw.
4-(5-Chlorvaleryl)-1,3-dimethyl-1,4,9,10-tetrahydropyrazolo[4,3-b][1,5]benzodiazepin-10-on
mit Dimethylamin.

**Beispiel 11**

Analog Beispiel 4 erhält man
1-Ethyl-3-methyl-4-(pyrrolidinoacetyl)-1,4,9,10-tetrahydropyrazolo[4,3-b][1,5]benzodiazepin-10-on,
1-Isopropyl-3-methyl-4-(pyrrolidinoacetyl)-1,4,9,10-tetrahydropyrazolo[4,3-b][1,5]benzodiazepin-10-on,
3-Ethyl-1-methyl-4-(pyrrolidinoacetyl)-1,4,9,10-tetrahydropyrazolo[4,3-b][1,5]benzodiazepin-10-on,
3-Isopropyl-1-methyl-4-(pyrrolidinoacetyl)-1,4,9,10-tetrahydropyrazolo[ 4,3-b][1,5]benzodiazepin-10-on,
1-Methyl-4-(pyrrolidinoacetyl)-1,4,9,10-tetrahydropyrazolo[4,3-b][1,5]benzodiazepin-10-on (F. 197–199°C),
1-Ethyl-4-(pyrrolidinoacetyl)-1,4,9,10-tetrahydropyrazolo[4,3-b][1,5]benzodiazepin-10-on,
1-Isopropyl-4-(pryyolidinoacetyl)-1,4,9,10-tetrahydropyrazolo[4,3-b][1,5]benzodiazepin-10-on,
1,3-Dimethyl-4-[3-(pyrrolidino)-propionyl]-1,4,9,10-tetrahydropyrazolo[4,3-b][1,5]benzodiazepin-10-on
1,3-Dimethyl-4-[2-(pyrrolidino)-propionyl]-1,4,9,10-tetrahydropyrazolo[4,3-b][1,5]benzodiazepin-10-on,
1,3-Dimethyl-4-[4-(pyrrolidino)-butyryl]-1,4,9,10-tetrahydropyrazolo[4,3-b][1,5]benzodiazepin-10-on,
1,3-Dimethyl-4-[5-(pyrrolidino)-valeryl]-1,4,9,10-tetrahydropyrazolo[4,3-b][1,5]benzodiazepin-10-on,
durch Umsetzung von
4-Chloracetyl-1-ethyl-3-methyl-1,4,9,10-tetrahydropyrazolo[ 4,3-b][1,5]benzodiazepin-10-on,
4-Chloracetyl-1-isopropyl-3-methyl-1,4,9,10-tetrahydropyrazolo[4,3-b][1,5]benzodiazepin-10-on,
4-Chloracetyl-3-ethyl-1-methyl-1,4,9,10-tetrahydropyrazolo[4,3-b][1,5]benzodiazepin-10-on,
4-Chloracetyl-3-isopropyl-1-methyl-1,4,9,10-tetrahydropyrazolo[4,3-b][1,5]benzodiazepin-10-on,
4-Chloracetyl-1-methyl-1,4,9,10-tetrahydropyrazolo[4,3-b][1,5]benzodiaz pin-10-on
4-Chloracetyl-1-ethyl-1,4,9,10-tetrahydropyrazolo[4,3-b][1,5]benzodiazepin-10-on,
4Chloracetyl-1-isopropyl-1,4,9,10-tetrahydropyrazolo[4,3-b][1,5]benzodiazepin-10-on,
4-(3-Chlorpropionyl)-1,3-dimethyl-1,4,9,10-tetrahydropyrazolo[4,3-b][1,5]benzodiazepin-10-on,
4-(2-Chlorpropionyl)-1,3-dimethyl-1,4,9,10-tetrahydropyrazolo[ 4,3-b][1,5]benzodiazepin-10-on,
4-(4-Chlorbutyryl)-1,3-dimethyl-1,4,9,10-tetrahydropyrazolo[4,3-b][1,5]benzodiazepin-10-on bzw.
4-(5-Chlorvaleryl)-1,3-dimethyl-1,4,9,10-tetrahydropyrazolo[4,3-b][1,5]benzodiazepin-10-on
mit Pyrrolidin.

**Beispiel 12**

Zu 2,9 1,3-Dimethyl-1,4,9,10-tetrahydropyrazolo[4,3-b][1,5]benzodiazepin-10-on, 6 g wasserfreiem Kaliumcarbonat, 40 ml Dioxan und 20 ml Toluol tropft man in 30 Minuten bei 70–80°C eine Lösong von 2,1 g Chloracetylchlorid in10 ml Toluol und rührt noch 2 Stunden bei dieser Temperatur. Man engt zur Trockne ein, kocht den Rückstand 3 mal mit je 25 ml Chloroform aus und engt das Filtrat zur Trockne ein. Man erhält 3,6 g 4-Chloracetyl-1,3-dimethyl-1,4,9,10-tetrahydropyrazolo[4,3-b][1,5]benzodiazepin-10-on, F. 246–248°C (Zers.).

**Beispiel 13**

Zu einer siedenden Lösung von 2,3 g 1,3-Dimethyl-1,4,9,10-tetrahydropyrazolo[ 4,3-b][1,5]benzodiazepin-10-on in

30 ml absolutem Dioxan werden in 40 Minuten gleichzeitig 2 g Chloracetylchlorid und 2 g Triethylamin getropft und 3 Stunden weitergerührt. Man läßt erkalten, filtriert, engt das Filtrat zur Trockne ein und chromatographiert über eine Kieselgelsäule mittels eines Gemisches von Petrolether/Essigsäureethylester (1:1), kristallisiert aud Toluol um und erhält 2,0 g 4-Chloracetyl-1,3-dimethyl-1,4,9,10-tetrahydropyrazolo[4,3-b][1,5]benzodiazepin-10-on, F. 246–248°C (Zers.)

**Beispiel 14**

Man rührt 6,7 g 1-Ethyl-3-methyl-1,4,9,10-tetrahydropyrazolo[4,3-b][1,5]benzodiazepin-10-on, 4,0 Chloracetylchlorid, 10 g wasserfreies Kaliumcarbonat und 70 ml trockenes Dioxan unter Stickstoff 1 Stunde bei 80°C, filtriert in der Wärme und rührt den Niederslag der anorgansichen Salze 3 mal mit Chloroform aus. Die organischen Filtrate werden im Vakuum zur Trockne eingeengt; der Rückstand wird mit Wasser und Natriumhydrogencarbonatlösung bei pH 6 gerührt, mit Wasser gewaschen und im Vakuum bei 50°C getrocknet. Man erhält 8,7 g 4-Chloracetyl-1-ethyl-3-methyl-1,4,9,10-tetrahydropyrazolo[4,3-b][1,5]benzodiazepin-10-on, F. 208–209,5°C.

Analog erhält man
4-Chloracetyl-1,3-dimethyl-1,4,9,10-tetrahydropyrazolo[4,3-b][1,5]benzodiazepin-10-on (F. 246–248°C unter Zers.),
4-Chloracetyl-3-ethyl-1-methyl-1,4,9,10-tetrahydropyrazolo[4,3-b][1,5]benzodiazepin-10-on (F. 217–218,5°C),
4-Chloracetyl-3-isopropyl-1-methyl-1,4,9,10-tetrahydropyrazolo[4,3-b][1,5]benzodiazepin-10-on,
4-Chloracetyl-1-methyl-1,4,9,10-tetrahydropyrazolo[4,3-b][1,5]benzodiazepin-10-on (F. 245–246°C under Zers.),
4-Chloracetyl-1-ethyl-1,4,9,10-tetrahydropyrazolo[4,3-b][1,5]benzodiazepin-10-on,
4-Chloracetyl-1-isopropyl-1,4,9,10-tetrahydropyrazolo[4,3-b][1,5]benzodiazepin-10-on
durch Umsetzung von Chloracetylchlorid mit
1,3-Dimethyl-1,4,9,10-tetrahydropyrazolo[4,3-b][1,5]benzodiazepin-10-on,
3-Ethyl-1-methyl-1,4,9,10-tetrahydropyrazolo[4,3-b][1,5]benzodiazepin-10-on,
3-Isopropyl-1-methyl-1,4,9,10-tetrahydropyrazolo[4,3-b][1,5]benzodiazepin-10-on,
1-Methyl-1,4,9,10-tetrahydropyrazolo[4,3-b][1,5]benzodiazepin-10-on,
1-Ethyl-1,4,9,10-tetrahydropyrazolo[4,3-b][1,5]benzodiazepin-10-on bzw.L
1-Isopropyl-1,4,9,10-tetrahydropyrazolo[4,3-b][1,5]benzodiazepin-10-on.

**Beispiel 15**

Man rührt 5,0 g 1,3-Dimethyl-1,4,9,10-tetrahydropyrazolo[4,3-b][1,5]benzodiazepin-10-on, 5,2 g 3-Chlorpropionylchlorid, 11 g gemahlenes Kaliumcarbonat und 60 ml absolutes Dioxan 1,5 Stunden bei 50–60°C und destilliert das Lösungsmittel im Vakuum ab. Man versetzt den Rückstand mit Eiswasser und Salzsäure bis pH 6, läßt im Eisschrank über Nacht stehen und kristallisiert den erhaltenen Niederschlag aus Ethylacetat/-Cyclohexan um. Man erhält 5,3 g 4-(3-Chlorpropionyl)-1,3-dimethyl-1,4,9,10-tetrahydropyrazolo[4,3-b][1,5]-benzodiazepin-10-on, F. 205–206,5°C (Zers.).

Analog erhält man
4-(3-Chlorpropionyl)-1-ethyl-3-methyl-1,4,9,10-tetrahydropyrazolo[4,3-b][1,5]benzodiazepin-10-on,
4-(3-Chlorpropionyl)-1-isopropyl-3-methyl-1,4,9,10-tetrahydropyrazolo[4,3-b][1,5]benzodiazepin-10-on,
4-(3-Chlorpropionyl)-3-ethyl-1-methyl-1,4,9,10-tetrahydropyrazolo[4,3-b[1,5]benzodiazepin-10-on,
4-(3-Chlorpropionyl)-3-isopropyl-1-methyl-1,4,9,10-tetrahydropyrazolo[4,3-b][1,5]benzodiazepin-10-on,
4-(3-Chlorpropionyl)-1-methyl-1,4,9,10-tetrahydropyrazolo[4,3-b][1,5]benzodiazepin-10-on,
4-(3-Chlorpropionyl)-1-ethyl-1,4,9,10-tetrahydropyrazolo[4,3-b][1,5]benzodiazepin-10-on,
4-(3-Chlorpropionyl)-1-isopropyl-1,4,9,10-tetrahydropyrazolo[4,3-b][1,5]benzodiazepin-10-on
durch Umsetzung von 3-Chlorpropionylchlorid mit
1-Ethyl-3-methyl-1,4,9,10-tetrahydropyrazolo[4,3-b][1,5]benzodiazepin-10-on,
1-Isopropyl-3-methyl-1,4,9,10-tetrahydropyrazolo[4,3-b][1,5]benzodiazepin-10-on,
3-Ethyl-1-methyl-1,4,9,10-tetrahydropyrazolo[4,3-b][1,5]benzodiazepin-10-on,
3-Isopropyl-1-methyl-1,4,9,10-tetrahydropyrazolo[4,3-b][1,5]benzodiazepin-10-on,
1-Methyl-1,4,9,10-tetrahydropyrazolo[4,3-b][1,5]benzodiazepin-10-on,
1-Ethyl-1,4,9,10-tetrahydropyrazolo[4,3-b][1,5]benzodiazepin-10-on bzw,
1-Isopropyl-1,4,9,10-tetrahydropyrazolo[4,3-b[1,5]benzodiazepin-10-on.

**Beispiel 16**

Analog Beispiel 14 erhält man
4-(2-Chlorpropionyl)-1,3-dimethyl-1,4,9,10-tetrahydropyrazolo[4,3-b][1,5]benzodiazepin-10-on,
4-(2-Chlorpropionyl)-1-ethyl-3-methyl-1,4,9,10-tetrahydropyrazolo[4,3-b][1,5]benzodiazepin-10-on,
4-(2-Chlorpropionyl)-1-isopropyl-3-methyl-1,4,9,10-tetrahydropyrazolo[4,3-b[1,5]benzodiazepin-10-on,
4-(2-Chlorpropionyl)-3-ethyl-1-methyl-1,4,9,10-tetrahydropyrazolo[4,3-b][1,5]benzodiazepin-10-on,
4-(2-Chlorpropionyl)-3-isopropyl-1-methyl-1,4,9,10-tetrahydropyrazolo[4,3-b][1,5]benzodiazepin-10-on,
4-(2-Chlorpropionyl)-1-methyl-1,4,9,10-tetrahydropyrazolo[4,3-b][1,5]benzodiazepin-10-on,
4-(2-Chlorpropionyl)-1-ethyl-1,4,9,10-tetrahydropyrazolo[4,3-b][1,5]benzodiazepin-10-on
4-(2-Chlorpropionyl)-1-isopropyl-1,4,9,10-tetrahydropyrazolo[4,3-b][1,5]benzodiazepin-10-on
durch Umsetzung von 2-Chlorpropionylchlorid mit
1,3-Dimethyl-1,4,9,10-tetrahydropyrazolo[4,3-b][1,5]benzodiazepin-10-on,

1-Ethyl-3-methyl-1,4,9,10-tetrahydropyrazolo[4,3-b][1,5]benzodiazepin-10-on,
1-Isopropyl-3-methyl-1,4,9,10-tetrahydropyrazolo[4,3-b][1,5]benzodiazepin-10-on,
3-Ethyl-1-methyl-1,4,9,10-tetrahydropyrazolo[4,3-b][1,5]benzodiazepin-10-on,
3-Isopropyl-1-methyl-1,4,9,10-tetrahydropyrazolo[4,3-b][1,5]benzodiazepin-10-on,
1-Methyl-1,4,9,10-tetrahydropyrazolo[4,3-b][1,5]benzodiazepin-10-on,
1-Ethyl-1,4,9,10-tetrahydropyrazolo[4,3-b][1,5]benzodiazepin-10-on bzw.
1-Isopropyl-1,4,9,10-tetrahydropyrazolo[4,3-b[1,5]benzodiazepin-10-on
und
4-(4-Chlorbutyryl)-1,3-dimethyl-1,4,9,10-tetrahydropyrazolo[4,3-b][1,5]benzodiazepin-10-on bzw.
4-(5-Chlorvaleryl)-1,3-dimethyl-1,4,9,10-tetrahydropyrazolo[4,3-b][1,5]benzodiazepin-10-on
durch Umsetzung von 4-Chlorbutylrylchlorid bzw. 5-Chlorvalerylchlorid mit
1,3-Dimethyl-1,4,9,10-tetrahydropyrazolo[4,3-b[1,5]benzodiazepin-10-on.

**Beispiel 17**

Man erhitzt 7,1 g 4-[(2-Aminophenyl)amino]-N,N,1,3-tetramethyl-pyrazol-5-carboxamid, 4,7 Benzoesäure und 8 ml trockenes Xylol 75 Minuten zum Sieden und destilliert das Lösungsmittel im Vakuum ab. Man löst den Rückstand in 70 ml Chloroform, wäscht die organische Lösung mit Natronlauge und Wasser, trocknet sie mit Magnesiumsulfat und filtriert über eine Schicht Kieselgel. Man erhält als gelbe Kristalle 5,45 g 1,3-Dimethyl-1,4,9,10-tetrahydropyrazolo[4,3-b][1,5]-benzodiazepin-10-on, F. 189–190°C.

Die Cyclisierung erfolgt analog, wenn anstelle von Benzoesäure andere mittelstarke Säuren, wie o-Chlorbenzoesäure, Nicotinsäure, m-Tolylsäure, Chloressigsäure, eingesetzt werden.

**Beispiel 18**

Man Rührt eine Schmelze aus 14,3 g 4-[(2-Aminophenyl)amino[-1-ethyl-N,N,3-trimethyl-pyrazol-5-carboxamid und 9,1 Benzoesäure 2,5 Stunden bei 150°C, versetzt in der Wärme mit 50 ml Chloroform, rührt die Lösung mit Natriumhydrogencarbonatlösung und Wasser aus und engt sie im Vakuum ein. Der Rückstand wird über eine Kieselgelsäule mit Petrolether (Kp. 50/70°C)/Essigsäureethylester (3:7) chromatographiert. Man erhält als gelbe Kristalle 8,2 g 1-Ethyl-3-methyl-1,4,9,10-tetrahydropyrazolo[4,3-b][1,5]benzodiazepin-10-on, F. 161–162°C.

In analoger Wiese erhält man
1,3-Dimethyl-1,4,9,10-tetrahydropyrazolo[4,3-b][1,5]benzodiazepin-10-on
durch Erhitzen von
4-[(2-Aminophenyl)amino]-N,N-diethyl-1,3-dimethyl-pyrazol-5-carboxamid,
4-[(2-Aminophenyl)amino]-N,1,3-trimethyl-N-phenyl-pyrazol-5-carboxamid,
1- (4-[(2-Aminophenyl)amino]-1,3-dimethyl-pyrazol-5-yl)-carbonyl -piperidin
oder
4-[(2-Aminophenyl)amino]-N,1,3-trimethyl-pyrazol-5-carboxamid
in Gegenwart von Benzoesäure.

**Beispiel 19**

Man erhitzt 8,3 g 4-[(2-Aminophenyl)amino]-1,3-dimethyl-pyrazol-5-carboxamid und 8,4 g Benzoesäure 45 Minuten auf 125°C. Die erkaltete Schmelze wird mit verdünnter natriumhydrogencarbonatlösung gerührt; der gelbe Niederschlag wird abgesaugt und mit Wasser gewaschen. Man erhält 7,1 g 1,3-Dimethyl-1,4,9,10-tetrahydropyrazolo[4,3-b][1,5]benzodiazepin-10-on, F. 189–190°C (aus Essigsäureethylester).

**Beispiel 20**

Man erhitzt 2,0 g 4-[(2-Aminphenyl)amino]-1,3-dimethyl-pyrazol-5-carbonsäuremethylester und 2,0 g Benzoesäure 1 Stunde auf 120°C und anschliessend 2 Stunden auf 140°C, wonach das Dünnschichtchromatogramm vollständige Umsetzung anzeigt. Die erkaltete Schmelze wird mit warmer Natriumhydrogencarbonatlösung gerührt, der abgesaugte Niederschlag (1,55) wird aus Essigsäureethylester und Cyclohexan umkristallisiert. Man erhält 1,3-Dimethyl-1,4,9,10-tetrahydropyrazolo[4,3-b][1,5]benzodiazepin-10-on, F. 189–190°C.

Analog erhält man 1,3-Dimethyl-1,4,9,10-tetrahydropyrazolo[4,3-b][1,5]benzodiazepin-10-on durch Erhitzen von
4-[(2-Aminophenyl)amino]-1,3-dimethyl-pyrazol-5-carbonsäureethylester bzw.
4-[(2-Aminophenyl)amino]-1,3-dimethyl-pyrazol-5-carbonsäure-n-amylester
in Gegenwart von Benzoesäure.

**Beispiel 21**

Analog Beispiel 17 erhält man
1-Isopropyl-3-methyl-1,4,9,10-tetrahydropyrazolo[4,3-b][1,5]benzodiazepiin-10-on,
3-Ethyl-1-methyl-1,4,9,10-tetrahydropyrazolo[4,3-b][1,5]benzodiazepin-10-on (F. 164–165°C),
3-Isopropyl-1-methyl-1,4,9,10-tetrahydropyrazolo[4,3-b][1,5]benzodiazepin-10-on,
1-Methyl-1,4,9,10-tetrahydropyrazolo[4,3-b][1,5]benzodiazepin-10-on (F. 201–203°C),
1-Ethyl-1,4,9,10-tetrahydropyrazolo[4,3-b][1,5]benzodiazepin-10-on,
1-Isopropyl-1,4,9,10-tetrahydropyrazolo[4,3-b][1,5]benzodiazepin-10-on

wenn man in Xylol in Gegewart von Benzoesäure
4-[(2-Aminophenyl)amino]-1-isopropyl-N,N,3-trimethyl-pyrazol-5-carboxamid,
4-[(2-Aminophenyl)amino]-3-ethyl-N,N,1-trimethyl-pyrazol-5-carboxamid,
4-[(2-Aminophenyl)amino]-3-isopropyl-N,N,1-trimethyl-pyrazol-5-carboxamid,
4-[(2-Aminophenyl)amino]-N,N,1-trimethyl-pyrazol-5-carboxamid,
4-[(2-Aminophenyl)amino]-1-ethyl-N,N-dimethyl-pyrazol-5-carboxamid bzw.
4-[(2-Aminophenyl)amino]-1-isopropyl-N,N-dimethyl-pyrazol-5-carboxamid
erhitzt.

**Beispiel 22**

Man hydriert 2,4 g 1,3-Dimethyl-4-[(2-nitropheyl)amino]-pyrazol-5-carbonsäurephenylester in 150 ml Toluol und 5 ml Eisessig mit 0,75 g 10 % Palladium-Kohle bei Raumtemperatur 2,1 Stunden in einer Umlaufhydrierungsapparatur. Man engt die filtrierte Lösung im Vakuum ein und chromatographiert den Rückstand über eine Kieselgelsäule mit Petrolether (50°/70°C)/Essigsäureethylester (1:1) und 1,1 g 1,3-Dimethyl-1,4,9,10-tetrahydropyrazolo[4,3-b][1,5]benzodiazepin-10-on, F. 189–190°C.

Hydriert man den Phenylester ohne Zusatz von Eisessig, bildet sich unter langsamer Wasserstoffaufnahme 4-[(2-Aminophenyl)amino]-1,3-dimethyl-pyrazol-5-carbonsäurephenylester, der nach Zusatz von Eisessig zum Pyrazolobenzodiazepinon ringschließt.

**Beispiel 23**

Man versetzt 4-[(2-Aminophenyl)amino[-1,3-dimethyl-pyrazol-5-carbonsäure (Rückstand aus Versuch 4) mit 80 ml trockenem Dioxan und 8,3 g Triethylamin, tropft bei 0°C 8,8 g Chlorameisensäureethylester in 15 Minuten zu und rührt noch 1,5 Stunden under Erwärmung auf Raumtemperatur. Man versetzt die Lösung mit Natriumhydrogencarbonatlösung, engt bis zur Trockne ein und kocht den Rückstand mit Chloroform aus. Man engt ein und chromatographiert den Rückstand über eine Kieselgelsäule mit Petrolether (50/70°C)/Essigester (1:1) und erhält 7,5 g 1,3-Dimethyl-1,4,9,10-tetrahydropyrazolo[4,3-b][1,5]benzodiazepin-10-on, F. 189–190°C (aus Toluol).
Die folgenden "Versuche" dienen zur näheren Erläuterung der Herstellung der Ausgansverbindungen (Zwischenprodukte)

A. *4-[2-(Aminophenyl)amino]-4-pyrazolcarbonsäurederivate*

Versuch 1: 4-[2-(Aminophenyl)amino]-N,N,1,3-tetramethylpyrazol-5-carboxamid
85,4 g N,N,1,3-Tetramethyl-4-[(2-nitrophenyl)amino]-pyrazol-5-carboxamid, 8,5 g 10 % Palladium-Kohle und 1100 ml Methanol werden 3,5 Stunden bei 25°C in einer Umlaufhydrierungsapparatur drucklos hydriert. Man filtriert, engt die Lösung zur Trockne ein und rührt den Rückstand mit einer Mischung aus 80 ml Petrolether (Kp. 50–70°C) und 40 ml Essigsäureethylester. Man erhält 75 g 4-[2-(Aminophenyl)amino]-N,N,1,3-tetramethyl-pyrazol-5-carboxamid, zuerst F. 137–139°C, beim Stehen Umwandlung in ein Produkt mit F. 171–172°C.

Analog erhält man
4-[(2-Aminophenyl)amino]-N,1,3-trimethyl-pyrazol-5-carboxamid (F. 151,5–152,5°C),
4-[(2-Aminophenyl)amino]-N,N-diethyl-1,3-dimethyl-pyrazol-5-carboxamid,
1-[ 4-[(2-Aminophenyl)amino]-1,3-dimethyl-pyrazol-5-yl -carbonyl]piperidin,
4-[(2-Aminophenyl)amino]-1-ethyl-N,N,3-trimethyl-pyrazol-5-carboxamid (F. 144–145°C),
4-[(2-Aminophenyl)amino]-1-isopropyl-N,N,3-tremethyl-pyrazol-5-carboxamid,
4-[(2-Aminophenyl)amino]-N,N,1-trimethyl-pyrazol-5-carboxamid (F. 142–144°C),
4-[(2-Aminophenyl)amino]-1-ethyl-N,N-dimethyl-pyrazol-5-carbonxamid,
4-[(2-Aminophenyl)amino]-1-isopropyl-N,N-dimethyl-pyrazol-5-carboxamid,
4-[(2-Aminophenyl)amino]-3-ethyl-N,N,1-trimethyl-pyrazol-5-carboxamid,
4-[(2-Aminophenyl)amino]-3-isopropyl-N,N,1-trimethyl-pyrazol-5-carboxamid,
4-[(2-Aminophenyl)amino]-N,1,3-trimethyl-N-phenyl-pyrazol-5-carboxamid
aus
N,1,3-Trimethyl-4-[(2-nitrophenyl)amino]-pyrazol-5-carboxamid,
N,N-Diethyl-1,3-dimethyl-4-[(2-nitrophenyl)amino]-pyrazol-5-carboxamid,
1-[ 1,3-Dimethyl-4-[(2-nitrophenyl)amino]-pyrazol-5-yl- -carbonyl[-piperidin,
1-Ethyl-N,N,3-trimethyl-4-[(2-nitrophenyl)amino]-pyrazol-5-carboxamid,
1-Isopropyl-N,N,3-trimethyl-4-[(2-nitrophenyl)amino]-pyrazol-5-carboxamid,
N,N,1-Trimethyl-4-[(2-nitrophenyl)amino]-pyrazol-5-carboxamid,
1-Ethyl-N,N-dimethyl-4-[(2-nitrophenyl)amino]-pyrazol-5-carboxamid,
1-Isopropyl-N,N-dimethyl-4-[(2-nitrophenyl)amino]-pyrazol-5-carboxamid,
3-Ethyl-N,N,1-trimethyl-4-[(2-nitrophenyl)amino]-pyrazol-5-carboxamid,
3-Isopropyl-N,N,1-trimethyl-4-[(2-nitrophenyl)amino]-pyrazol-5-carboxamid bzw.
N,1,3-Trimethyl-4-[(2-nitrophenyl)amino]-N-phenyl-pyrazol-5-carboxamid
durch Reduktion der Nitrogruppe.

Versuch 2: 4-[(2-Aminophenyl)amino]-1,3-dimethyl-pyrazol-5-carboxamid

Man hydriert 8,6 g 1,3-Dimethyl-4-[(2-nitrophenyl)amino]-pyrazol-5-carboxamid an 0,8 g 10 % Palladium-Kohle und 400 ml Ethanol 1 Stunde bei 50°C analog Versuch 1. Man filtriert, engt das Filtrat zur Trockne ein, kristallisiert den Eindampfungsrückstand aus 160 ml Essigsäureethylester und 120 ml Cyclohexan um und erhält 7,1 g 4-[(2-Aminophenyl)amino]-1,3-dimthyl-pyrazol-5-carboxamid. F. 182–184°C (Zers.).

Versuch 3: 4-[(2-Aminphenyl)amino]-1,3-dimethyl-pyrazol-5-carbonsäure-n-amylester
15 g 1,3-Dimethyl-4-[(2-nitrophenyl)amino]-pyrazol-5-carbonsäure-n-amylester werden in 250 ml Ethanol mit 4 g 10 % Palladium-Kohle 5 Stunden in einer drucklosen Umlaufhydrierungsapparatur bei Raumtemperatur hydriert, wonach das Dünnschichtchromatogramm quantitative Umsetzung anzeigt. Man filtriert den Katalysator ab, engt die Lösung im Vakuum ein und erhält als öligen Rückstand 13,5 g 4-[(2-Aminophenyl)amino]-1,3-dimethyl-pyrazol-carbonsäure-n-amylester.

Analog erhält man
4-[(2-Aminophenyl)amino]-1,3-dimethyl-pyrazol-5-carbonsäuremethylester (F. 100–101,5°C).
4-[(2-Aminophenyl)amino]-1,3-dimethyl-pyrazol-5-carbonsäureethylester,
4-[(2-Aminophenyl)amino]-1,3-dimethyl-pyrazol-5-carbonsäurephenylester
aus
1,3-Dimethyl-4-[(2-nitrophenyl)amino]-pyrazol-5-carbonsäuremethylester,
1,3-Dimethyl-4-[(2-nitrophenyl)amino]-pyrazol-5-carbonsäureethylester bzw.
1,3-Dimethyl-4-[(2-nitrophenyl)amino]-pyrazol-5-carbonsäurephenylester
durch Reduktion der Nitrogruppe (Hydrierung) in Methanol.

Versuch 4: 4-[(2-Aminophenyl)amino]-1,3-dimethyl-pyrazol-5-carbonsäure. Man versetzt 13 g 1,3-Dimethyl-4-[(2-Aminophenyl)amino]-1,3-dimethyl-pyrazol-5-carbonsäure-n-amylester bei Raumtemperatur mit einer Lösung von 2,9 Natriumhydroxid in 100 ml entgastem Ethanol und rührt 3 Stunden bei Raumtemperatur. Man stellt die Lösung mit verdünnter Salzsäure auf pH 9, destilliert den Alkohol ab, schüttelt die wässerige Lösung mit Dichlormethan aus, stellt die wässerige Lösung auf pH 7 und engt bei 60°C im Vakuum ein. Als Rückstand erhält man vermischt mit anorganischen Salzen zum Teil ölige 4-(2-Aminophenylamino)-1,3-dimethyl-pyrazol-5-carbonsäure.

B: *4-[(2-Nitrophenyl)amino]-pyrazol-5-carbonsäurederivate*
Versuch 5: N,N,1,3-Tetramethyl-4-[(2-nitrophenyl)amino]-pyrazol-5-carboxamid
Zu einer Lösung von 26,5 g 4-Amino-N,N,1,3-tetramethylpyrazol-5-carboxamid in 100 ml Dimethylformamid fügt man unter Stickstoff 10,5 Natriumhydrid (75 % in Paraffinöl), tropft zu der Suspension innerhalb 2 Stunden bei 40–45°C eine Lösung von 28 g 1-Fluor-2-nitrobenzol in 25 ml Dimethylformamid, rührt noch 4 Stunden bei 40°C, neutralisiert die rote Lösung durch Zugabe von Eisessig und destilliert das Lösungsmittel im Vakuum ab. Man unterwirft den Rückstand einer Wasserdampfdestillation, löst den Rückstand in Dichlormethan/Methanol (9:1), chromatographiert über Kieselgel und erhält 35,0 g gelbe Kristalle von N,N,1,3-Tetramethyl-4-[(2-nitrophenyl)amino]-pyrazol-5-carboxamid. F. 144–146°C (aus Essigsäureethylester).

Analog erhält man
N,N-Diethyl-1,3-dimethyl-4-[(2-nitrophenyl)amino]-pyrazol-5-carboxamid,
1-[ 1,3-Dimethyl-4-[(2-nitrophenyl)amino]-pyrazol-5-yl- -carbonyl]-piperidin,
1-Ethyl-N,N,3-trimethyl-4-[(2-nitrophenyl)amino]-pyrazol-5-carboxamid (F. 97–98°C),
1-Isopropyl-N,N,3-trimethyl-4-[(2-nitrophenyl)amino]-pyrazol-5-carboxamid,
N,N,3-Trimethyl-4-[(2-nitrophenyl)amino]-pyrazol-5-carboxamid (F. 138,5–140°C).
1-Ethyl-N,N-dimethyl-4-[(2-nitrophenyl)amino]-pyrazol-5-carboxamid,
1-Isopropyl-N,N-dimethyl-4-[(2-nitrophenyl)amino]-pyrazol-5-carbozamid,
3-Ethyl-N,N,1-trimethyl-4-[(2-nitrophenyl)amino]-pyrazol-5-carboxamid (F. 114–115°C) bzw.
3-Isopropyl-N,N,1-trimethyl-4-[(2-nitrophenyl)amino]-pyrazol-5-carboxamid,
aus
4-Amino-N,N-diethyl-1,3-dimethyl-pyrazol-5-carboxamid,
1-[(4-Amino-1,3-dimethyl-pyrazol-5-yl)-carbonyl]-piperidin,
4-Amino-1-ethyl-N,N,3-trimethyl-pyrazol-5-carboxamid,
4-Amino-1-isopropyl-N,N,3-trimethyl-pyrazol-5-carboxamid,
4-Amino-N,N,1-trimethyl-pyrazol-5-carboxamid,
4-Amino-1-ethyl-N,N-dimethyl-pyrazol-5-carboxamid,
4-Amino-1-isopropyl-N,N-dimethyl-pyrazol-5-carboxamid,
4-Amino-3-ethyl-N,N,1-trimethyl-pyrazol-5-carboxamid bzw.
4-Amino-3-isopropyl-N,N,1-trimethyl-pyrazol-5-carboxamid
durch Umsetzung mit 1-Fluor-2-nitrobenzol.

Versuch 6: N,N,1,3-Tetramethyl-4-[(2-nitropheyl)amino]-pyrazol-5-carboxamid
Zu einer Lösung von 2,0 g 4-Amino-N,N,1,3-tetramethyl-pyrazol-5-carboxamid in 20 ml Dimethylformamid fügt man unter Stickstoff 1,1 g Natriumhydrid (75 % in Paraffinöl) und dann 3,5 g 1-Chlor-2-nitrobenzol, rührt 4

Stunden bei 40°C und 24 Stunden bei Raumtemperatur und arbeitet analog Versuch 5 auf. Man erhält 2,0 g N,N,1,3-Tetramethyl-4-[(2-nitrophenyl)amino]-pyrazol-5-carbboxamid, F. 144–146°C.

Versuch 7: N,N,1,3-Tetramethyl-4-[(2-nitrophenyl)amino]-pyrazol-5-carboxamid
2,0 g 4-Amino-N,N,1,3-tetramethyl-pyrazol-5-carboxamid, 2,0 g 1-Fluor-2-nitrobenzol und 1,8 g N-Ethylmorpholin werden 12 Stunden auf 150°C erhitzt. Man versetzt das Reaktionsprodukt mit verdünnter Salzsäure, saugt den kristallinen Rückstand ab, trocknet ihn und rührt mit wenig Petrolether (Kp. 40°C) aus. Man erhält 2,5 g N,N,1,3-Tetramethyl-4-[(2-nitrophenyl)amino]-pyrazol-5-carboxamid, F. 144–146°C (aus Essigester).

Analog erhält man aus 4-Amino-N,1,3-trimethyl-pyrazol-5-carboxamidhydrochlorid, N-Ethylmorpholin und 1-Fluor-2-nitrobenzol als gelbe Kristalle, N,1,3-Tremethyl-4-[(2-nitrophenyl)amino]-pyrazol-5-carboxamid, F. 223–225°C.

Versuch 8: 1,3-Dimethyl-4-[(2-nitrophenyl)amino]-pyrazol-5-carboxamid
Man behandelt eine Mischung aus 10,35 g 4-Amino-1,3-dimethyl-pyrazol-5-carboxamid-hydrochlorid, 22,4 g 1-Brom-2-nitrobenzol, 15 g gemahlenem Kaliumcarbonat, 2,7 g Kupfer(I)-chlorid und 55 ml Dimethylformamid zur Homogenisierung mit Ultraschall und erwärmt 1 Stunde auf 90°C. Man destilliert das Lösungsmittel im Vakuum ab und chromatographiert den Rückstand mit einer Mischung aus Methylenchlorid/Methanol (93:7) über Kieselgel und kristallisiert aus Toluol um. Man erhält 10,0 g gelbe Kristalle von 1,3-Dimethyl-4-[(2-nitrophenyl)amino]-pyrazol-5-carboxamid, F. 232–233°C.

Analog erhält man
N,1,3-Trimethyl-4-[(2-nitrophenyl)amino]-pyrazol-5-carboxamid (F. 223–225°C)
aus
4-Amino-N,1,3-trimethyl-pyrazol-5-carboxamid-hydrochlorid
durch Umsetzung mit 1-Brom-2-nitrobenzol.

Versuch 9: 1,3-Dimethyl-4-[(2-nitrophenyl)amino]-pyrazol-5-carbonsäure-n-amylester
16 g 4-Amino-1,3-dimethyl-pyrazol-5-cabonsäureethylester, 36 g 1-Brom-2-nitrobenzol, 24 g wasserfreies Kaliumcarbonat und 150 ml n-Amylalkohol werden 12 Stunden unter langsamem Abdestillieren von Reaktionswasser über eine kurze Kolonne erhitzt, wobei 8,2 g Kupferpulver und 8,2 g Kupfer(I)-chlorid, in jeweils 4 Portionen verteilt, zugesetzt werden. Man verdünnt die abgekühlte Reaktionsmischung mit 250 ml Essigsäureethylester, filtriert die anorganischen Salze ab, engt ein und chromatographiert den erhaltenen Rückstand über eine Kieselgelsäule mit Petrolether (50/70°C)/Essigsäureethylester (4:1). Durch Umesterung mit dem Lösungsmittel während der Reaktion isoliert man als Hauptfraktion nicht den entrsprechenden Ethylester, sondern 20 g öligen 1,3-Dimethyl-4-[(2-nitrophenyl)amino]-pyrazol-5-carbonsäure-n-amylester. F. 60–62°C (aus Methanol/Wasser).

Versuch 10: 1,3-Dimethyl-4-[(2-nitrophenyl)amino]pyrazol-5-carbonsäure 4,05 g 1,3-Dimethyl-4-[(2-nitrophenyl)amino]pyrazol-5-carbonsäure-n-amylester (Versuch 9) und eine Lösung von 0,7 g Natriumhydroxid in 80 ml Ethanol werden 4 Stunden bei 40°C in einer Stickstoffatmosphäre gerührt. Man stumpft die Lösung mit Eisessig auf pH 9–10 ab, engt im Vakuum ein, versetzt den Rückstand mit Wasser, schüttelt mit Dichlormethan aus und säuert die wässerige Lösung mit verdünnter Salzsäure an. Man erhält 2,7 g 1-3-Dimethyl-4-[(2-nitrophenyl)amino]-pyrazol-5-carbonsäure, F. 253–254°C (Zers.).

Versuch 11: 1,3-Dimethyl-4-[(2-nitrophenyl)amino]-pyrazol-5-carbonsäurechlorid
2,6 g 1,3-Dimethyl-4-[(2-nitrophenyl)amino]-pyrazol-5-carbonsäure und 10 ml Thionylchlorid werden 3 Stunden bei 80°C gerührt, die Lösung eingeengt und je 3 mal mit 10 ml Tolouol versetzt und wieder eingeengt. Man erhält als orangefarbenes Öl 2,9 g 1,3-Dimethyl-4-[(2-nitrophenyl)amino]-pyrazol-5-carbonsäurechlorid.

Versuch 12: 1,3-Dimethyl-4-[(2-nitrophenyl)amino]-pyrazol-5-carbonsäurephenylester
Das Carbonsäurechlorid aus Versuch 11 wird mit 5 ml absolutem Dioxan ung 1,6 g Natriumphenolat 30 Minuten auf 80°C erwärmt, die Lösung filtriert und eingeengt. Man versetzt mit Toluol, schüttelt mehrmals mit Wasser aus und destilliert das Lösungsmittel im Vakuum ab. Man erhält als Öl 3,0 g 1,3-Dimethyl-4-[(2-nitrophenyl)amino]-pyrazol-5-carbonsäurephenylester.

Versuch 13: 1,3-Dimethyl-4-[(2-nitrophenyl)amino]-pyrazol-5-carbonsäuremethylester
Man erhitzt 13,0 g 4-Amino-1,3-dimethyl-pyrazol-5-carbonsäuremethylester, 32,0 g 1-Brom-2-nitrobenzol, 10,7 g wasserfreies Kaliumcarbonat, 8,0 g Kupfer(I)-chlorid (Zugabe in 3 Portionen) und 75 ml Dimethylformamid 9 Stunden auf 150°C, destilliert das Lösungsmittel im Vakuum av und chromatographiert den Rückstand mit Cyclohexan/Essigsäurethylester (1:1) über Kieselgel. Man erhält 3,1 g 1,3-Dimethyl-4-[(2-nitrophenyl)amino]pyrazol-5-carbonsäuremethylester, F. 127–129°C, und als Nebenprodukt 1,3-Dimethyl-4-[(2-nitrophenyl)amino]pyrazol, F. 171–173°C.

Versuch 14: N,1,3-Trimethyl-4-[(2-nitrophenyl)amino]-N-phenyl-pyrazol-5-carboxamid

Zu einer Lösung von 2,95 g 1,3-Dimethyl-4-[(2-nitrophenyl)amino]-pyrazol-5-carbonsäurechlorid in 10 ml Dioxan tropft man 2,7 g N-Methylanilin und erwärmt 1 Stunde auf 90°C. Man engt die Lösung im Vakuum ein, versetzt mit Wasser und etwas verdünnter Salzsäure und erhält 3,1 g N,1,3-Trimethyl-4-[(2-nitrophenyl)amino]-N-phenyl-pyrazol-5-carboxamid.

Analog erhält man
N,N-Diethyl-1,3-dimethyl-4-[(2-nitrophenyl)amino]-pyrazol-5-carboxamid,
1-[ 1,3-Dimethyl-4-[(2-nitrophenyl)amino]-pyrazol-5-yl- -carbonyl]-piperidin,
N,1,3-Trimethyl-4-[(2-nitrophenyl)amino]-pyrazol-5-carboxamid
durch Umsetzung des Säurechlorids mit Diethylamin, Piperidin oder Methylamin.

C: *4-Aminopyrazol-5-carbonsäurederivate*
Versuch 15: 4-Amino-N,N,1,3-tetramethyl-pyrazol-5-carboxamid 19,0 g N,N,1,3-Tetramethyl-4-nitro-pyrazol-5-carboxamid in 400 ml Methanol werden in Gegenwart von 2,0 g 10 % Palladium-Kohle im Autoklaven mit 80–110 bar (8000–11000 kPa) Wasserstoffdruck 8 Stunden bei Raumtemperatur hydriert. Man filitriert, destilliert das Lösungsmittel im Vakuum ab und erhält 16,1 g kristallines 4-Amino-N,N,1,3-tetramethyl-pyrazol-5-carboxamid, F. 116–118°C (aus Ethylacetat).

Analog erhält man
4-Amino-N,1,3-trimethyl-pyrazol-5-carboxamid (Hydrochlorid F. 242–244°C),
4-Amino-1-ethyl-N,N,3-trimethyl-pyrazol-5-carboxamid (F. 81–82°C),
4-Amino-N,N-diethyl-1,3-dimethyl-pyrazol-5-carboxamid,
1-[(4-Amino-1,3-dimethyl-pyrazol-5-yl)-carbonyl]-piperidin,
4-Amino-N,N,1-trimethyl-pyrazol-5-carboxamid (F. 127–133°C),
4-Amino-1-Ethyl-N,N-dimethyl-pyrazol-5-carboxamid,
4-Amino-1-isopropyl-N,N-dimethyl-pyrazol-5-carboxamid,
4-Amino-3-ethyl-N,N,1-trimethyl-pyrazol-5-carboxamid bzw.
4-Amino-3-isopropyl-N,N,1-trimethyl-pyrazol-5-carboxamid
durch Hydrierung von
N,1,3-Trimethyl-4-nitro-pyrazol-5-carboxamid,
1-Ethyl-N,N,3-trimethyl-4-nitro-pyrazol-5-carboxamid,
N,N,-Diethyl-1,3-dimethyl-4-nitro-pyrazol-5-carboxamid,
1-[(1,3-Dimethyl-4-nitro-pyrazol-5-yl)-carbonyl]-piperidin,
N,N,1-Trimethyl-4-nitro-pyrazol-5-carboxamid,
1-Ethyl-N,N-dimethyl-4-nitro-pyrazol-5-carboxamid,
1-Isopropyl-N,N-dimethyl-4-nitro-pyrazol-5-carboxamid,
3-Ethyl-N,N,1-trimethyl-4-nitro-pyrazol-5-carboxamid bzw.
3-Isopropyl-N,N,1-trimethyl-4-nitro-pyrazol-5-carboxamid.

Versuch 16: 4-Amino-1,3-dimethyl-pyrazol-5-carboxamid
a) Man hydrierth 10,5 g 1,3-Dimethyl-4-nitro-pyrazol-5-carboxamid in 150 ml Methanol mit 1,0 g 10 % Palladium-Kohle im Autoklaven mit 50 bar Wasserstoffdruck 2 Stunden bei 70°C. Man filtriert und engt im Vakuum zur Trockne ein. Die freie Base (F. 155–157°C nach Umkristallisation aus Essigsäureethylester/Cyclohexan) wird in Ethanol gelöst und mit etherischer Chlorwasserstofflösung werden 7,4 g Hydrochlorid (F. 232–234°C under Zers.) gefällt.

b) Zu einer Suspension von 2,0 g 1,3-Dimethyl-4-nitropyrazol-5-carboxamid und ca. 0,2 g feuchtem Raney-Nickel in 15 ml Ethanol tropft man bei 60°–75°C 1,1 ml Hydrazinhydrat, hält die Lösung 1,5 Stunden am Sieden, filtriert, engt im Vakuum zur Trockne ein und fällt mit etherischer Chlorwasserstofflösung 1,3 g Hydrochlorid aus, F. 232–234°C (Zers.).

Versuch 17: 4-Amino-1,3-dimethyl-pyrazol-5-carbonsäureethylester 20 g 1,3-Dimethyl-4-nitro-pyrazol-5-carbonsäureethylester werden in 250 ml Ethanol mit 4 g 10 % Palladium-Kohle 5,5 Stunden in einer drucklosen Umlaufhydrierungsapparatur bei Raumtemperatur hydriert. Man engt die Lösung ein und bringt den Rückstand durch Zusatz von Petrolether (50/70°C) zur Kristallation. Man erhält 15,6 g 4-Amino-1,3-dimethyl-pyrazol-5-carbonsäureethylester, F. 55,5–56,5°C.

Analog erhält man
4-Amino-1,3-dimethyl-pyrazol-5-carbonsäuremethylester (F. 76–78°C, Hydrochlorid F. 183–185°C unter Zers.) bzw.
4-Amino-1,3-dimethyl-pyrazol-5-carbonsäure-n-amylester (Hydrochlorid F. 131–133°C) aus
1,3-Dimethyl-4-nitro-pyrazol-5-carbonsäuremethylester bzw.
1,3-Dimethyl-4-nitro-pyrazol-5-carbonsäure-n amylester.

D: *4-Nitro-pyrazol-5-carbonsäurederivate*
Versuch 18: N,N,1,3-Tetramethyl-4-nitro-pyrazol-5-carboxamid

Man tropft eine Lösung von 40 g 1,3-Dimethyl-4-nitro-pyrazol-5-carbonsäurechlorid in 50 ml Dichlormethan bei 10–20°C unter Kühlung zu 43 g einer 40 % wässerigen Dimethylaminlösung, rührt noch 20 Minuten, macht mit kaliumcarbonat alkalisch, trennt die organische Schicht ab und schüttelt die wässerige Phase mit Dichlormethan aus. Man trocknet die organische Lösung, engt sie ein, verreibt den Rückstand mit Petrolether (Kp. 40–70°C) und erhält 38,3 g N,N,1,3-Tetramethyl-4-nitro-pyrazol-5-carboxamid, F. 57–59,5°C, das sich beim Umkristallisieren aus Cyclohexan in ein Produkt mit F. 114–115,5°C umwandelt.

Analog werden
N,1,3-Trimethyl-4-nitro-pyrazol-5-carboxamid (F. 158–160°C),
1-Ethyl-N,N,3-trimethyl-4-nitro-pyrazol-5-carboxamid (F. 59–60,5°C),
1-[(1,3-Dimethyl-4-nitro-pyrazol-5-yl)-carbonyl]-piperidin,
N,N,1-Trimethyl-4-nitro-pyrazol-5-carboxamid (F. 104–119°C),
1-Ethyl-N,N-dimethyl-4-nitro-pyrazol-5-carboxamid,
1-Isopropyl-N,N-dimethyl-4-nitro-pyrazol-5-carboxamid,
3-Ethyl-N,N,1-trimethyl-4-nitro-pyrazol-5-carboxamid (F. 91–92°C),
3-Isopropyl-N,N,1-trimethyl-4-nitro-pyrazol-5-carboxamid bzw.
4-[(1-Ethyl-3-methyl-4-nitro-pyrazol-5-yl)-carbonyl]-morpholin,

aus den entsprechenden 4-Nitro-pyrazol-5-carbonsäurechloriden und Aminen erhalten.

Versuch 19: 1,3-Dimethyl-4-nitro-pyrazol-5-carboxamid
27,2 g 1,3-Dimethyl-4-nitro-pyrazol-5-carbonitril und 65 ml konzentrierte Schwefelsäure werden 6 Stunden bei 80°C gerührt. Man gießt auf 700 g Eis und Wasser, filtriert den Niederslag ab, suspendiert ihn in Eiswasser und neutralisiert mit Natriumhydrogencarbonat und erhält 27,3 g 1,3-Dimethyl-4-nitro-pyrazol-5-carboxamid, F. 164–165°C.

Versuch 20: 1,3-Dimethyl-4-nitro-pyrazol-5-carbonsäureethylester
Zu 37 g 1,3-Dimethyl-4-nitro-pyrazol-5-carbonsäure werden in Portionen 44,6 g Phosphorpentachlorid zugegeben, wobei sich der Ansatz allmählich verflüssigt. Unter Rühren wird auf 100°C Badtemperatur geheizt und nach 1 Stunde Rühren im Rotationsverdampfer eingeengt, 3 mal TOluol zugegeben und wieder eingeengt. Zu dem kristallisierten Rückstand von 1,3-Dimethyl-4-nitropyrazol-5-carbonsäurechlorid fügt man unter Kühlen 37 ml absolutes Ethanol hinzu, erwärmt 1 Stunde auf 50°C, engt die Lösung im Vakuum ein, versetzt mit 300 ml Essigsäureethylester, schüttelt mit Natriumhydrogencarbonatlösung aus und egnt ein. Man erhält 41,2 g 1,3-Dimethyl-4-nitro-pyrazol-5-carbonsäureethylester, F. 47–48°C.

Analog erhält man
1,3-Dimethyl-4-nitro-pyrazol-5-carbonsäuremethylester (F. 71–72°C) bzw.
1,3-Dimethyl-4-nitro-pyrazol-5-carbonsäure-n-amylester (Öl)
aus 1,3-Dimethyl-4-nitro-pyrazol-5-carbonsäurechlorid und dem entsprechenden Alkohol.

Versuch 21: 1-Methyl-4-nitro-pyrazol-5-carbonsäure
18,8 g 1-Methyl-pyrazol-5-carbonsäure (F. 227–228°C) werden in einer Mischung aus 23,5 g 100 %iger Salpetersäure un 17 ml 25 %igem Oleum 8 Stunden bei 55–60°C und 4,5 Stunden bei 70–75°C gerührt. Man gießt auf Eis, extrahiert mit Dichlormethan/Ethanol (9:1) und engt ein. Man erhält 27 g 1-Methyl-4-nitro-pyrazol-5-carbonsäure, F. 162–164°C unter Zers. (aus Ethylacetat).

Versuch 22: 3-Isopropyl-1-methyl-4-nitro-pyrazol-5-carbonsäure
a) Zu einer Lösung von 23,0 g Natrium in 290 ml absolutem Ethanol tropft man bei 6–20°C unter leichtem Kühlen eine Mischung aus 148 g Oxalsäurediethylester und 86 g Aceton und läßt die kristallisierende Mischung über Nacht stehen. Man verdünnt mit 500 ml Ethanol, filtriert und erhält 185 g Natrium-5-methyl-2,4-dioxo-hexanoat.
b) Zu 180 g Natriumsalz aus (a) in 800 ml Ethanol tropft man 44,6 g Eisessig in 200 ml Ethanol und bei −3° bis 0°C 52,9 g Hydrazinhydrat, destilliert das Lösungsmittel im Vakuum ab, versetzt mit Wasser und extrahiert mit Chloroform. Man destiliert das Lösungsmittel im Vakuum ab, versetzt mit Toluol, engt ein, trocknet den kristallinen Rückstand über Paraffin und trubiniert ihn in 600 ml Eiswasser. Man erhält 141 g 5-Isopropyl-pyrazol-3-carbonsäureethylester, F. 70,5–71°C (aus Wasser).
c) 5,9 des Esters aus (b) und 4,1 g Dimethylsulfat werden 2,5 Stunden auf 90°C erwärmt, dann tropft man 18 ml 6n Natronlauge zu, rührt 2,5 Stunden bei 80°C, säuert in der Wärme mit konzentrierter Salzsäure an, filtriert den Niederschlag ab und rührt ihn in Eiswasser und erhält 4,1 g 3-Isopropyl-1-methyl-pyrazol-5-carbonsäure, F. 159–160°C (aus Dioxan/Cyclohexan).
d) 4 g Carbonsäure aus (c), 3,6 ml 100 %ige Salpetersäure und 4,2 ml 25 %iges Oleum werden 20 Stunden bei 60°C und 48 Stunden bei Raumtemperatur gerührt. Man gießt auf Eis und erhält 3-Isopropyl-1-methyl-4-nitro-pyrazol-5-carbonsäure.

Die folgenden Beispiele beschreiben die Formulierung der erfindungsgemäßen Verbindung zu Arzneimitteln.

**Beispiel 1**

10 000 Tabletten mit einem Wirkstoffgehalt von 20 mg werden aus folgenden Bestandteilen hergestellt: 200 g 1,3-Dimethyl-4-[(4-methyl-piperazin-1-yl)acetyl]-1,4,9,10-tetrahydropyrazolo[4,3-b][1,5]benzodiazepin-10-on, 900 g Maisstärke, 500 g Milchzucker, 30 g amorphe Kieselsäure und 40 g Natriumlaurylsulfat werden gemischt und gesiebt. Diese Mischung wird mit einer Lösung von 50 g Polyvinylpyrrolidon (mittl. Mol.-Gewicht 25 000) in 320 ml Alkohol befeuchtet und druch ein Sieb der Maschenweite von 1,25 mm granuliert. Das Granulat wird bei 40° getrocknet und mit 160 g Pektin, 100 g Talk und 20 g Magnesiumstearat gemischt. Diese Mischung wird zu Tabletten à 200 mg mit 8 mm Druchmesser verpreßt.

**Beispiel 2**

100 000 Kapseln mit einem Wirkstoffgehalt von 30 mg werden aus folgenden Bestandteilen hergestellt: 3 000 g 1,3-Dimethyl-4-[(4-methyl-piperazin-1-yl)acetyl]-1,4,9,10-tetrahydropyrazolo[4,3-b][1,5]benzodiazepin-10-on werden mit 5 000 g Neutralöl (Miglyol[R]812) gemischt und in Weichgelatinekapseln abgefüllt.

**Beispiel 3**

100 000 Kapseln mit einem Wirkstoffgehalt von 30 mg werden aus folgenden Bestandteilen hergestellt: 1 500 g 1,3-Dimethyl-4-[(4-methyl-piperazin-1-yl)acetyl]-1,4,9,10-tetrahydropyrazolo[4,3-b][1,5]benzodiazepin-10-on und 1 500 g Magnesiumtrisilikat werden mit 5 000 g Neutralöl (Miglyol[R]812) gemischt und in Weichgelatinekapseln abgefüllt.

**Pharmakologie**

Die ausgezeichnete Magenschutzwirkung der pharmakologisch wirksamen Pyrazolobenzodiazepinone läßt sich am Modell der sogenannten Shay-Ratte nachweisen. Hierbei erweisen sich die erfindungsgemäßen Verbindungen dem bekannten Handelsprodukt Carbenoxolon(1) in der Magenschutzwirkung und der Toxizität eindeutig überlegen, wie sich beispielsweise am Vergleich von (1) und 1,3-Dimethyl-4-[(4-methyl-piperazin-1-yl)acetyl]-1,4,9,10-tetrahydropyrazolo[4,3-b][(1,5]benzodiazepin-10-on(2) zeigen läßt.

Tabelle I

Antiulcerogene Wirkung und Toxizität von Pyrazolobenzodiazepinonen

| Lfd.Nr. - | Toxizität $LD_{50}$[mg/kg] i.v. Maus | Magenschutzwirkung $ED_{50}$[mg/kg] p.o. Ratte | TQ $LD_{50}/ED_{50}$ | Magensekretion+ % Hemmung Ratte |
|---|---|---|---|---|
| 1 | 290 | ~ 70 | 4,1 | 7 |
| 2 | 210 | 2,5 | 84,0 | 20 |

$ED_{50}$ = Dosis, die den Ulcusindex bei der behandelten Gruppe gegenüber der Kontrollgruppe um 50 % mindert
$LD_{50}$ = Dosis, bei der 50 % der Tiere sterben
TQ = Therapeutischer Quotient $LD_{50}/ED_{50}$
+) % Hemmung = Hemmung der Magensekretion (in %) 4 Stunden nach Applikation der antiulcerogenen $ED_{50}$

Es sei besonders darauf hingewiesen, daß bei Verbindung 1 zwar eine $ED_{50}$ noch bestimmbar ist, die Dosiswirkungskurve dann jedoch sehr stark abflacht, so daß selbst bei 300 mg/kg keine wesentliche Steigerung der antiulcerogenen Wirkung erreichbar ist. Im Gegensatz dazu ist die Wirkung von Verbindung 2 streng dosisabhängig; es lassen sich Hemmeffekte bis 95 % (30 mg/kg) erreichen.

Die Prüfung der antiulcerogenen Wirkung erfolgte nach der Methode der sogenannten Shay-Ratte:

Die Ulcusprovokation erfolgt bei 24 Stunden nüchtern gehaltenen Ratten (weiblich, 180–200 g, 4 Tiere je Käfig auf hohem Gitterrost) durch Pylorusligatur (unter Diethylethernarkose) und orale Applikation von 100 mg/10 ml kg Acetylsalicylsäure. Die zu testenden Substanzen werden oral (10 ml/kg) 1 Stunde vor der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen) bestimmt wurde; mit einem Stereomikroskop werden bei 10-facher Vergrößerung Anzahl und Große (= Durchmesser) vorhandener Ulcera ermittelt. Das Produkt aus Schweregrad (gemäß nachfolgender Punkteskala) und Anzahl der Ulcera dient als individueller Ulcusindex.

Punkteskala:

| | | |
|---|---|---|
| keine Ulcera | | 0 |
| Ulcusdurchmesser | 0,1–1,4 mm | 1 |
| | 1,5–2,4 mm | 2 |
| | 2,5–3,4 mm | 3 |
| | 3,5–4,4 mm | 4 |

4,5–5,4 mm 5
>5,5 mm 6

Als Maß für den antiulcerogenen Effekt dient die Minderung des mittleren Ulcus-Index jeder behandelten Gruppe gegenüber dem der Kontrollgruppe (= 100 %). Die $ED_{50}$ bezeichnet die Dosis, die den mittleren Ulcusindex um 50 % mindert.

**Bestimmung der Toxizität**
Die Toxizitätsuntersuchungen werden an weiblichen NMRI-Mäusen (Körpergewicht 22–26 g) durchgeführt. Die Tiere (5 Tiere pro Dosis) erhalten Futter und Wasser ad libitum. Verschiedene Dosen der Substanzen werden intravenös (Injektionsdauer 1 Minute) verabreicht. Die Beobachtungsdauer beträgt 7 Tage. Die $LD_{50}$, d.h. die Dosis, bei der 50 % der Tiere sterben, wird mittels linearer Regression ermittelt.

**Patentansprüche für die Vertragsstaaten BE CH DE FR GB IT U LU NL SE**

1. Substituierte Pyrazolobenzodiazepinone der allgemeinen Formel I

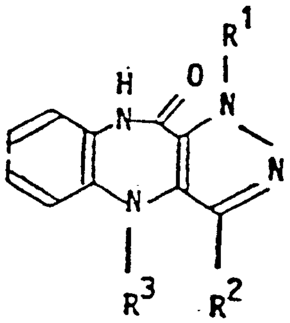

(I),

worin
$R^1$ einen Alkylrest mit 1 bis 4 Kohlenstoffatomen,
$R^2$ ein Wasserstoffatom oder einen Alkylrest mit 1 bis 4 Kohlenstoffatomen,
$R^3$ die Gruppe -CO-A-$R^4$,
$R^4$ die Gruppe -N($R^5$)$R^6$ und
$R^5$ einen Alkylrest mit 1 bis 4 Kohlenstoffatomen oder einen Allyl- oder 2-Methylallylrest bedeuten,
$R^6$ eine der Bedeutungen von $R^5$ hat oder die Gruppe $-(CH_2)_m-N(R^7)R^8$ darstellt oder
$R^5$ und $R^6$ gemeinsam unter Einschluß des Stickstoffatoms, an das sie gebunden sind, eine Morpholinogruppe, eine Pyrrolidinogruppe, eine Piperdinogruppe, eine Hexahydroazepin-1-yl-gruppe, eine gegebenenfalls in 4-Position durch eine Methyl- oder Ethylgruppe oder durch eine Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1 H-1,4-diazepin-1-yl-gruppe bedeuten und
$R^7$ eine Alkylgruppe mit 1 bis 4 Kohlenstoffatomen,
$R^8$ eine Alkylgruppe mit 1 bis 4 Kohlenstoffatomen,
A eine geradkettige oder verzweigte Alkylengruppe mit 1 bis 5 Kohlenstoffatomen und
m 2 oder 3 bedeuten,
und ihre Säureadditionssalze.

2. Substituierte Pyrazolobenzodiazepinone der allgemeinen Formel I nach Anspruch 1, worin
$R^1$ einen Methylrest,
$R^2$ einen Methylrest,
$R^3$ die Gruppe -CO-A-$R^4$,
$R^4$ die Gruppe -N($R^5$)$R^6$ und
$R^5$ einen Alkylrest mit 1 bis 4 Kohlenstoffatomen oder einen Allyl- oder Methylallylrest bedeuten,
$R^6$ eine der Bedeutungen von $R^5$ hat oder
$R^5$ und $R^6$ gemeinsam unter Einschluß des Stickstoffatoms, an das sie gebunden sind, eine Pyrrolidinogruppe, eine Piperidinogruppe, eine Hexahydroazepin-1-yl-gruppe, eine in 4-Position durch eine Methyl- oder Ethylgruppe substituierte Piperazin-1-yl-gruppe, oder eine in 4-Position durch eine Methyl- oder Ethylgruppe substituierte Hexahydro-1H-1,4-diazepin-1-yl-gruppe bedeuten und
A eine geradkettige Alkylengruppe mit 1 oder 2 Kohlenstoffatomen bedeuten,
und ihre Säureadditionssalze.

3. Substituierte Pyrazolobenzodiazepinone nach Anspruch 1, gekennzeichnet durch die allgemeine Formel I**

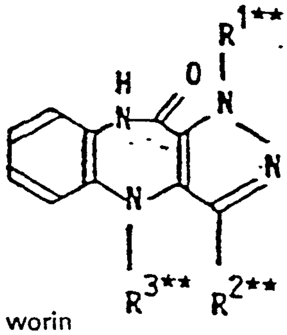

(I**),

worin
$R^{1**}$ einen Methyl- oder Ethylrest,
$R^{2**}$ ein Wasserstoffatom, einen Methyl- oder Ethylrest,
$R^{3**}$ die Gruppe -CO-A**-$R^{4**}$,
$R^{4**}$ die Gruppe -N($R^{5**}$)$R^{6**}$ und
$R^{5**}$ einen Alkylrest mit 1 bis 4 Kohlenstoffatomen oder einen Allyl- oder Methylallylrest bedeuten,
$R^{6**}$ die Bedeutung von $R^{5**}$ hat oder die Gruppe $-(CH_2)_{m**}-N(R^{7**})R^{8**}$ darstellt oder
$R^{5**}$ und $R^{6**}$ gemeinsam unter Einschluß des Stickstoffatoms, an das die gebunden sind, eine Morpholinogruppe, eine Pyrrolidinogruppe, eine Piperidinogruppe, eine Hexahydroazepin-1-yl-gruppe, eine in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexahydro-1H-1,4-diazepin-1-yl-gruppe bedeuten und
$R^{7**}$ eine Methyl- oder Ethylgruppe,
$R^{8**}$ eine Methyl- oder Ethylgruppe,
m** 2 oder 3,
A** eine geradkettige oder verzweigte Alkylengruppe mit 1 oder 2 Kohlenstoffatomen bedeuten,
und ihre Säureadditionssalze.

4. Verbindungen nach Anspruch 3, in denen $R^{1**}$ einen Methyl- oder Ethylrest, $R^{2**}$ ein Wasserstoffatom, einen Methyl- oder Ethylrest, $R^{5**}$ einen Methyl- oder Ethylrest bedeuten, $R^{6**}$ die Bedeutung von $R^{5**}$ hat oder die Gruppe $(CH_2)_{m**}-N(R^{7**})R^{8**}$ darstellt oder $R^{5**}$ und $R^{6**}$ gemeinsamt unter Einschluß des Stickstoffatoms einen Pyrrolidino-, Piperidino- oder Hexahydroazepin-1-yl-rest, $R^{7**}$ und $R^{8**}$ einen Methyl- oder Ethylrest, m** 2 und A** eine Methylengruppe bedeuten, und ihre pharmakologisch verträglichen Säureadditionssalze.

5. Verbindungen nach Anspruch 3, in denen $R^{1**}$ einen Methyl- oder Ethylrest, $R^{2**}$ ein Wasserstoffatom, einen Methyl- oder Ethylrest, $R^{5**}$ und $R^{6**}$ gemeinsam unter Einschluß des Stickstoffatoms einen in 4-Stellung durch eine Methyl-, Ethyl- oder Benzylgruppe substituierte Piperazin-1-yl-gruppe, eine 2,4-Dimethyl-piperazin-1-yl-gruppe oder eine in 4-Stellung durch eine Methyl- oder Ethylgruppe substituierte Hexahydro-1H-1,4-diazepin-1-yl-gruppe und A** eine Methylengruppe bedeuten, und ihre pharmakologisch verträglichen Säureadditionssalze.

6. Verbindungen nach Anspruch 3, in denen $R^{1**}$ einen Methyl- oder Ethylrest, $R^{2**}$ ein Wasserstoffatom, einen Methyl- oder Ethylrest, $R^{5**}$ und $R^{6**}$ gemeinsam unter Einschluß des Stickstoffatoms eine in 4-Stellung durch eine Methylgruppe substituierte Piperazin-1-yl-gruppe und A** eine Methylengruppe bedeuten, und ihre pharmakologisch verträglichen Säureadditionssalze.

7. Verbindung nach Anspruch 3, in der $R^{1**}$ und $R^{2**}$ einen Methylrest, $R^{5**}$ und $R^{6**}$ unter Einschluß des Stickstoffatoms eine in 4-Position durch eine Methylgruppe substituierte Piperazin-1-yl-gruppe und A** eine Methylengruppe bedeuten, und ihre pharmakologisch verträglichen Säureadditionssalze.

8. Verbindung nach Anspruch 3, in der $R^{1**}$ einen Methylrest, $R^{2**}$ ein Wasserstoffatom, $R^{5**}$ und $R^{6**}$ unter Einschluß des Stickstoffatoms eine in 4-Position durch eine Methylgruppe substituierte Piperazin-1-yl-gruppe und A** eine Methylengruppe bedeuten, und ihre pharmakologisch verträglichen Säureadditionssalze.

9. Arzneimittel, enthaltend ein oder mehrere Pyrazolobenzodiazepinone der allgemeinen Formel I nach einem der Ansprüche 1 bis 8 und/oder ihre pharmakologisch verträglichen Säureadditionssalze.

10. Verfahren zur Herstellung der substituierten Pyrazolobenzodiazepinone der allgemeinen Formel I nach Anspruch 1 und ihrer Säureadditionssalze, dadurch gekennzeichnet, daß man Pyrazolobenzodiazepinone der allgemeinen Formel II

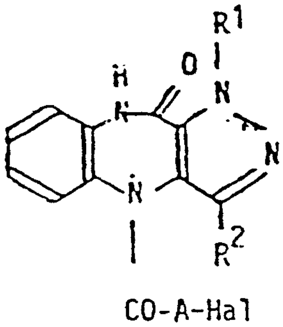

(II),

worin
$R^1$, $R^2$ und A die in Anspruch 1 angegebene Bedeutung haben und Hal ein Halogenatom bedeutet, mit sekundären

Aminen der allgemeinen Formel V

$$HN(R^5)R^6 \quad (V),$$

worin
$R^5$ und $R^6$ die in Anspruch 1 angegebene Bedeutung haben, umsetzt und gegebenenfalls anschließend erhaltene Basen in die Säureadditionssalze oder erhaltene Säureadditionssalze in die freie Base oder pharmakologisch verträgliche Säureadditionssalze überführt.

11. Verfahren nach Anspruch 10, dadurch gekennzeichnet, daß man Verbindungen II, in denen $R^1$ und $R^2$ eine Methylgruppe und A eine Methylen- oder Ethylengruppe bedeuten, und sekundäre Amine V, in denen $R^5$ eine Alkylgruppe mit 1 bis 4 Kohlenstoffatomen oder einen Allyl- oder 2-Methylallylrest bedeutet, $R^6$ eine der Bedeutungen von $R^5$ hat oder $R^5$ und $R^6$ gemeinsam eine Pyrrolidino-, Piperidino- oder Hexahydroazepin-1-yl-gruppe, eine in 4-Position durch eine Methyl- oder Ethylgruppe substituierte Piperazinogruppe oder eine in 4-Position durch eine Methyl- oder Ethylgruppe substituierte Hexahydro-1H-1,4-diazepin-1-yl-gruppe bedeuten, einsetzt.

12. Wirkstoffe nach einem der Ansprüche 1 bis 8 zur Behandlung von Krankheiten, die auf Erkrankungen des Magens oder Darms beruhen.

13. Zwischenprodukte der allgemeinen Formel I

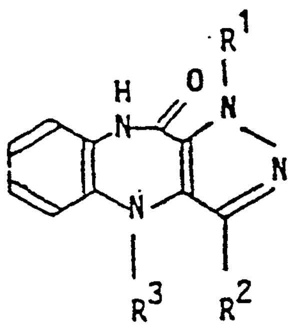 (I),

worin
$R^1$ einen Alkylrest mit 1 bis 4 Kohlenstoffatomen,
$R^2$ ein Wasserstoffatom oder einen Alkylrest mit 1 bis 4 Kohlenstoffatomen,
$R^3$ ein Wasserstoffatom oder die Gruppe-CO-A-$R^4$,
$R^4$ ein Halogenatom und
A ein geradkettige oder verzweigte Alkylengruppe mit 1 bis 5 Kohlenstoffatomen bedeuten,
und ihre Säureadditionssalze.

14. Zwischenprodukte der allgemeinen Formel I nach Anspruch 13, worin
$R^1$ einen Methylrest,
$R^2$ einen Methylrest,
$R^3$ ein Wasserstoffatom oder die Gruppe -CO-A-$R^4$,
$R^4$ ein Halogenatom und
A eine geradkettige Alkylengruppe mit 1 oder 2 Kohlenstoffatomen bedeuten,
und ihre Säureadditionssalze.

15. Zwischenprodukte nach Anspruch 13, gekennzeichnet durch die allgemeine Formel I*

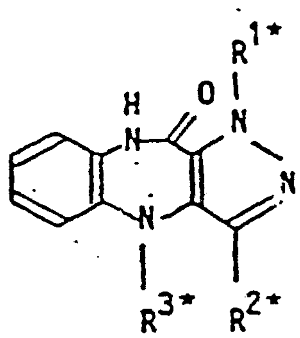 (I*),

worin
$R^{1*}$ einen Methyl- oder Ethylrest,
$R^{2*}$ ein Wasserstoffatom, einen Methyl- oder Ethylrest,
$R^{3*}$ ein Wasserstoffatom oder die Gruppe -CO-A*-$E^{4*}$
$R^{4*}$ ein Chloratom und
A* eine geradkettige oder verzweigte Alkylengruppe mit 1 oder 2 Kohlerstoffatomen bedeuten,
und ihre Säureadditionssalze.

16. Verbindungen nach Anspruch 15, in denen A* eine Methylengruppe bedeutet.

17. Verbindungen nach Anspruch 15, in denen $R^{1*}$ einen Methyl- oder Ethyrlrest und $R^{2*}$ ein Wasserstoffatom, einen Methyl- oder Ethylrest, $R^{3*}$ ein Wasserstoffatom oder die Gruppe $-CO-CH_2-C1$ bedeuten, und ihre Säureadditionssalze.

18. Verfahren zur Herstellung Zwischenprodukte der allgemeinen Formel I

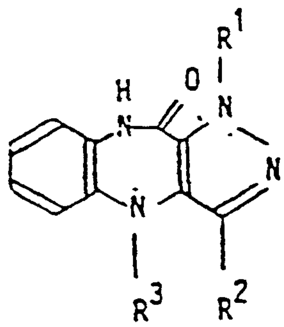

(I),

worin
$R^1$ einen Alkylrest mit 1 bis 4 Kohlenstoffatomen,
$R^2$ ein Wasserstoffatom oder einen Alkylrest mit 1 bis 4 Kohlenstoffatomen,
$R^3$ ein Wasserstoffatom oder die Gruppe $-CO-A-R^4$,
$R^4$ ein Halogenatom und
A eine geradkettige oder verzweigte Alkylengruppe mit 1 bis 5 Kohlenstoffatomen bedeuten,
und ihrer Säureadditionssalze, dadurch gekennzeichnet, daß man Anilinopyrazol-carbonsäurederivate der allgemeinen Formel II

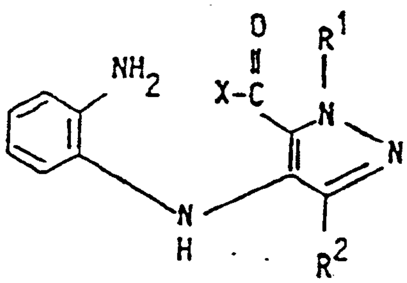

(II),

worin
X eine Fluchtgruppe bedeutet,
cyclisiert und gegebenenfalls anschließend in dem erhaltenen Reaktionsprodukt der Formel I, worin $R^3$ ein Wasserstoffatom bedeutet, das Wasserstoffatom durch eine $-CO-A-R^4$-Gruppe substituiert und/oder erhaltene Basen in die Säureadditionssalze oder erhaltene Säureadditionssalze in die freie Base überführt.

19. Verfahren nach Anspruch 18, dadurch gekennzeichnet, daß man Verbindungen II einsetzt, in denen X eine -OH-Gruppe, eine $O-R^9$-Gruppe oder eine $-N(R^{10})R^{11}$-Gruppe darstellt, worin
$R^9$ eine Alkylgruppe, eine Cycloalkylgruppe, eine gegebenenfalls substituierte Aralkylgruppe oder eine gegebenenfalls substituierte Arylgruppe und
$R^{10}$ ein Wasserstoffatom, eine Alkylgruppe, eine Cycloalkylgruppe, eine gegebenenfalls substituierte Aralkylgruppe oder eine gegebenenfalls substituierte Arylgruppe bedeuten,
$R^{11}$ eine der Bedeutungen von $R^{10}$ hat oder
$R^{10}$ und $R^{11}$ gemeinsam unter Einschluß des Stickstoffatoms, an das sie gebunden sind, einen gegebenenfalls substituierten nichtaromatischen heterocyclischen Rest bedeuten.

20. Verfahren nach Anspruch 19, dadurch gekennzeichnet, daß man Verbindungen II einsetzt, in denen
$R^1$ und $R^2$ eine Methylgruppe und
X eine -OH-Gruppe oder eine $-O-R^9$-Gruppe darstellen, worin
$R^9$ eine Alkylgruppe, eine gegebenenfalls substituierte Aralkylgruppe oder eine gegebenenfalls substituierte Arylgruppe bedeutet.

21. Verfahren nach Anspruch 18, dadurch gekennzeichnet, daß man die sich gegebenenfalls anschließende Substitution mit Verbindungen der allgemeinen Formeln Hal-A-CO-Hal' (III) oder $(Hal-A-CO)_2O$ (IV), worin Hal und Hal' ein Halogenatom und A eine Alkylengruppe mit 1 bis 5 Kohlenstoffatomen bedeuten, durchführt.

**Patentansprüche für den Vertragsstast AT**

1. Verfahren zur Herstellung von substituierten Pyrazolobenzodiazepinonen der allgemeinen Formel I

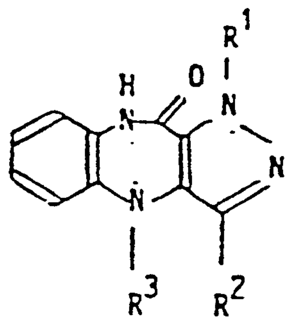

(I),

worin
$R^1$ einen Alkylrest mit 1 bis 4 Kohlenstoffatomen,
$R^2$ en Wasserstoffatom oder einen Alkylrest mit 1 bis 4 Kohlenstoffatomen,
$R^3$ die Gruppe -CO-A-$R^4$,
$R^4$ die Gruppe -N($R^5$)$R^6$ und
$R^5$ einen Alkylrest mit 1 bis 4 Kohlenstoffatomen oder einen Allyl- oder 2-Methylallylrest bedeuten,
$R^6$ eine der Bedeutungen von $R^5$ hat oder die Gruppe -$(CH_2)$m-N($R^7$)$R^8$ darstellt oder
$R^5$ und $R^6$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl- oder Ethylgruppe oder durch eine Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1 H-1,4-diazepin-1-yl-gruppe bedeuten und
$R^7$ eine Alkylgruppe mit 1 bis 4 Kohlenstoffatomen,
$R^8$ eine Alkylgruppe mit 1 bis 4 Kohlenstoffatomen,
A eine geradkettige oder verzweigte Alkylengruppe mit 1 bis 5 Kohlenstoffatomen und
m 2 oder 3 bedeuten,
und ihren Säureadditionssalzen, dadruch gekennzeichnet, daß man Pyrazolobenzodiazepinone der allgemeinen Formel II

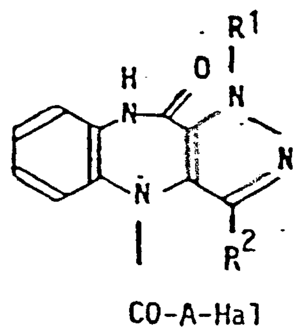

(II),

worin
$R^1$, $R^2$ und A die in Anspruch 1 angegebene Bedeutung haben und Hal ein Halogenatom bedeutet, mit sekundären Aminen der allgemeinen Formel V

$$HN(R^5)R^6 \qquad (V),$$

worin
$R^5$ und $R^6$ die in Anspruch 1 angegebene Bedeutung haben, umsetzt und gegebenenfalls anschließend erhaltene Basen in die Säureadditionssalze oder erhaltene Säureadditionssalze in die freie Base oder pharmakologisch verträgliche Säureadditionssalze überführt.

2. Verfahren nach Anspruch 1, dadurch gekennzeichnet, daß man Verbindungen II, in denen $R^1$ und $R^2$ eine Methylgruppe und A eine Methylen- oder Ethylengruppe bedeuten, und sekundäre Amine V, in denen $R^5$ eine Alkylgruppe mit 1 bis 4 Kohlenstoffatomen oder einen Allyl- oder 2-Methylallylrest bedeuten,
$R^6$ eine der Bedeutungen von $R^5$ hat oder
$R^5$ und $R^6$ gemeinsam eine Pyrrolidino-, Piperidino- oder Hexahydroazepin-1-yl-gruppe, eine in 4-Position durch eine Methyl- oder Ethylgruppe substituierte Piperazinogruppe oder eine in 4-Position durch eine Methyl- oder Ethylgruppe substituierte Hexahydro-1H-1,4-diazepin-1-yl-gruppe-bedeuten, einsetzt.

3. Verfahren nach Anspruch 1, dadurch gekennzeichnet, daß man substituierte Pyrazolobenzodiazepinone der allgemeinen Formel I''

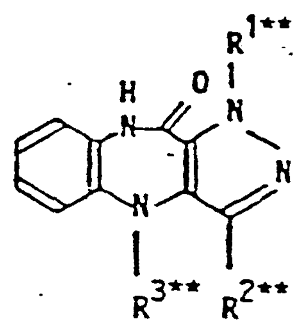

(I**),

worin
$R^{1**}$ einen Methyl-oder Ethylrest,
$R^{2**}$ ein Wasserstoffatom, einen Methyl- oder Ethylrest,
$R^{3**}$ die Gruppe -CO-A**-$R^{4**}$,
$R^{4**}$ die Gruppe -N($R^{5**}$)$R^{6**}$ und
$R^{5**}$ einen Alkylrest mit 1 bis 4 Kohlenstoffatomen oder einen Allyl- oder 2-Methylallylrest bedeuten,
$R^{6**}$ die Bedeutung von $R^{5**}$ hat oder die Gruppe $-(CH_2)_{m^{**}}-N(R^{7**})R^{8**}$
darstellt oder
$R^{5**}$ und $R^{6**}$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexahydro-1H-1,4-diazepin-1-yl-gruppe bedeuten und
$R^{7**}$ eine Methyl- oder Ethylgruppe,
$R^{8**}$ eine Methyl- oder Ethylgruppe,
$m^{**}$ 2 oder 3,
A** eine geradkettige oder verzweigte Alkylengruppe mit 1 oder 2 Kohlenstoffatomen bedeuten,
oder ihre Säureadditionssalze durch Umsetzung von Pyrazolobenzodiazepinonen der allgemeinen Formel II**

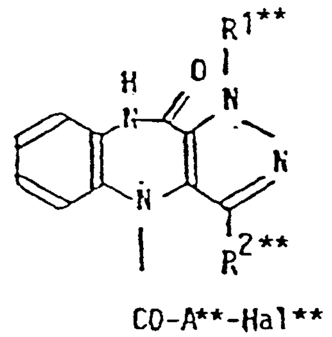

(II**),

worin
$R^{1**}$, $R^{2**}$ und A** die oben angegebene Bedeutung haben und Hal** ein Halogenatom darstellt, mit sekundären Aminen der allgemeinen Formel V**

$$HN(R^{5**})R^{6**} \quad (V^{**}),$$

worin $R^{5**}$ und $R^{6**}$ die oben angegebene Bedeutung haben, herstellt.

4. Verfahren nach Anspruch 3, dadurch gekennzeichnet, daß man substituierte Pyrazolobenzodiazepinone der allgemeinen Formel II**, worin $R^{1**}$ einen Methyl- oder Ethylrest, $R^{2**}$ ein Wasserstoffatom, einen Methyl- oder Ethylrest, $R^{5**}$ einen Methyl- oder Ethylrest bedeuten, $R^{6**}$ die Bedeutung von $R^{5**}$ hat oder die Gruppe $(CH_2)_{m^{**}}-N(R^{7**})(R^{8**}$ darstellt oder $R^{5**}$ und $R^{6**}$
gemeinsam unter Einschluß des Stickstoffatoms einen Pyrrolidino-, Piperidino- oder Hexahydroazepin-1-yl-rest, $R^{7**}$ und $R^{8**}$ einen Methyl- oder Ethylrest, m** 2 und A** eine Methylengruppe bedeuten, oder ihre pharmakologisch verträglichen Säureadditionssalze, herstellt.

5. Verfahren nach Anspruch 3, dadurch gekennzeichnet, daß man substituierte Pyrazolobenzodiazepinone der allgemeinen Formel I**, worin $R^{1**}$ einen Methyl- oder Ethylrest, $R^{2**}$ ein Wasserstoffatom, einen Methyl- oder Ethylrest, $R^{5**}$ und $R^{6**}$ gemeinsam unter Einschluß des Stickstoffatoms eine in 4-Stellung durch eine Methyl-, Ethyl- oder Benzylgruppe substituierte Piperazin-1-yl-gruppe, eine 2,4-Dimethyl-piperazin-1-yl-gruppe oder eine in 4-Stellung durch eine Methyl- oder Ethylgruppe substituierte Hexahydro-1H-1,4-diazepin-1-yl-gruppe und A** eine Methylengruppe bedeuten, oder ihre pharmakologisch verträglichen Säureadditionssalze herstellt.

6. Verfahren nach Anspruch 3, dadurch gekennzeichnet, daß man substituierte Pyrazolobenzodiazepinone der allgemeinen Formel I**, worin $R^{1**}$ einen Methyl- oder Ethylrest, $R^{2**}$ ein Wasserstoffatom, einen Methyl- oder Ethylrest, $R^{5**}$ und $R^{6**}$ gemeinsam unter Einschluß des Stickstoffatoms eine in 4-Stellung durch eine Methylgruppe substituierte Piperazin-1-yl-gruppe und A** eine Methylengruppe bedeuten, oder ihre pharmakologisch verträglichen Säureadditionssalze herstellt.

7. Verfahren nach Anspruch 3, dadurch gekennzeichnet, daß man das substituierte Pyrazolobenzodiazepinon der allgemeinen Formel I**, worin $R^{1**}$ und $R^{2**}$ einen Methylrest, $R^{5**}$ und $R^{6**}$ unter Einschluß des Stickstoffatoms eine in 4-Position durch eine Methylgruppe substituierte Piperazin-1-yl-gruppe und A** eine Methylengruppe bedeuten, oder seine pharmakologisch verträglichen Säureadditionssalze herstellt.

8. Verfahren nach Anspruch 3, dadurch gekennzeichnet, daß man das substituierte Pyrazolobenzodiazepinon der allgemeinen Formel I**, worin $R^{1**}$ einen Methylrest, $R^{2**}$ ein Wasserstoffatom, $R^{5**}$ und $R^{6**}$ unter Einschluß des Stickstoffatoms eine in 4-Position durch eine Methylgruppe substituierte Piperazin-1-yl-gruppe und A** eine Methylengruppe bedeuten, oder seine pharmakologisch verträglichen Säureadditionssalze herstellt.

9. Verfahren zur Herstellung der Zwischenprodukte der allgemeinen Formel I

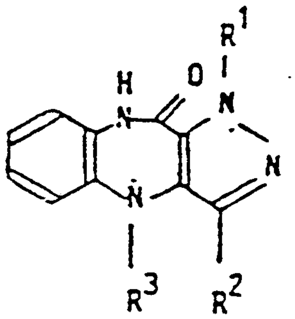

(I),

worin
$R^1$ einen Alkylrest mit 1 bis 4 Kohlenstoffatomen,
$R^2$ ein Wasserstoffatom oder einen Alkylrest mit 1 bis 4 Kohlenstoffatomen,
$R^3$ ein Wasserstoffatom oder die Gruppe -CO-A-$R^4$,
$R^4$ ein Halogenatom und
A eine geradkettige oder verzweigte Alkylengruppe mit 1 bis 5 Kohlenstoffatomen bedeuten,
und ihrer Säureadditionssalze, dadurch gekennzeichnet, daß man Anilinopyrazol-carbonsäurederivate der allgemeinen Formel II

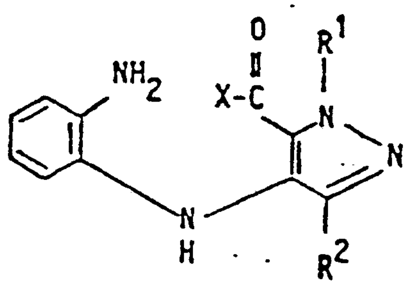

(II),

worin
X eine Fluchtgruppe bedeutet,
cyclisiert und gegebenenfalls anschließend in dem erhaltenen Reaktionsprodukt der Formel I, worin $R^3$ ein Wasserstoffatom bedeutet, das Wasserstoffatom durch eine -CO-A-$R^4$-Gruppe substituiert und/oder erhaltene Basen in die Säureadditionssalze oder erhaltene Säureadditionssalze in die freie Base überführt.

10. Verfahren nach Anspruch 9, dadurch gekennzeichnet, daß man Verbindungen II einsetzt, in denen X eine -OH-Gruppe, eine O-$R^9$-Gruppe oder eine -N($R^{10}$)$R^{11}$-Gruppe darstellt, worin
$R^9$ eine Alkylgruppe, eine Cycloalkylgruppe, eine gegebenenfalls substituierte Aralkylgruppe oder eine gegebenenfalls substituierte Arylgruppe und
$R^{10}$ ein Wasserstoffatom, eine Alkylgruppe, eine Cycloalkylgruppe, eine gegebenenfalls substituierte Aralkylgruppe oder eine gegebenenfalls substituierte Arylgruppe bedeuten,
$R^{11}$ eine der Bedeutungen von $R^{10}$ hat oder
$R^{10}$ und $R^{11}$ gemeinsam unter Einschluß des Stickstoffatoms, an das sie gebunden sind, einen gegebenenfalls substituierten nichtaromatischen heterocyclischen Rest bedeuten.

11. Verfahren nach Anspruch 9, dadurch gekennzeichnet, daß man Verbindungen II einsetzt, in denen
$R^1$ und $R^2$ eine Methylgruppe und
X eine -OH-Gruppe oder eine -O-$R^9$-Gruppe darstellen, worin
$R^9$ eine Alkylgruppe, eine gegebenenfalls substituierte Aralkylgruppe oder eine gegebenenfalls substituierte Arylgruppe bedeutet.

12. Verfahren nach Anspruch 9, dadurch gekennzeichnet, daß man die sich gegebenenfalls anschließende Substitution mit Verbindungen der allgemeinen Formeln Hal-A-CO-Hal' (III) oder $(Hal\text{-}A\text{-}CO)_2O$ (IV), worin Hal und Hal' ein Halogenatom und A eine Alkylengruppe mit 1 bis 5 Kohlenstoffatomen bedeuten, durchführt.

13. Verfahren nach Anspruch 12, dadurch gekennzeichnet, daß man Verbindungen der allgemeinen Formel III oder IV, in denen A eine Methylen- oder Ehtylengruppe bedeutet, einsetzt.

14. Verfahren nach Anspruch 9, 10 oder 12, dadurch gekennzeichnet, daß man Zwischenprodukte der allgemeinen Formel I*

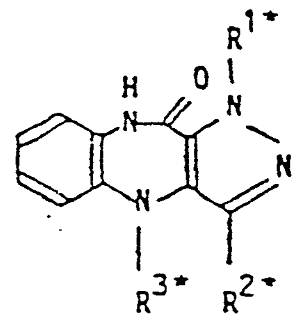

(I*),

worin
$R^{1*}$ einen Methyl- oder Ethylrest,
$R^{2*}$ ein Wasserstoffatom, einen Methyl- oder Ethylrest,
$R^{3*}$ ein Wasserstoffatom oder die Gruppe -CO-A*-$R^{4*}$
$R^{4*}$ ein Chloratom und
A* eine geradkettige oder verzweigte Alkylengruppe mit 1 oder 2 Kohlenstoffatomen bedeuten,
oder ihre Säureadditionssalze herstellt.

15. Verfahren nach Anspruch 10, dadurch gekennzeichnet, daß man eine Verbindung II einsetzt, in der $R^1$ und $R^2$ eine Methylgruppe, X eine -N($R^{10}$)$R^{11}$-Gruppe und $R^{10}$ und $R^{11}$ ein Wasserstoffatom bedeuten.

16. Verfahren nach Anspruch 11, dadurch gekennzeichnet, daß man eine Verbindung II einsetzt, in der X eine -O-$R^9$-Gruppe und $R^9$ eine Alkylgruppe bedeuten.

17. Verfahren nach Anspruch 11 und 12, dadurch gekennzeichnet, daß man Chloracetylchlorid als Verbindung III einsetzt.

**Claims for the contracting states BE, CH, DE, FR, GB, IT, LI, LU, NL, SE**

1. Substituted pyrazolobenzodiazepinones of the general formula I

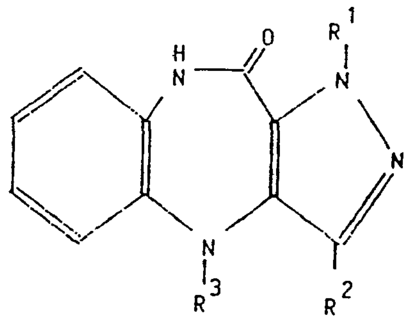

(I)

wherein
$R^1$ denotes an alkyl radical with 1 to 4 carbon atoms,
$R^2$ denotes a hydrogen atom or an alkyl radical with 1 to 4 carbon atoms,
$R^3$ denotes the group -CO-A-$R^4$,
$R^4$ denotes the group -N($R^5$)$R^6$ and
$R^5$ denotes an alkyl radical with 1 to 4 carbon atoms or an allyl or 2-methylallyl radical,
$R^6$ has one of the meanings of $R^5$ or represents the group $-(CH_2)_m-N(R^7)R^8$, or
$R^5$ and $R^6$ together, and including the nitrogen atom to which they are linked, denote a morpholino group, a pyrrolidino group, a piperidino group, a hexahydroazepin-1-yl group, a piperazin-1-yl group which is optionally substituted in the 4-position by a methyl or ethyl group or by a benzyl group, a 2,4-dimethylpiperazin-1-yl group or a hexahydro-1H-1,4-diazepin-1-yl group which is substituted in the 4-position by a methyl or ethyl group, and
$R^7$ denotes an alkyl group with 1 to 4 carbon atoms,
$R^8$ denotes an alkyl group with 1 to 4 carbon atoms,
A denotes a straight-chain or branched alkylene group with 1 to 5 carbon atoms and
m denotes 2 or 3,
and their acid addition salts.

2. Substituted pyrazolobenzodiazepinones of the general formula I according to Claim 1, wherein
$R^1$ denotes a methyl radical,
$R^2$ denotes a methyl radical,
$R^3$ denotes the group -CO-A-$R^4$,

$R^4$ denotes the group $-N(R^5)R^6$ and
$R^5$ denotes an alkyl radical with 1 to 4 carbon atoms or an allyl or methylallyl radical,
$R^6$ has one of the meanings of $R^5$, or
$R^5$ and $R^6$ together, and including the nitrogen atom to which they are linked, denote a pyrrolidino group, a piperidino group, a hexahydroazepin-1-yl group, a piperazin-1-yl group which is substituted in the 4-position by a methyl or ethyl group, or a hexahydro-1H-1,4-diazepin-1-yl group which is substituted in the 4-position by a methyl or ethyl group, and
A denotes a straight-chain alkylene group with 1 or 2 carbon atoms, and their acid addition salts.

3. Substituted pyrazolobenzodiazepinones according to Claim I, characterised in that they have the general formula I**

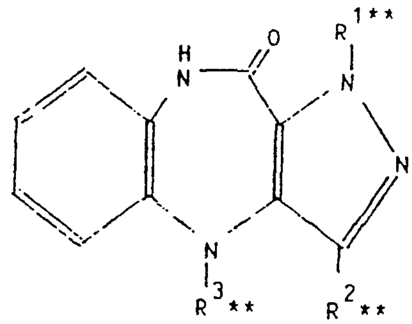

(I**)

wherein
$R^{1**}$ denotes a methyl or ethyl radical,
$R^{2**}$ denotes a hydrogen atom or a methyl or ethyl radical,
$R^{3**}$ denotes the group $-CO-A^{**}-R^{4**}$,
$R^{4**}$ denotes the group $-N(R^{5**})R^{6**}$ and
$R^{5**}$ denotes an alkyl radical with 1 to 4 carbon atoms or an allyl or methylallyl radical,
$R^{6**}$ has the meaning of $R^{5**}$ or represents the group
$-(CH_2)_m{}^{**}-N(R^{7**})R^{8**}$, or
$R^{5**}$ and $R^{6**}$ together, and including the nitrogen atom to which they are linked, denote a morpholino group, a pyrrolidino group, a piperidino group, a hexahydroazepin-1-yl group, a piperazin-1-yl group which is substituted in the 4-position by a methyl, ethyl or benzyl group, a 2,4-dimethylpiperazin-1-yl group or a hexahydro-1H-1,4-diazepin-1-yl group which is substituted in the 4-position by a methyl or ethyl group, and
$R^{7**}$ denotes a methyl or ethyl group,
$R^{8**}$ denotes a methyl or ethyl group,
m** denotes 2 or 3,
A** denotes a straight-chain or branched alkylene group with 1 or 2 atoms,
and their acid addition salts.

4. Compound according to Claim 3, in which $R^{1**}$ denotes a methyl or ethyl radical, $R^{2**}$ denotes a hydrogen atom or a methyl or ethyl radical, $R^{5**}$ denotes a methyl or ethyl radical, $R^{6**}$ has the meaning of $R^{5**}$ or represents the group $(CH_2)_m{}^{**}-N(R^{7**})R^{8**}$, or $R^{5**}$ and $R^{6**}$ together, and including the nitrogen atom, denote a pyrrolidino, piperidino or hexahydroazepin-1-yl radical, $R^{7**}$ and $R^{8**}$ denote a methyl or ethyl radical, m** denotes 2 and A** denotes a methylene group, and their pharmacologically acceptable acid addition salts.

5. Compounds according to Claim 3, in which $R^{1**}$ denotes a methyl or ethyl radical, $R^{2**}$ denotes a hydrogen atom or a methyl or ethyl radical, $R^{5**}$ and $R^{6**}$ together, and including the nitrogen atom, denote a piperazin-1-yl group which is substituted in the 4-position by a methyl, ethyl or benzyl group, a 2,4-dimethylpiperazin-1-yl group or a hexahydro-1H-1,4-diazepin-1-yl group which is substituted in the 4-position by a methyl or ethyl group and A** denotes a methylene group, and their pharmacologically acceptable acid addition salts.

6. Compounds according to Claim 3, in which $R^{1**}$ denotes a methyl or ethyl radical, $R^{2**}$ denotes a hydrogen atom or a methyl or ethyl radical, $R^{5**}$ and $R^{6**}$ together, and including the nitrogen atom, denote a piperazin-1-yl group which is substituted in the 4-position by a methyl group and A** denotes a methylene group, and their pharmacologically acceptable acid addition salts.

7. Compound according to Claim 3, in which $R^{1**}$ and $R^{2**}$ denote a methyl radical, $R^{5**}$ and $R^{6**}$, including the nitrogen atom, denote a piperazin-1-yl group which is substituted in the 4-position by a methyl group and A** denotes a methylene group, and their pharmacologically acceptable acid addition salts.

8. Compound according to Claim 3, in which $R^{1**}$ denotes a methyl radical, $R^{2**}$ denotes a hydrogen atom, $R^{5**}$ and $R^{6**}$ together, and including the nitrogen atom, denote a piperazin-1-yl group which is substituted in the 4-position by a methyl group and A** denotes a methylene group, and their pharmacologically acceptable acid

addition salts.

9. Medicaments containing one or more pyrazolobenzodiazepionones of the general formula I according to one of the Claims 1 to 8 and/or their pharmacologically acceptable acid addition salts.

10. Process for the preparation of the substituted pyrazolobenzodiazepinones of the general formula I according to Claim 1, and their acid addition salts, characterized in that pyrazolobenzodiazepinones of the general formula II

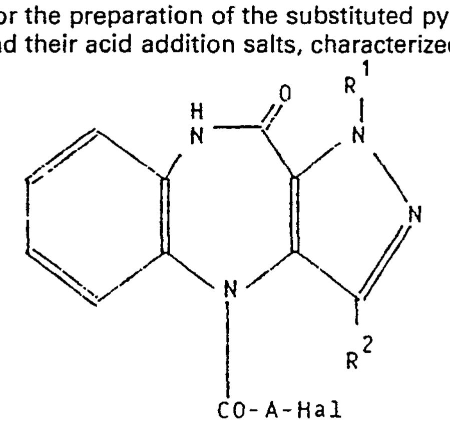

(II)

wherein
$R^1$, $R^2$ and A have the meaning given in Claim 1 and Hal denotes a halogen atom, are reacted with secondary amines of the general formula V

$$HN(R^5)R^6 \quad (V),$$

wherein
$R^5$ and $R^6$ have the meaning given in Claim 1, and optionally resulting bases are subsequently converted into the acid addition salts or resulting acid addition salts are converted into the free base or into pharmacologically acceptable acid addition salts.

11. Process according to Claim 10, characterized in that compounds II, wherein $R^1$ and $R^2$ denote a methyl group, and A denotes a methylene group or an ethylene group, and secondary amines V, wherein $R^5$ denotes an alkyl radical with 1 to 4 carbon atoms or an allyl or methylallyl radical, $R^6$ has one of the meanings of $R^5$, or $R^5$ and $R^6$ together, and including the nitrogen atom to which they are linked, denote a pyrrolidino group, a piperidino group, a hexahydroazepin-1-yl group, a piperazin-1-yl group which is substituted in the 4-position by a methyl or ethyl group, or a hexahydro-1H-1,4-diazepin-1-yl group which is substituted in the 4-position by a methyl or ethyl group, are used.

12. Active compounds according to one of the claims 1 to 8 for the treatment of diseases caused by affections of the stomach or intestines.

13. Intermediates of the general formula I

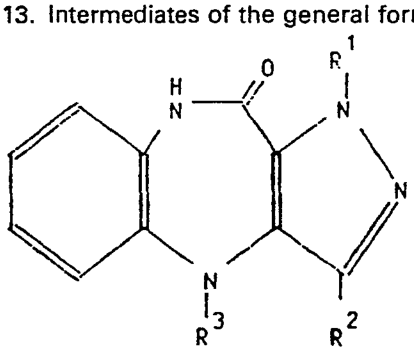

(I

wherein
$R^1$ denotes an alkyl radical with 1 to 4 carbon atoms,
$R^2$ denotes hydrogen atom or an alkyl radical with 1 to 4 carbon atoms,
$R^3$ denotes a hydrogen atom or the group -CO-A-$R^4$,
$R^4$ denotes a halogen atom and
A denotes a straight-chain or branched alkylene group with 1 to 5 carbon atoms,
and their acid addition salts.

14. Intermediates of the general formula I according to Claim 13, wherein
$R^1$ denotes a methyl group

$R^2$ denotes a methyl group
$R^3$ denotes a hydrogen atom or the group -CO-A-$R^4$,
$R^4$ denotes a halogen atom and
A denotes a straight-chain alkylene group with 1 or 2 carbon atoms,
and their acid addition salts.

15. Intermediates according to Claim 13, characterized in that they have the general formula I*

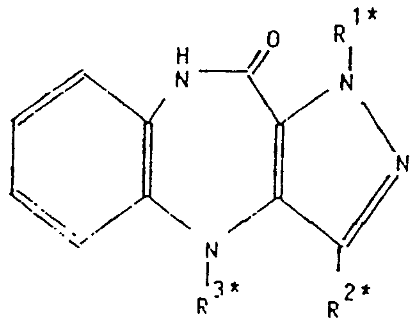

wherein
$R^{1*}$ denotes a methyl or ethyl radical,
$R^{2*}$ denotes a hydrogen atom or a methyl or ethyl radical,
$R^{3*}$ denotes a hydrogen atom or the group -CO-A*-$R^{4*}$,
$R^{4*}$ denotes a chlorine atom and
A* denotes a straight-chain or branched alkylene group with 1 or 2 carbon atoms,
and their acid addition salts.

16. Compounds according to Claim 15, in which A* denotes a methylene group.

17. Compounds according to Claim 15, in which $R^{1*}$ denotes a methyl or ethyl radical, $R^{2*}$ denotes a hydrogen atom, a methyl or ethyl radical, $R^{3*}$ denotes a hydrogen atom or the group -CO-$CH_2$-C1, and their acid addition salts.

18. Process for the preparation of the intermediates of the general formula I

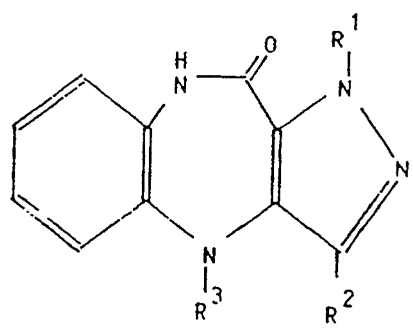

(I)

wherein
$R^1$ denotes an alkyl radical with 1 to 4 carbon atoms,
$R^2$ denotes a hydrogen atom or an alkyl radical with 1 to 4 carbon atoms,
$R^3$ denotes a hydrogen atom or the group -CO-A-$R^4$,
$R^4$ denotes a halogen atom and
A denotes a straight-chain or branched alkylene group with 1 to 5 carbon atoms, and their acid addition salts, characterized in that anilinopyrazolecarboxylic acid derivatives of the general formula II

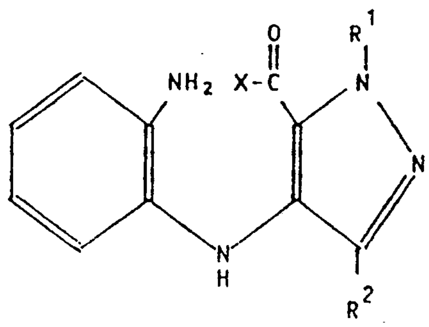

(II)

wherein X denotes a leaving group, are cyclised and, in the resulting reaction product of the formula I wherein $R^3$ denotes a hydrogen atom, the hydrogen atom is then replaced by a -CO-A-$R^4$ group, and/or resulting bases are converted into the acid addition salts or resulting acid addition salts are converted into the free base.

19. Process according to Claim 18, characterized in that compounds II in which X represents an -OH group, an O-$R^9$ group or an -N($R^{10}$)$R^{11}$ group, wherein $R^9$ denotes an alkyl group, a cycloalkyl group, an optionally substituted aralkyl group or an optionally substituted aryl group, $R^{10}$ denotes a hydrogen atom, an alkyl group, a cycloalkyl group, an optionally substituted aralkyl group or an optionally substituted aryl group and $R^{11}$ has one of the meanings of $R^{10}$, or $R^{10}$ and $R^{11}$ together, and including the nitrogen atom to which they are linked, denote an optionally substituted, non-aromatic, heterocyclic radical, are employed.

20. Process according to Claim 19, characterized in that compounds II, wherein $R^1$ and $R^2$ denote a methyl group and X denotes a -OH-group or a -O-$R^9$-group, wherein $R^9$ denotes an alkyl group, an optionally substituted aralkyl group or an optionally substituted aryl group, are employed.

21. Process according to Claim 18, characterized in that the substitution which optionally follows later is carried out by means of compounds of the general formulae Hal-A-CO-Hal' (III) or $(Hal\text{-}A\text{-}CO)_2O$ (IV), wherein Hal and Hal' denote a halogen atom and A denotes an alkylene group with 1 to 5 carbon atoms.

**Claims for the contracting state AT**

1. Process for the preparation of substituted pyrazolobenzodiazepinones of the general formula I

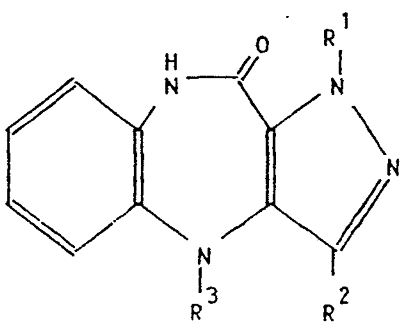

(I)

wherein
$R^1$ denotes an alkyl radical with 1 to 4 carbon atoms,
$R^2$ denotes a hydrogen atom or an alkyl radical with 1 to 4 carbon atoms,
$R^3$ denotes the group -CO-A-$R^4$,
$R^4$ denotes the group -N($R^5$)$R^6$ and
$R^5$ denotes an alkyl radical with 1 to 4 carbon atoms or an allyl or 2-methylallyl radical,
$R^6$ has one of the meanings of $R^5$ or represents the group
$-(CH_2)_m$-N($R^7$)$R^8$, or
$R^5$ and $R^6$ together, and including the nitrogen atom to which they are linked, denote a morpholino group, a pyrrolidino group, a piperidino group, a hexahydroazepin-1-yl group, a piperazin-1-yl group which is optionally substituted in the 4-position by a methyl or ethyl group or by a benzyl group, a 2,4-dimethylpiperazin-1-yl group or a hexahydro-1H-1,4-diazepin-1-yl group which is substituted in the 4-position by a methyl or ethyl group, and
$R^7$ denotes an alkyl group with 1 to 4 carbon atoms,
$R^8$ denotes an alkyl group with 1 to 4 carbon atoms,
A denotes a straight-chain or branched alkylene group with 1 to 5 carbon atoms and
m denotes 2 or 3,
and their acid addition salts, characterized in that pyrazolobenzodiazepinones of the general formula II

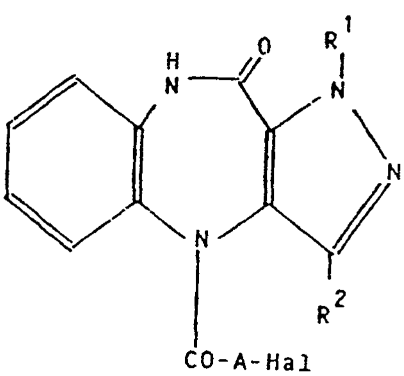

(II)

wherein
$R^1$, $R^2$ and A have the meaning given in Claim 1 and Hal denotes a halogen atom, are reacted with secondary amines of the general formula V

$$HN(R^5)R^6 \quad (V),$$

wherein
$R^5$ and $R^6$ have the meaning given in Claim 1, and optionally resulting bases are subsequently converted into the acid addition salts or resulting acid addition salts are converted into the free base or into pharmacologically acceptable acid addition salts.

2. Process according to Claim 1, charachterized in that compounds II, wherein
$R^1$ and $R^2$ denote a methyl group and
A denotes a methylene or ethylene group, and secondary amines V, wherein
$R^5$ denotes an alkyl radical with 1 to 4 carbon atoms or an allyl or methylallyl radical,
$R^6$ has one of the meanings of $R^5$, or
$R^5$ and $R^6$ together, and including the nitrogen atom to which they are linked, denote a pyrrolidino group, a piperidino group, a hexahydroazepin-1-yl group, a piperazin-1-yl group which is substituted in the 4-position by a methyl or ethyl group, or a hexahydro-1H-1,4-diazepin-1-yl group which is substituted in the 4-position by a methyl or ethyl group are used.

3. Process according to Claim 1, characterized in that substituted pyrazolobenzodiazepinones of the general formula I**

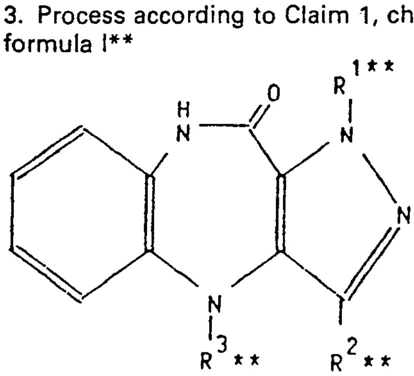

(I**)

wherein
$R^{1**}$ denotes a methyl or ethyl radical,
$R^{2**}$ denotes a hydrogen atom or a methyl or ethyl radical,
$R^{3**}$ denotes the group $-CO-A^{**}-R^{4**}$,
$R^{4**}$ denotes the group $-N(R^{5**})R^{6**}$ and
$R^{5**}$ denotes an alkyl radical with 1 to 4 carbon atoms or an allyl or methylallyl radical,
$R^{6**}$ has the meaning of $R^{5**}$ or represents the group
$-(CH_2)_m{}^{**}-N(R^{7**})R^{8**}$, or
$R^{5**}$ and $R^{6**}$ together, and including the nitrogen atom to which they are linked, denote a morpholino group, a pyrrolidino group, a piperidino group, a hexahydroazepin-1-yl group, a piperazin-1-yl group which is substituted in the 4-position by a methyl, ethyl or benzyl group, a 2,4-dimethylpiperazin-1-yl group or a hexahydro-1H-1,4-diazepin-1-yl group which is substituted in the 4-position by a methyl or ethyl group, and
$R^{7**}$ denotes a methyl or ethyl group,
$R^{8**}$ denotes a methyl or ethyl group,
m** denotes 2 or 3,
A** denotes a straight-chain or branched alkylene group with 1 or 2 atoms,
or their acid addition salts are prepared by reacting pyrazolobenzodiazepinones of the general formula II**

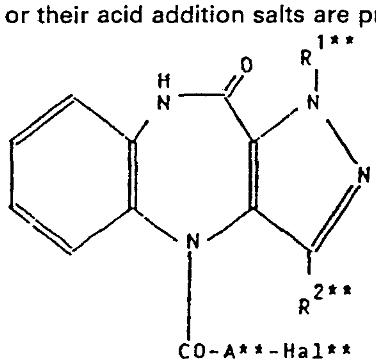

(II**)

wherein $R^{1**}$, $R^{2**}$ and A** have the meaning given above and Hal** denotes a halogen atom, with secondary

amines of the general formula V**

$$HN(R^{5**})R^{6**} \quad (V^{**})$$

wherein $R^{5**}$ and $R^{6**}$ have the meaning given above.

4. Process according to Claim 3, characterized in that pyrazolobenzodiazepinones of the general formula II**, in which $R^{1**}$ denotes a methyl or ethyl radical, $R^{2**}$ denotes a hydrogen atom or a methyl or ethyl radical, $R^{5**}$ denotes a methyl or ethyl radical, $R^{6**}$ has the meaning of $R^{5**}$ or represents the group $(CH_2)_{m}$**-$N(R^{7**})R^{8**}$, or $R^{5**}$ and $R^{6**}$ together, and including the nitrogen atom, denote a pyrrolidino, piperidino or hexahydroazepin-1-yl radical, $R^{7**}$ and $R^{8**}$ denote a methyl or ethyl radical, m** denotes 2 and A** denotes a methylene group, or their pharmacologically acceptable acid addition salts are prepared.

5. Process according to Claim 3, characterized in that pyrazolobenzodiazepinones of the general formula II**, in which $R^{1**}$ denotes a methyl or ethyl radical, $R^{2**}$ denotes a hydrogen atom or a methyl or ethyl radical, $R^{5**}$ and $R^{6**}$ together, and including the nitrogen atom, denote a piperazin-1-yl group which is substituted in the 4-position by a methyl, ethyl or benzyl group, a 2,4-dimethylpiperazin-1-yl group or a hexahydro-1H-1,4-diazepin-1-yl group which is substituted in the 4-position by a methyl or ethyl group and A** denotes a methylene group, or their pharmacologically acceptable acid addition salts are prepared.

6. Process according to Claim 3, characterized in that pyrazolobenzodiazepinones of the general formula II**, in which $R^{1**}$ denotes a methyl or ethyl radical, $R^{2**}$ denotes a hydrogen atom or a methyl or ethyl radical, $R^{5**}$ and $R^{6**}$ together, and including the nitrogen atom, denote a piperazin-1-yl group which is substituted in the 4-position by a methyl group and A** denotes a methylene group, or their pharmacologically acceptable acid addition salts are prepared.

7. Process according to Claim 3, characterized in that a pyrazolobenzodiazepinone of the general formula II**, in which $R^{1**}$ and $R^{2**}$ denote a methyl radical, $R^{5**}$ and $R^{6**}$, including the nitrogen atom, denote a piperazin-1-yl group which is substituted in the 4-position by a methyl group and A** denotes a methylene group, or its pharmacologically acceptable acid addition salts are prepared.

8. Process according to Claim 3, characterized in that a pyrazolobenzodiazepinone of the general formula II**, in which $R^{1**}$ denotes a methyl radical, $R^{2**}$ denotes a hydrogen atom, $R^{5**}$ and $R^{6**}$ togehter, and including the nitrogen atom, denote a piperazin-1-yl group which is substituted in the 4-position by a methyl group and A** denotes a methylene group, or its pharmacologically acceptable acid addition salts are prepared.

9. Process for the preparation of the intermediates of the general formula I

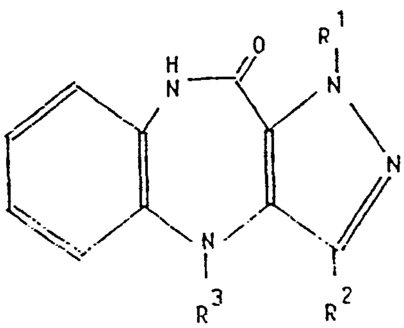

(I)

wherein
$R^1$ denotes an alkyl radical with 1 to 4 carbon atoms,
$R^2$ denotes a hydrogen atom or an alkyl radical with 1 to 4 carbon atoms,
$R^3$ denotes a hydrogen atom or the group -CO-A-$R^4$,
$R^4$ denotes a halogen atom and
A denotes a straight-chain or branched alkylene group with 1 to 5 carbon atoms,
and their cid addition salts, characterized in that anilinopyrazolecarboxylic acid derivatives of the general formula II

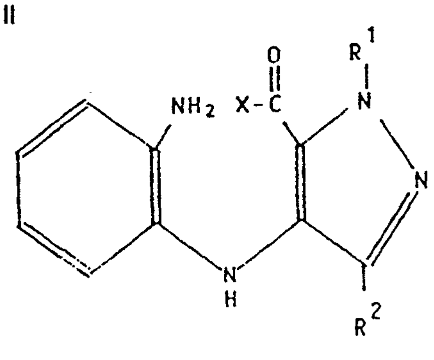

wherein X denotes a leaving group, are cyclised and, in the resulting reaction product of the formula I wherein $R^3$ denotes a hydrogen atom, the hydrogen atom is then replaced by a -CO-A-$R^4$ group, and/or resulting bases are converted into the acid addition salts or resulting acid addition salts are converted into the free base.

10. Process according to Claim 9, characterized in that compounds II in which X represents an -OH group, an O-$R^9$ group or an -N($R^{10}$)$R^{11}$ group, wherein $R^9$ denotes an alkyl group, a cycloalkyl group, an optionally substituted aralkyl group or an optionally substituted aryl group, $R^{10}$ denotes a hydrogen atom, an alkyl group, a cycloalkyl group, an optionally substituted aralkyl group or an optionally substituted aryl group and $R^{11}$ has one of the meanings of $R^{10}$, or $R^{10}$ and $R^{11}$ together, and including the nitrogen atom to which they are linked, denote an optionally substituted, non-aromatic, heterocyclic radical, are employed.

11. Process according to Claim 9, characterized in that compounds II, wherein $R^1$ and $R^2$ denote a methyl group and X denotes a -OH-group or a -O-$R^9$-group, wherein $R^9$ denotes an alkyl group, an optionally substituted aralkyl group or an optionally substituted aryl group, are employed.

12. Process according to Claim 9, characterized in that the substitution which optionally follows later is carried out by means of compounds of the general formula Hal-A-CO-Hal' (III) or $(Hal\text{-}A\text{-}CO)_2O$ (IV), wherein Hal and Hal' denote a halogen atom and A denotes an alkylene group with 1 to 5 carbon atoms.

13. Process according to Claim 12, characterized in that compounds of the general formula III or IV, wherein A denotes a methylene group or ethylene group, are used.

14.Process according to Claims 9, 10 or 12, characterized in that intermediates of the general formula I*

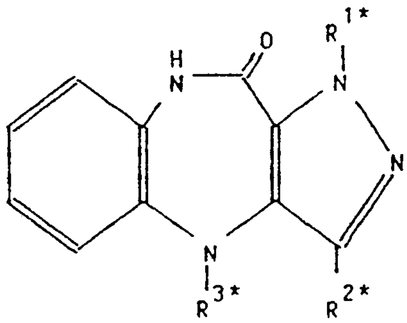

(I)

wherein
$R^{1*}$ denotes a methyl or ethyl radical,
$R^{2*}$ denotes a hydrogen atom or a methyl or ethyl radical,
$R^{3*}$ denotes a hydrogen atom or the group -CO-A*-$R^{4*}$,
$R^{4*}$ denotes a chlorine atom and
A* denotes a straight-chain or branched alkylene group with 1 or 2 carbon atoms,
or their acid addition salts are prepared.

15. Process according to Claim 10, characterized in that a compound II is used, wherein $R^1$ and $R^2$ are a methyl group, X is a -N($R^{10}$)$R^{11}$-group and $R^{10}$ and $R^{11}$ are hydrogen atoms.

16. Process according to Claim 11, characterized in that a compound II is used, wherein X is a -O-$R^9$-group and $R^9$ is an alkyl group.

17. Process according to Claim 11 and 12, characterized in that chloroacetylchloride is used as compound III.

**Revendications pour les Etats contractants BE CH DE FR GB IT LI LU NL SE**

1. Pyrazolobenzodiazépinones substituées répondant à la formule générale I

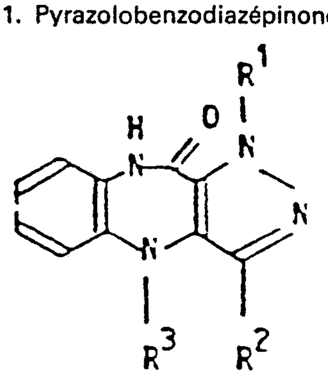

(I),

dans laquelle
$R^1$ représente un reste alkyle comportant 1 à 4 atomes de carbone,
$R^2$ représente un atome d'hydrogène ou un reste alkyle comportant 1 à 4 atomes de carbone,
$R^3$ représente le groupe -CO-A-$R^4$,
$R^4$ représente le groupe -N($R^5$)$R^6$, et
$R^5$ représente un reste alkyle comportant 1 à 4 atomes de carbone, ou bien un reste allyle ou 2-méthylallyle,
$R^6$ a l'une des significations de $R^5$ ou représente le groupe $-(CH_2)_m-N(R^7)R^8$, ou bien
$R^5$ et $R^6$ représentent ensemble, avec inclusion de l'atome d'azote auquel ils sont liés, un groupe morpholino, un groupe pyrrolidino, un groupe pipéridino, un groupe hexahydroazepine-1-yle, un groupe pipérazine-1-yle éventuellement substitué en position 4 par un groupe méthyle ou éthyle ou par un groupe benzyle, un groupe 2,4-diméthylpipérazine-1-yle ou un groupe hexahydro-1H-1,4-diazepine-1-yle substitué en position 4 par un groupe méthyle ou éthyle, et
$R^7$ représente un groupe alkyle comportant 1 à 4 atomes de carbone,
$R^8$ représente un groupe alkyle comportant 1 à 4 atomes de carbone,
A représente un groupe alkylène linéaire ou ramifié comportant 1 à 5 atomes de carbone, et
m représente un nombre égal à 2 ou 3,
et leurs sels d'addition avec des acides.

2. Pyrazolobenzodiazépinones substituées répondant à la formule générale I selon la revendication 1, où
$R^1$ représente un reste méthyle,
$R^2$ représente un reste méthyle,
$R^3$ représente le groupe -CO-A-$R^4$,
$R^4$ représente le groupe -N($R^5$)$R^6$, et
$R^5$ représente un reste alkyle comportant 1 à 4 atomes de carbone, ou bien un reste allyle ou méthylallyle,
$R^6$ a l'une des significations de $R^5$, ou bien
$R^5$ et $R^6$ représentent ensemble, avec inclusion de l'atome d'azote auquel ils sont liés, un groupe pyrrolidino, un groupe pipéridino, un groupe hexahydroazepine-1-yle, un groupe pipérazine-1-yle substitué en position 4 par un groupe méthyle ou éthyle, ou bien un groupe hexahydro-1H-1,4-diazepine-1-yle substitué en position 4 par un groupe méthyle ou éthyle, et
A représente un groupe alkylène linéaire comportant 1 ou 2 atomes de carbone,
et leurs sels d'addition avec des acides.

3. Pyrazolobenzodiazépinones substituées selon la revendication 1, caractérisées par la formule générale I** dans laquelle

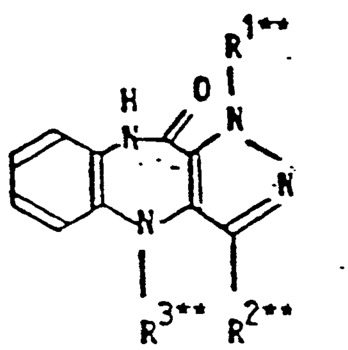

(I**),

$R^{1**}$ représente un reste méthyle ou éthyle,
$R^{2**}$ représente un atome d'hydrogène, un reste méthyle ou éthyle,
$R^{3**}$ représente le groupe -CO-A**-$R^{4**}$,
$R^{4**}$ représente le groupe -N($R^{5**}$)$R^{6**}$, et
$R^{5**}$ représente un reste alkyle comportant 1 à 4 atomes de carbone, ou bien un reste allyle ou méthylallyle,
$R^{6**}$ a la signification de $R^{5**}$ ou représente le groupe $-(CH_2)_{m}$**-N($R^{7**}$)$R^{8**}$, ou bien
$R^{5**}$ et $R^{6**}$ représentent ensemble, avec inclusion de l'atome d'azote auquel ils sont liés, un groupe morpholino, un groupe pyrrolidino, un groupe pipéridino, un groupe hexahydroazepine-1-yle, un groupe pipérazine-1-yle substitué en position 4 par un groupe méthyle, éthyle ou benzyle, un groupe 2,4-diméthyl-pipérazine-1-yle ou un groupe hexahydro-1H-1,4-diazepine-1-yle substiué en position 4 par un groupe méthyle ou éthyle, et
$R^{7**}$ représente un groupe méthyle ou éthyle,
$R^{8**}$ représente un groupe méthyle ou éthyle,
m** représente un nombre égal à 2 ou 3,

A** représente un groupe alkylène linéaire ou ramifié comportant 1 ou 2 atomes de carbone, et leurs sels d'addition avec des acides.

4. Composés selon la revendication 3, dans lesquels R£O1** représente un groupe méthyle ou éthyle, $R^{2**}$ représente un atome d'hydrogène, un reste méthyle ou éthyle, $R^{5**}$ représente un reste méthyle ou éthyle, $R^{6**}$ a la signification de $R^{5**}$ ou représenten le groupe $(CH_2)_m$**-N($R^{7**}$($R^{7**}$)$R^{8**}$, ou bien $R^{5**}$ et $R^{6**}$ représentent ensemble, avec inclusion de l'atome d'azote, un groupe pyrrolidino, pipéridino ou hexahydroazepine-1-yle, $R^{7**}$ et $R^{8**}$ représentent un reste méthyle ou éthyle, m** est égal à 2 et A** représente un groupe méthylène, et leurs sels d'addition avec des acides pharmacologiquement compatibles.

5. Composés selon la revendication 3, dans les quels $R^{1**}$ représente un reste méthyle ou éthyle, $R^{2**}$ représente un atome d'hydrogène, un reste méthyle ou éthyle, $R^{5**}$ et $R^{6**}$ représentent ensemble, avec inclusion de l'atome d'azote, un groupe pipérazine-1-yle substitué en position 4 par un groupe méthyle, éthyle ou benzyle, un groupe 2,4-diméthyl-pipérazine-1-yle ou un groupe hexahydro-1H-1,4-diazépine-1-yle substitué en position 4 par un groupe méthyle ou éthyle et A** représente un groupe méthylène, et leurs sels d'addition avec des acides pharmacologiquement compatibles.

6. Composés selon la revendication 3, dans lesquels $R^{1**}$ représente un reste méthyle ou éthyle, $R^{2**}$ représente un atome d'hydrogène, un reste méthyle ou éthyle, $R^{5**}$ et $R^{6**}$ représentent ensemble, avec inclusion de l'atome d'azote, un groupe pipérazine-1-yle substitué en position 4 par un groupe méthyle et A** représente un groupe méthylène, et leurs sels d'addition avec des acides pharmacologiquement compatibles.

7. Composé selon la revendication 3, dans lequel $R^{1**}$ et $R^{2**}$ représentent un reste méthyle, $R^{5**}$ et $R^{6**}$ représentent, avec inclusion de l'atome d'azote, un groupe pipérazine-1-yle substitué en position 4 par un groupe méthyle et A** représente un groupe méthylène, et leurs sels d'addition avec des acides pharmacologiquement compatibles.

8. Composé selon la revendication 3, dans lequel $R^{1**}$ représente un reste méthyle, $R^{2**}$ représente un atome d'hydrogène, $R^{5**}$ et $R^{6**}$ représentent, avec inclusion de l'atome d'azote, un groupe pipérazine-1-yle substitué en position 4 par un groupe méthyle et A** représente un groupe méthylène, et leurs sels d'addition avec des acides pharmacologiquement compatibles.

9. Médicaments renfermant une ou plusieurs pyrazolobenzodiazépinones répondant la formule générale I selon l'une quelconque des revendications 1 à 8 et/ou leurs sels d'addition avec des acides pharmacologiquement compatibles.

10. Procédé de préparation des pyrazolobenzodiazepinones substituées répondant à la formule générale I selon la revendication 1 et de leurs sels d'addition avec des acides, caractérisé en ce que l'on fait réagir des pyrazolobenzodiazepinones répondant à la formule générale II

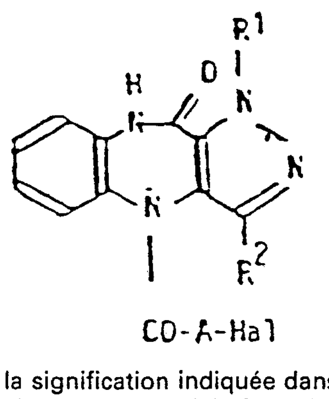

(II),

dans laquelle $R^1$, $R^2$ et A ont la signification indiquée dans la revendication 1 et Hal représente un atome d'halogène, sur des amines secondaires répondant à la formule générale V

$$HN(R^5)R^6 \quad (V)$$

dans laquelle $R^5$ et $R^6$ ont signification indiquée dans la revendication 1, et l'on transforme éventuellement ensiuite les bases obtenues en les sels d'addition avec des acides ou les sels d'addition avec des acides obtenus en les bases libres ou en des sels d'addition avec des acides pharmacologiquement compatibles.

1. Procédé selon la revendication 10, caractérisé en ce qu'on utilise des composés II dans lesquels $R^1$ et $R^2$ représentent un groupe méthyle et A un groupe méthylène ou éthylène et des amines secondaires V dans lesquelles $R^5$ représente un groupe alkyle comportant 1 à 4 atomes de carbone, ou un reste allyle ou 2-méthylallyle, $R^6$ a l'une des significations de $R^5$, ou bien $R^5$ et $R^6$ représentent ensemble un groupe pyrrolidino-1-yle, pipéridino-1-yle ou hexahydroazépine-1-yle, un groupe pipérazino substitué en position 4 par un

groupe méthyle ou éthyle ou un groupe hexahydro-1H-1,4-diazépine-1-yle substitué en position 4 par un groupe méthyle ou éthyle.

12. Composés actifs selon l'une quelconque des revendications 1 à 8, pour traiter des maladies basées sur des atteintes de l'estomac ou de l'intestin.

13. Produits intermédiaires répondant à la formule générale I

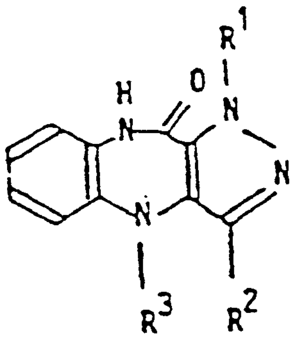

(I),

dans laquelle
$R^1$ représente un reste alkyle comportant 1 à 4 atomes de carbone,
$R^2$ représente un atome d'hydrogène ou un reste alkyle comportant 1 à 4 atomes de carbone,
$R^3$ représente un atome d'hydrogène ou le groupe -CO-A-$R^4$,
$R^4$ représente un atome d'halogène, et
A représente un groupe alkylène linéaire ou ramifié comportant 1 à 5 atomes de carbone,
ainsi que leurs sels d'addition avec des acides.

14. Produits intermédiaires répondant à la formule générale I selon la revendication 13, où
$R^1$ représente un reste méthyle,
$R^2$ représente un reste méthyle,
$R^3$ représente un atome d'hydrogène ou le groupe -CO-A-$R^4$,
$R^4$ représente un atome d'halogène, et
A représente un groupe alkylène linéaire comportant 1 ou 2 atomes de carbone,
et leurs sels d'addition avec des acides.

15. Produits intermédiaires selon la revendication 13, caractérisés par la formule générale I*

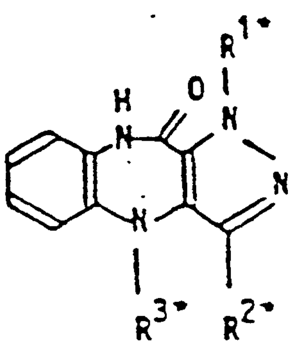

(I*),

dans laquelle
$R^1$ représente un reste méthyle ou éthyle,
$R^2$ représente un atome d'hydrogène, un reste méthyle ou éthyle,
$R^3$ représente un atome d'hydrogène ou le groupe -CO-A*-$R^{4*}$,
$R^4$ représente un atome de chlore, et
A* représente un groupe alkylène linéaire ou ramifié, comportant 1 ou 2 atomes de carbone,
et leurs sels d'addition avec des acides.

16. Composés selon la revendication 15, caractérisés en ce que A* représente un groupe méthylène.

17. Composés selon la revendication 15, dans lesquels $R^{1*}$ représente un reste méthyle ou éthyle, et $R^{2*}$ représente un atome d'hydrogène, un reste méthyle ou éthyle,
$R^{3*}$ représente un atome d'hydrogène ou le groupe -CO-$CH_2$-C1, et leurs sels d'addition avec des acides.

18. Procédé de préparation des produits intermédiaires répondant à la formule générale I

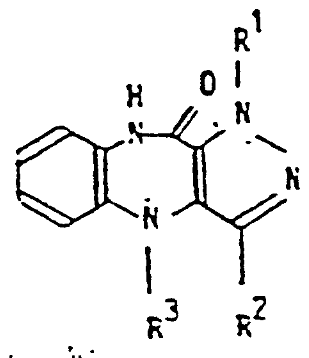

(I),

dans laquelle
$R^1$ représente un reste alkyle comportant 1 à atomes de carbone,
$R^2$ représente un atome d'hydrogène ou un reste alkyle comportant 1 à 4 atomes de carbone,
$R^3$ représente un atome d'hydrogène ou le groupe -CO-A-$R^4$,
$R^4$ représente un atome d'halogène, et
A représente un groupe alkylène linéaire ou ramifié, comportant 1 à 5 atomes de carbone,
ainsi que de leurs sels d'addition avec des acides, caractérisé en ce que l'on cyclise des dérivés d'acides anilinopyrazol-carboxyliques répondant à la formule générale II

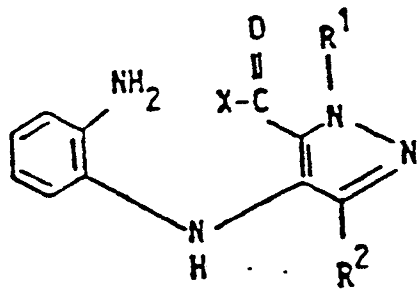

(II),

dans laquelle X représente un groupe partant,
et on les substitue éventuellement ensuite dans le produit de réaction répondant à la formule I obtenu, où $R^3$ représente un atome d'hydrogène, l'atome d'hydrogène par un groupe -CO-A-$R^4$- et/ou l'on transforme les bases obtenues en les sels d'addition avec des acides ou bien les sels d'addition avec des acides obtenus en les bases libres.

19. Procédé selon la revendication 18, caractérisé en ce qu'on utilise des composés II dans lesquels X représente un groupe -OH, un groupe O-$R^9$ ou un groupe -N($R^{10}$)$R^{11}$, où
$R^9$ représente un groupe alkyle, un groupe cycloalkyle, un groupe arylalkyle éventuellement substitué ou un groupe aryle éventuellement substitué, et
$R^{10}$ représente un atome d'hydrogène, un groupe alkyle, un groupe cycloalkyle, un groupe arylalkyle éventuellement substitué ou un groupe aryle éventuellement substitué,
$R^{11}$ a l'une des signifacations de $R^{10}$, ou bien
$R^{10}$ et $R^{11}$ représentent ensemble, avec inclusion de l'atome d'azote auquel ils sont liés, un reste hétérocyclique non-aromatique, éventuellement substitué.

20. Procédé selon la revendication 19, caractérisé en ce qu'on utilise des composés II, dans lesquels $R^1$ et $R^2$ représentent un groupe méthyle, et
X représente un groupe -OH ou un groupe -O-$R^9$, où
$R^9$ représente un groupe alkyle, un groupe arylalkyle éventuellement substitué ou un groupe aryle éventuellement substitué.

21. Procédé selon la revendication 18, caractérisé en ce qu'on effectue la substitution éventuellement subséquente avec des composés répondant à la formule générale Hal-A-CO-Hal' (III) ou $(Hal\text{-}A\text{-}CO)_2O$ (IV), où Hal et Hal' représentent un atome d'halogène et A un groupe alkylène comportant 1 à 5 atomes de carbone.

**Revendications pour l'Etat contractant AT**

1. Procédé de préparation de pyrazolobenzodiazépinones substituées répondant à la formule générale I

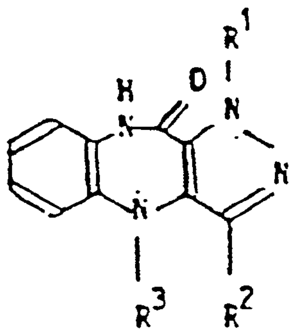

(I),

dans laquelle
$R^1$ représente un reste alkyle comportant 1 à 4 atomes de carbone,
$R^2$ représente un atome d'hydrogène ou un reste alkyle comportant 1 à 4 atomes de carbone,
$R^3$ représente le groupe -CO-A-$R^4$,
$R^4$ représente le groupe -N($R^5$)$R^6$, et
$R^5$ représente un reste alkyle comportant 1 à 4 atomes de carbone, ou un reste allyle ou 2-méthylallyle,
$R^6$ a l'une des significations de $R^5$ ou représente le groupe $-(CH_2)_m$-N($R^7$)$R^6$, ou bien
$R^5$ et $R^6$ représentent ensemble, avec inclusion de l'atome d'azote auquel ils sont liés, un groupe morpholino, un groupe pyrrolidino, un groupe pipéridino, un groupe hexahydroazépine-1-yle, un groupe pipérazine-1-yle éventuellement substitué en position 4 par un groupe méthyle ou éthyle, ou par un groupe benzyle, un groupe 2,4-diméthylpipérazine-1-yle ou un groupe hexahydro-1H-1,4-diazépine-1-yle substitué en position 4 par un groupe méthyle ou éthyle, et
$R^7$ représente un groupe alkyle comportant 1 à atomes de carbone,
$R^8$ représente un groupe alkyle comportant 1 à 4 atomes de carbone,
A représente un groupe alkylène linéaire ou ramifié comportant 1 à 5 atomes de carbone, et
m représente un entier égal à 2 ou 3,
et de leurs sels d'addition avec des acides,
caractérisé en ce que l'on fait réagir des pyrazolobenzodiazépinones répondant à la formule générale II

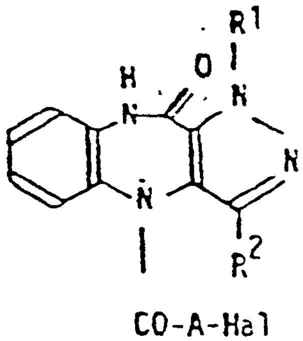

(II),

dans laquelle $R^1$, $R^2$ et A ont la signification indiquée précédemment et Hal représente un atome d'halogène, sur des amines secondaires répondant à la formule générale V

HN($R^5$)$R^6$ (V)

dans laquelle $R^5$ et $R^6$ ont la signification indiquée précédemment et l'on transforme éventuellement ensuite les bases obtenues en les sels d'addition avec des acides ou les sels d'addition avec des acides obtenus en les bases libres ou bien des sels d'addition avec des acides pharmacologiquement compatibles.

2. Procédé selon la revendication 1, caractérisé en ce qu'on utilise des composés II dans lesquels $R^1$ et $R^2$ représentent un groupe méthyle et A représente un groupe méthylène ou éthylène, et des amines secondaires V, dans lesquelles $R^5$ représente un groupe alkyle comportant 1 à 4 atomes de carbone, ou un reste allyle ou 2-méthylallyle,
$R^6$ a l'une des significations de $R^5$, ou bien
$R^5$ et $R^6$ représentent ensemble un groupe pyrrolidino-1-yle, pipéridino-1-yle ou hexahydroazepine-1-yle, un groupe pipérazino substiué en position 4 par un groupe méthyle ou éthyle, ou un groupe hexahydro-1H-1,4-diazépine-1-yle substitué en position 4 par un groupe méthyle ou éthyle.

3. Procédé selon la revendication 1, caractérisé en ce qu'on prépare des pyrazolobenzodiazépinones répondant à la formule générale I**

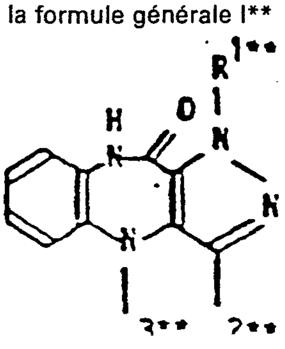

(I**),

dans laquelle
$R^{1**}$ représente un reste méthyle ou éthyle,
$R^{2**}$ représente atome d'hydrogène, un reste méthyle ou éthyle,
$R^{3**}$ représente le groupe -CO-A**-$R^{4**}$,
$R^{4**}$ représente le groupe -N($R^{5**}$)$R^{6**}$, et
$R^{5**}$ représente un reste alkyle comportant 1 à 4 atomes de carbone, ou un reste allyle ou 2-méthylallyle,
$R^{6**}$ a la signification de $R^{5**}$ ou représente le groupe $-(CH_2)_{m**}$-N($R^{7**}$)$R^{8**}$,
ou bien
$R^{5**}$ et $R^{6**}$ représentent ensemble, avec inclusion de l'atome d'azote auquel ils sont liés, un groupe morpholino, un groupe pyrrolidino, un groupe pipéridino, un groupe hexahydroazépine-1-yle, un groupe pipérazine-1-yle substitué en position 4 par un groupe méthyle, éthyle ou benzyle, un groupe 2,4-diméthyl-pipérazine-1-yle ou un groupe hexahydro-1H-1,4-diazépine-1-yle substitué en position 4 par un groupe méthyle ou éthyle, et
$R^{7**}$ représente un groupe méthyle ou éthyle,
$R^{8**}$ représente un groupe méthyle ou éthyle,
m** est un nombre égal à 2 ou 3,
A** représente un groupe alkylène linéaire ou ramifié comportant 1 ou 2 atomes de carbone,
ou leurs sels d'addition avec des acides,
en faisant réagir des pyrazolobenzodiazépinones répondant à la formule générale II**

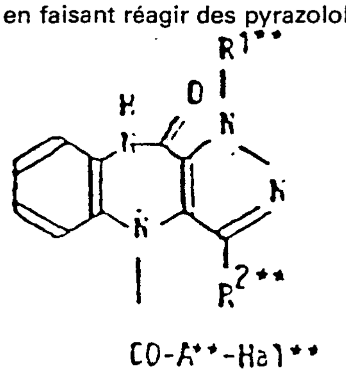

(II**),

dans laquelle $R^{1**}$, $R^{2**}$ et A** ont la signification indiquée précédemment et Hal** représente un atome d'halogène, sur des amines secondaires répondant à la formule générale V**

HN($R^{5**}$)$R^{6**}$ (V**)

dans laquelle $R^{5**}$ et $R^{6**}$ ont la signification indiquée précédemment.

4. Procédé selon la revendication 3, caractérisé en ce qu'on prépare des pyrazolobenzodiazépinones substituées répondant à la formule générale II**, dans laquelle $R^{1**}$ représente un reste méthyle ou éthyle, $R^{2**}$ représente un atome d'hydrogène, un reste méthyle ou éthyle, $R^{5**}$ représente un reste méthyle ou éthyle, $R^{6**}$ a la signification de $R^{5**}$ ou bien représente le groupe $(CH_2)_{m**}$-N($R^{7**}$)($R^{8**}$), ou bien $R^{5**}$ et $R^{6**}$ représentent ensemble, avec inclusion de l'atome d'azote, un reste pyrrolidimyle-1, pipéridyle-1 ou hexahydroazépine-1-yle, $R^{7**}$ et $R^{8**}$ représentent un reste méthyle ou éthyle, m** est égal à 2 et A** représente un groupe méthylène, ou bien leurs sels d'addition avec des acides pharmacologiquement compatibles.

5. Procédé selon la revendication 3, caractérisé en ce qu'on prépare des pyrazolobenzodiazépinones substituées répondant à la formule générale I**, dans laquelle $R^{1**}$ représente un reste méthyle ou éthyle, $R^{2**}$ représente un atome d'hydrogène, un reste méthyle ou éthyle, $R^{5**}$ et $R^{6**}$ représentent ensemble avec inclusion de l'atome d'azote un groupe pipérazine-1-yle substitué en position 4 par un groupe méthyle, éthyle ou benzyle, un groupe 2,4-diméthyl-pipérazine-1-yle ou un groupe hexahydro-1H-1,4-diazépine-1-yle substitué en position 4 par un groupe méthyle ou éthyle, et A** représente un groupe méthylène, ou bien leurs sels d'addition avec des acides pharmacologiquement compatibles.

6. Procédé selon la revendication 3, caractérisé en ce qu'on prépare des pyrazolobenzodiazépinones substituées répondant à la formule générale I**, dans laquelle $R^{1**}$ représente un reste méthyle ou éthyle, $R^{2**}$ représente un atome d'hydrogène, un reste méthyle ou éthyle, $R^{5**}$ et $R^{6**}$ représentent ensemble, avec inclusion de l'atome d'azote, un groupe pipérazine-1-yle substitué en position 4 par un groupe méthyle, et A** représente un groupe méthyle, ou bin leurs sels d'addition avec des acides pharmacologiquement compatibles.

7. Procédé selon la revendication 3, caractérisé en ce qu'on prépare la pyrazolobenzodiazépinone substituée

répondant à la formule générale I**, dans laquelle $R^{1**}$ et $R^{2**}$ représentent un reste méthyle, $R^{5**}$ et $R^{6**}$ représentent, avec inclusion de l'atome d'azote, un groupe pipérazine-1-yle substitué en position 4 par un groupe méthyle et A** représente un groupe méthylène, ou bien ses sels d'addition avec des acides pharmacologiquement compatibles.

8. Procédé selon la revendication 3, caractérisé en ce qu'on prépare la pyrazolobenzodiazépinone répondant à la formule générale I**, dans laquelle $R^{1**}$ représente un reste méthyle, $R^{2**}$ représente un atome d'hydrogène, $R^{5**}$ et $R^{6**}$ représentent, avec inclusion de l'atome d'azote, un groupe pipérazine-1-yle substitué en position 4 par un groupe méthyle et A** représente un groupe méthylène, ou bien ses sels d'addition avec des acides pharmacologiquement compatibles.

9. Procédé de préparation des produits intermédiaires répondant à la formule générale I

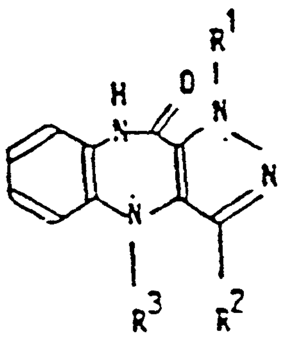

(I),

dans laquelle
$R^1$ représente un reste alkyle comportant 1 à 4 atomes de carbone,
$R^2$ représente un atome d'hydrogène ou un reste alkyle comportant 1 à 4 atomes de carbone,
$R^3$ représente un atome d'hydrogène ou le groupe -CO-A-$R^4$,
$R^4$ représente un atome d'hydrogène, et
A représente un groupe alkylène linéaire ou ramifié comportant 1 à 5 atomes de carbone,
et de leurs sels d'addition avec des acides,
caractérisé en ce que l'on cyclise des dérivés d'acides anilinopyrazol-carboxyliques répondant à la formule générale II

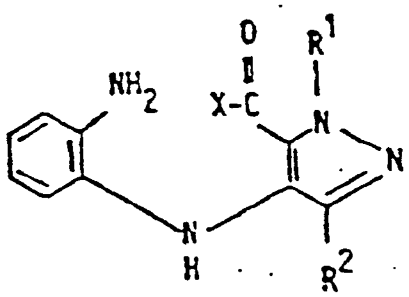

(II),

dans laquelle
X représente un groupe partant,
et l'on substitue éventuellement ensuite, dans le produit de réaction de la formule I obtenu, dans laquelle $R^3$ représente un atome d'hydrogène, l'atome d'hydrogène par un groupe -CO-A-$R^4$ et/ou l'on transforme les bases obtenues en sels d'addition avec des acides ou les sels d'addition avec des acides obtenus en bases libres.

10. Procédé selon la revendication 9, caractérisé en ce qu'on utilise des composés II dans lesquels X représente un groupe -OH, un groupe O-$R^9$ ou un groupe -N($R^{10}$)$R^{11}$, où
$R^9$ représente un groupe alkyle, un groupe cycloalkyle, un groupe arylalkyle éventuellement substitué ou un groupe aryle éventuellement substitué, et
$R^{10}$ représente un atome d'hydrogène, un groupe alkyle, un groupe cycloalkyle, un groupe arylalkyle éventuellement substitué ou un groupe aryle éventuellement substitué,
$R^{11}$ a l'une des significations de $R^{10}$, ou bien
$R^{10}$ et $R^{11}$ représentent ensemble, avec inclusion de l'atome d'azote auquel ils sont liés, un reste hétérocyclique nonaromatique éventuellement substitué.

11. Procédé selon la revendication 9, caractérisé en ce qu'on utilise des composés II, dans lesquels $R^1$ et $R^2$ représentent un groupe méthyle, et
X représente un groupe OH ou un groupe -O-$R^9$, où
$R^9$ représente un groupe alkyle, un groupe arylalkyle éventuellement substitué ou un groupe aryle éventuellement

substitué.

12. Procédé selon la revendication 9, caractérisé en ce qu'on effectue la substitution éventuellement subséquente avec des composés répondant à la formule générale Hal-A-CO-Hal' (III) ou $(Hal\text{-}A\text{-}CO)_2O$ (IV), où Hal et Hal' représentent un atome d'halogène et A représente un groupe alkylène comportant 1 à 5 atomes de carbone.

13. Procédé selon la revendication 12, caractérisé en ce qu'on utilise des composés répondant à la formule générale III ou IV, dans lesquelles A représente un groupe méthylène ou éthylène.

14. Procédé selon les revendications 9, 10 ou 12, caractérisé en ce que l'on prépare des produits intermédiaires répondant à la formule générale I*

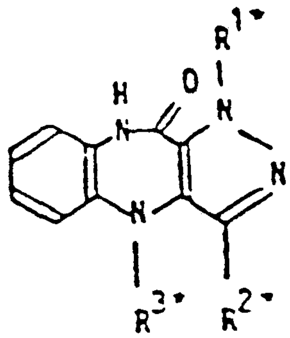

(I*),

dans laquelle
$R^{1*}$ représente un reste méthyle ou éthyle,
$R^{2*}$ représente un atome d'hydrogène, ou reste méthyle ou éthyle,
$R^{3*}$ représente un atome d'hydrogène ou le groupe $-CO-A^*-R^{4*}$,
$R^{4*}$ représente un atome de chlore, et
A* représente un groupe alkylène linéaire ou ramifié comportant 1 ou 2 atomes de carbone,
ou leurs sels d'addition avec des acides.

15. Procédé selon la revendication 10, caractérisé en ce qu'on utilise un composé II dans lequel $R^1$ et $R^2$ représentent un groupe méthyle, X représente un groupe $-N(R^{10})R^{11}$, et $R^{10}$ et $R^{11}$ représentent un atome d'hydrogène.

16. Procédé selon la revendication 11, caractérisé en ce qu'on utilise un composé II, dans lequel X représente un groupe $-O-R^9$, et $R^9$ représente un groupe alkyle.

17. Procédé selon les revendications 11 et 12, caractérisé en ce qu'on utilise, comme composé III du chlorure de chloracétyle.